# EUROPEAN PATENT SPECIFICATION

(11) **EP 2 055 718 B1**
(45) Date of publication and mention of the grant of the patent: **26.12.2012**
(21) Application number: 08019358.4
(22) Date of filing: 13.11.2006
(51) Int. Cl.: C07K 16/18, C07K 16/46, C07K 19/00

(54) **Targeting and tracing of antigens in living cells**
Zielwertermittlung und Verfolgung von Antigenen in lebenden Zellen
Ciblage et suivi d'antigènes dans des cellules vivantes

(30) Priority: 11.11.2005 EP 05024739
(43) Date of publication of application: 06.05.2009
(62) Divisional of application: 06818501.6
(73) Proprietor: Ludwig-Maximilians-Universität München, 80539 München (DE)
(72) Inventor: Rothbauer, Ulrich, 80796 München (DE); Leonhardt, Heinrich, 80331 München (DE)
(74) Representative: Vossius & Partner

(56) References cited:
- EP-A- 1 433 793
- WO-A-2004/041867
- WO-A-2004/044204
- WO-A-2004/065416
- TANHA J ET AL: "Selection by phage display of llama conventional VH fragments with heavy chain antibody VHH properties" JOURNAL OF IMMUNOLOGICAL METHODS, ELSEVIER SCIENCE PUBLISHERS B.V.,AMSTERDAM, NL, vol. 263, no. 1-2, 1 May 2002 (2002-05-01), pages 97-109, XP004354388 ISSN: 0022-1759

### SEQUENCE LISTING

<110> Ludwig-Maximilians-Universität München
<120> Targeting and tracing of antigens in living cells
<130> L 2417 EP/1
<160> 33
<170> PatentIn version 3.3
<210> 1
   <211> 117
   <212> PRT
   <213> artificial sequence
<220>
   <223> generic variable domain
<220>
   <221> misc feature
   <222> (29)..(37)
   <223> Xaa can be any naturally occurring amino acid
<220>
   <221> misc feature
   <222> (52)..(68)
   <223> Xaa can be any naturally occurring amino acid
<220>
   <221> misc feature
   <222> (101)..(106)
   <223> Xaa can be any naturally occurring amino acid
<400> 1
   Met Ala Asp Val Gln Leu Val Glu Ser Gly Gly Ala Leu Val Gln Pro
   1               5                   10                  15
   Gly Gly Ser Leu Arg Leu Ser Cys Ala Ala Ser Gly Xaa Xaa Xaa Xaa
               20                  25                  30
   Xaa Xaa Xaa Xaa Xaa Trp Tyr Arg Gln Ala Pro Gly Lys Glu Arg Glu
           35                  40                  45
   Trp Val Ala Xaa Xaa Xaa Xaa Xaa Xaa Xaa Xaa Xaa Xaa Xaa Xaa Xaa
       50                  55                  60
   Xaa Xaa Xaa Xaa Arg Phe Thr Ile Ser Arg Asp Asp Ala Arg Asn Thr
   65                  70                  75                  80
   Val Tyr Leu Gln Met Asn Ser Leu Lys Pro Glu Asp Thr Ala Val Tyr
                   85                  90                  95
   Tyr Cys Asn Val Xaa Xaa Xaa Xaa Xaa Xaa Trp Gly Gln Gly Thr Gln
               100                 105                 110
   Val Thr Val Ser Ser
           115
<210> 2
   <211> 351
   <212> DNA
   <213> artificial sequence
<220>
   <223> generic variable domain, a coding sequence for SEQ ID NO: 1
<220>
   <221> misc feature
   <222> (85)..(111)
   <223> n is a, c, g, or t
<220>
   <221> misc_feature
   <222> (154)..(204)
   <223> n is a, c, g, or t
<220>
   <221> misc_feature
   <222> (301)..(318)
   <223> n is a, c, g, or t
<400> 2
   atggccgatg tgcagctggt ggagtctggg ggagccttgg tgcagccggg ggggtctctg     60
   agactctcct gtgcagcctc tgganinnnn nnnnnnnnnn nnnnnnnnnn ntggtaccgc    120
   caggctccag ggaaggagcg cgagtgggtc gcgnnnnnnn nnnnnnnnnn nnnnnnnnnn    180
   nnnnnnnnnn nnnnnnnnnn nnnncgattc accatctcca gagacgacgc caggaatacg    240
   gtgtatctgc aaatgaacag cctgaaacct gaggacacgg ccgtgtatta ctgtaatgtc    300
   nnnnnnnnnn nnnnnnnntg ggccaggggg acccaggtca ccgtctcctc a             351
<210> 3
   <211> 9
   <212> PRT
   <213> artificial sequence
<220>
   <223> CDR 1
<400> 3
   Phe Pro Val Asn Arg Tyr Ser Met Arg
   1               5
<210> 4
   <211> 17
   <212> PRT
   <213> artificial sequence
<220>
   <223> CDR 2
<400> 4
   Gly Met Ser Ser Ala Gly Asp Arg Ser Ser Tyr Glu Asp Ser Val Lys
   1               5                   10                  15
   Gly
<210> 5
   <211> 6
   <212> PRT
   <213> artificial sequence
<220>
   <223> CDR 3
<400> 5
   Asn Val Gly Phe Glu Tyr
   1               5
<210> 6
   <211> 238
   <212> PRT
   <213> Aequorea victoria
<400> 6
   Met Ser Lys Gly Glu Glu Leu Phe Thr Gly Val Val Pro Ile Leu Val
   1               5                   10                  15
   Glu Leu Asp Gly Asp Val Asn Gly His Lys Phe Ser Val Ser Gly Glu
               20                  25                  30
   Gly Glu Gly Asp Ala Thr Tyr Gly Lys Leu Thr Leu Lys Phe Ile Cys
           35                  40                  45
   Thr Thr Gly Lys Leu Pro Val Pro Trp Pro Thr Leu Val Thr Thr Phe
       50                  55                  60
   Ser Tyr Gly Val Gln Cys Phe Ser Arg Tyr Pro Asp His Met Lys Gln
   65                  70                  75                  80
   His Asp Phe Phe Lys Ser Ala Met Pro Glu Gly Tyr Val Gln Glu Arg
                   85                  90                  95
   Thr Ile Phe Phe Lys Asp Asp Gly Asn Tyr Lys Thr Arg Ala Glu Val
               100                 105                 110
   Lys Phe Glu Gly Asp Thr Leu Val Asn Arg Ile Glu Leu Lys Gly Ile
           115                 120                 125
   Asp Phe Lys Glu Asp Gly Asn Ile Leu Gly His Lys Leu Glu Tyr Asn
       130                 135                 140
   Tyr Asn Ser His Asn Val Tyr Ile Met Ala Asp Lys Gln Lys Asn Gly
   145                 150                 155                 160
   Ile Lys Val Asn Phe Lys Ile Arg His Asn Ile Glu Asp Gly Ser Val
                   165                 170                 175
   Gln Leu Ala Asp His Tyr Gln Gln Asn Thr Pro Ile Gly Asp Gly Pro
               180                 185                 190
   Val Leu Leu Pro Asp Asn His Tyr Leu Ser Thr Gln Ser Ala Leu Ser
           195                 200                 205
   Lys Asp Pro Asn Glu Lys Arg Asp His Met Val Leu Leu Glu Phe Val
       210                 215                 220
   Thr Ala Ala Gly Ile Thr His Gly Met Asp Glu Leu Tyr Lys
   225                 230                 235
<210> 7
   <211> 717
   <212> DNA
   <213> Aequorea victoria
<400> 7
   atgagtaaag gagaagaact tttcactgga gttgtcccaa ttcttgttga attagatggt      60
   gatgttaatg ggcacaaatt ttctgtcagt ggagagggtg aaggtgatgc aacatacgga     120
   aaacttaccc ttaaatttat ttgcactact ggaaaactac ctgttccatg gccaacactt     180
   gtcactactt tctcttatgg tgttcaatgc ttttcaagat acccagatca tatgaaacag     240
   catgactttt tcaagagtgc catgcccgaa ggttatgtac aggaaagaac tatatttttc     300
   aaagatgacg ggaactacaa gacacgtgct gaagtcaagt ttgaaggtga tacccttgtt     360
   aatagaatcg agttaaaagg tattgatttt aaagaagatg gaaacattct tggacacaaa     420
   ttggaataca actataactc acacaatgta tacatcatgg cagacaaaca aaagaatgga     480
   atcaaagtta acttcaaaat tagacacaac attgaagatg gaagcgttca actagcagac     540
   cattatcaac aaaatactcc aattggcgat ggccctgtcc ttttaccaga caaccattac     600
   ctgtccacac aatctgccct ttcgaaagat cccaacgaaa agagagacca catggtcctt     660
   cttgagtttg taacagctgc tgggattaca catggcatgg atgaactata caaataa        717
<210> 8
   <211> 225
   <212> PRT
   <213> Discosoma
<400> 8
   Met Arg Ser Ser Lys Asn Val Ile Lys Glu Phe Met Arg Phe Lys Val
   1               5                   10                  15
   Arg Met Glu Gly Thr Val Asn Gly His Glu Phe Glu Ile Glu Gly Glu
               20                  25                  30
   Gly Glu Gly Arg Pro Tyr Glu Gly His Asn Thr Val Lys Leu Lys Val
           35                  40                  45
   Thr Lys Gly Gly Pro Leu Pro Phe Ala Trp Asp Ile Leu Ser Pro Gln
       50                  55                  60
   Phe Gln Tyr Gly Ser Lys Val Tyr Val Lys His Pro Ala Asp Ile Pro
   65                  70                  75                  80
   Asp Tyr Lys Lys Leu Ser Phe Pro Glu Gly Phe Lys Trp Glu Arg Val
                   85                  90                  95
   Met Asn Phe Glu Asp Gly Gly Val Val Thr Val Thr Gln Asp Ser Ser
               100                 105                 110
   Leu Gln Asp Gly Cys Phe Ile Tyr Lys Val Lys Phe Ile Gly Val Asn
           115                 120                 125
   Phe Pro Ser Asp Gly Pro Val Met Gln Lys Lys Thr Met Gly Trp Glu
       130                 135                 140
   Ala Ser Thr Glu Arg Leu Tyr Pro Arg Asp Gly Val Leu Lys Gly Glu
   145                 150                 155                 160
   Ile His Lys Ala Leu Lys Leu Lys Asp Gly Gly His Tyr Leu Val Glu
                   165                 170                 175
   Phe Lys Ser Ile Tyr Met Ala Lys Lys Pro Val Gln Leu Pro Gly Tyr
               180                 185                 190
   Tyr Tyr Val Asp Ser Lys Leu Asp Ile Thr Ser His Asn Glu Asp Tyr
           195                 200                 205
   Thr Ile Val Glu Gln Tyr Glu Arg Thr Glu Gly Arg His His Leu Phe
       210                 215                 220
   Leu
   225
<210> 9
   <211> 678
   <212> DNA
   <213> Discosoma
<400> 9
   atgaggtctt ccaagaatgt tatcaaggag ttcatgaggt ttaaggttcg catggaagga      60
   acggtcaatg ggcacgagtt tgaaatagaa ggcgaaggag aggggaggcc atacgaaggc     120
   cacaataccg taaagcttaa ggtaaccaag gggggacctt tgccatttgc ttgggatatt     180
   ttgtcaccac aatttcagta tggaagcaag gtatatgtca agcaccctgc cgacatacca     240
   gactataaaa agctgtcatt tcctgaagga tttaaatggg aaagggtcat gaactttgaa     300
   gacggtggcg tcgttactgt aacccaggat tccagtttgc aggatggctg tttcatctac     360
   aaggtcaagt tcattggcgt gaactttcct tccgatggac ctgttatgca aaagaagaca     420
   atgggctggg aagccagcac tgagcgtttg tatcctcgtg atggcgtgtt gaaaggagag     480
   attcataagg ctctgaagct gaaagacggt ggtcattacc tagttgaatt caaaagtatt     540
   tacatggcaa agaagcctgt gcagctacca gggtactact atgttgactc caaactggat     600
   ataacaagcc acaacgaaga ctatacaatc gttgagcagt atgaaagaac cgagggacgc     660
   caccatctgt tcctttag                                                   678
<210> 10
   <211> 117
   <212> PRT
   <213> artificial sequence
<220>
   <223> VHH fragment of fluorescent chromobody against GFP
<400> 10
   Met Ala Asp Val Gln Leu Val Glu Ser Gly Gly Ala Leu Val Gln Pro
   1               5                   10                  15
   Gly Gly Ser Leu Arg Leu Ser Cys Ala Ala Ser Gly Phe Pro Val Asn
               20                  25                  30
   Arg Tyr Ser Met Arg Trp Tyr Arg Gln Ala Pro Gly Lys Glu Arg Glu
           35                  40                  45
   Trp Val Ala Gly Met Ser Ser Ala Gly Asp Arg Ser Ser Tyr Glu Asp
       50                  55                  60
   Ser Val Lys Gly Arg Phe Thr Ile Ser Arg Asp Asp Ala Arg Asn Thr
   65                  70                  75                  80
   Val Tyr Leu Gln Met Asn Ser Leu Lys Pro Glu Asp Thr Ala Val Tyr
                   85                  90                  95
   Tyr Cys Asn Val Asn Val Gly Phe Glu Tyr Trp Gly Gln Gly Thr Gln
               100                 105                 110
   Val Thr Val Ser Ser
           115
<210> 11
   <211> 351
   <212> DNA
   <213> artificial sequence
<220>
   <223> sequence encoding VHH fragment of fluorescent chromobody of SEQ ID NO: 10
<400> 11
   atggccgatg tgcagctggt ggagtctggg ggagccttgg tgcagccggg ggggtctctg     60
   agactctcct gtgcagcctc tggattcccc gtcaatcgct atagtatgag gtggtaccgc    120
   caggctccag ggaaggagcg cgagtgggtc gcgggtatga gtagtgctgg tgatcgttca    180
   agttatgaag actccgtgaa gggccgattc accatctcca gagacgacgc caggaatacg    240
   gtgtatctgc aaatgaacag cctgaaacct gaggacacgg ccgtgtatta ctgtaatgtc    300
   aatgtgggct ttgagtactg gggccagggg acccaggtca ccgtctcctc a             351
<210> 12
   <211> 123
   <212> PRT
   <213> artificial sequence
<220>
   <223> VHH fragment of fluorescent chromobody against lamin
<400> 12
   Met Ala Gln Val Gln Leu Val Glu Ser Gly Gly Gly Leu Val Gln Pro
   1               5                   10                  15
   Gly Glu Ser Leu Arg Leu Ser Cys Ala Ala Ser Gly Phe Asn Phe Ala
               20                  25                  30
   Ser Tyr Ser Met Thr Trp Val Arg Gln Ala Pro Gly Lys Glu Leu Asp
           35                  40                  45
   Trp Val Ala Thr Ile Ser Phe Ser Gly Gly Ser Thr Tyr Tyr Asp Asp
       50                  55                  60
   Ser Val Lys Gly Arg Ala Thr Ile Ser Arg Asp Asn Ala Lys Ser Thr
   65                  70                  75                  80
   Val Tyr Leu Gln Leu Asn Ser Leu Lys Thr Glu Asp Thr Gly Met Tyr
                   85                  90                  95
   Tyr Cys Thr Lys Gly Arg Val Ser Pro Pro Ile Pro Asp Gly Leu Ser
               100                 105                 110
   Arg Gly Gln Gly Thr Gln Val Thr Val Ser Ser
           115                 120
<210> 13
   <211> 369
   <212> DNA
   <213> artificial sequence
<220>
   <223> sequence encoding VHH fragment of fluorescent chromobody of SEQ ID NO: 12
<400> 13
   atggctcagg tgcagctggt ggagtctggg ggaggcttgg tgcagcctgg ggagtctctc      60
   agactctcct gtgcagcctc tggattcaac ttcgcaagtt attccatgac ctgggtccgc     120
   caggctccag ggaaggaact cgactgggtc gcaacaataa gtttcagtgg tgggtccact     180
   tactatgatg actccgtgaa gggccgggcc acaatctcca gagacaacgc caagagcaca     240
   gtgtatctcc agttaaacag cctgaaaact gaggacacgg gaatgtatta ctgtacaaaa     300
   ggcagagtat ccccgcccat tcccgatggc ctttctcgag gccaggggac ccaggtcacc     360
   gtctcctca                                                             369
<210> 14
   <211> 128
   <212> PRT
   <213> artificial sequence
<220>
   <223> VHH fragment of fluorescent chromobody against cytokeratin-8
<400> 14
   Met Ala Gln Val Gln Leu Gln Glu Ser Gly Gly Gly Leu Val Gln Ala
   1               5                   10                  15
   Gly Gly Ser Leu Thr Leu Ser Cys Thr Tyr Ser Gly Leu Thr Phe Asp
               20                  25                  30
   Asp Tyr Asn Met Gly Trp Phe Arg Gln Gly Pro Gly Lys Glu Arg Glu
           35                  40                  45
   Arg Val Ser Ala Ile Ser Phe Arg Gly Ile Thr Tyr Tyr Val Asp Ser
       50                  55                  60
   Val Lys Gly Arg Phe Thr Ile Ser Arg Asp Asn Ala Lys Lys Thr Leu
   65                  70                  75                  80
   Tyr Leu Gln Met Asn Gly Leu Thr Pro Asp Asp Thr Ala Thr Tyr Tyr
                   85                  90                  95
   Cys Ala Gly Ser Arg Phe Leu Ser Pro Phe Val Arg Asp Gly Asp Thr
               100                 105                 110
   Lys Leu Ile Asn Asp Trp Gly Gln Gly Thr Gln Val Thr Val Ser Ser
           115                 120                 125
<210> 15
   <211> 384
   <212> DNA
   <213> artificial sequence
<220>
   <223> sequence encoding VHH fragment of fluorescent chromobody of SEQ ID NO: 14
<400> 15
   atggctcagg tacagctgca ggagtctgga ggaggcttgg tgcaggcagg ggggtctctg      60
   acactctcct gtacatactc tggcttgact tttgatgatt ataacatggg ctggttccgc     120
   cagggtccag ggaaggagcg cgagagagtt tcggcgatta gctttcgtgg catcacctat     180
   tatgtagact ccgttaaggg ccgattcacc atctccaggg ataacgccaa gaagacgctg     240
   tatctgcaaa tgaacggcct gacacctgac gatacggcca cgtattactg tgcgggtagt     300
   cgcttcttaa gtccctttgt acgcgatggt gacacgaaat tgattaacga ttggggccag     360
   gggacccagg tcaccgtctc ctca                                            384
<210> 16
   <211> 229
   <212> PRT
   <213> artificial sequence
<220>
   <223> mRFP1
<400> 16
   Met Ala Ser Ser Glu Asp Val Ile Lys Glu Phe Met Arg Phe Lys Val
   1               5                   10                  15
   Arg Met Glu Gly Ser Val Asn Gly His Glu Phe Glu Ile Glu Gly Glu
               20                  25                  30
   Gly Glu Gly Arg Pro Tyr Glu Gly Thr Gln Thr Ala Lys Leu Lys Val
           35                  40                  45
   Thr Lys Gly Gly Pro Leu Pro Phe Ala Trp Asp Ile Leu Ser Pro Gln
       50                  55                  60
   Phe Gln Tyr Gly Ser Lys Ala Tyr Val Lys His Pro Ala Asp Ile Pro
   65                  70                  75                  80
   Asp Tyr Leu Lys Leu Ser Phe Pro Glu Gly Phe Lys Trp Glu Arg Val
                   85                  90                  95
   Met Asn Phe Glu Asp Gly Gly Val Val Thr Val Thr Gln Asp Ser Ser
               100                 105                 110
   Leu Gln Asp Gly Glu Phe Ile Tyr Lys Val Lys Leu Arg Gly Thr Asn
           115                 120                 125
   Phe Pro Ser Asp Gly Pro Val Met Gln Lys Lys Thr Met Gly Trp Glu
       130                 135                 140
   Ala Ser Thr Glu Arg Met Tyr Pro Glu Asp Gly Ala Leu Lys Gly Glu
   145                 150                 155                 160
   Ile Lys Met Arg Leu Lys Leu Lys Asp Gly Gly His Tyr Asp Ala Glu
                   165                 170                 175
   Val Lys Thr Thr Tyr Met Ala Lys Lys Pro Val Gln Leu Pro Gly Ala
               180                 185                 190
   Tyr Lys Thr Asp Ile Lys Leu Asp Ile Thr Ser His Asn Glu Asp Tyr
           195                 200                 205
   Thr Ile Val Glu Gln Tyr Glu Arg Ala Glu Gly Arg His Ser Thr Gly
       210                 215                 220
   Ala Gly Leu Tyr Lys
   225
<210> 17
   <211> 690
   <212> DNA
   <213> artificial sequence
<220>
   <223> mRFP1
<400> 17
   atggcctcct ccgaggacgt catcaaggag ttcatgcgct tcaaggtgcg catggagggc      60
   tccgtgaacg gccacgagtt cgagatcgag ggcgagggcg agggccgccc ctacgagggc     120
   acccagaccg ccaagctgaa ggtgaccaag ggcggccccc tgcccttcgc ctgggacatc     180
   ctgtcccctc agttccagta cggctccaag gcctacgtga agcaccccgc cgacatcccc     240
   gactacttga agctgtcctt ccccgagggc ttcaagtggg agcgcgtgat gaacttcgag     300
   gacggcggcg tggtgaccgt gacccaggac tcctccctgc aggacggcga gttcatctac     360
   aaggtgaagc tgcgcggcac caacttcccc tccgacggcc ccgtaatgca gaagaagacc     420
   atgggctggg aggcctccac cgagcggatg taccccgagg acggcgccct gaagggcgag     480
   atcaagatga ggctgaagct gaaggacggc ggccactacg acgccgaggt caagaccacc     540
   tacatggcca agaagcccgt gcagctgccc ggcgcctaca agaccgacat caagctggac     600
   atcacctccc acaacgagga ctacaccatc gtggaacagt acgagcgcgc cgagggccgc     660
   cactccaccg gcgccggcct gtacaagtaa                                      690
<210> 18
   <211> 368
   <212> PRT
   <213> artificial sequence
<220>
   <223> anti-GFP chromobody
<400> 18
   Met Ala Asp Val Gln Leu Val Glu Ser Gly Gly Ala Leu Val Gln Pro
   1               5                   10                  15
   Gly Gly Ser Leu Arg Leu Ser Cys Ala Ala Ser Gly Phe Pro Val Asn
               20                  25                  30
   Arg Tyr Ser Met Gly Trp Tyr Arg Gln Ala Pro Gly Lys Glu Arg Glu
           35                  40                  45
   Trp Val Ala Gly Met Ser Ser Ala Gly Asp Arg Ser Ser Tyr Glu Asp
       50                  55                  60
   Ser Val Lys Gly Arg Phe Thr Ile Ser Arg Asp Asp Ala Arg Asn Thr
   65                  70                  75                  80
   Val Tyr Leu Gln Met Asn Ser Leu Lys Pro Glu Asp Thr Ala Val Tyr
                   85                  90                  95
   Tyr Cys Asn Val Asn Val Gly Phe Glu Tyr Trp Gly Gln Gly Thr Gln
               100                 105                 110
   Val Thr Val Ser Ser Arg Asn Ser Ile Ser Ser Leu Ser Ile Pro Ser
           115                 120                 125
   Thr Val Pro Arg Ala Arg Asp Pro Pro Val Asp Met Ala Ser Ser Glu
       130                 135                 140
   Asp Val Ile Lys Glu Phe Met Arg Phe Lys Val Arg Met Glu Gly Ser
   145                 150                 155                 160
   Val Asn Gly His Glu Phe Glu Ile Glu Gly Glu Gly Glu Gly Arg Pro
   165 170 175
   Tyr Glu Gly Thr Gln Thr Ala Lys Leu Lys Val Thr Lys Gly Gly Pro
   180 185 190
   Leu Pro Phe Ala Trp Asp Ile Leu Ser Pro Gln Phe Gln Tyr Gly Ser
   195 200 205
   Lys Ala Tyr Val Lys His Pro Ala Asp Ile Pro Asp Tyr Leu Lys Leu
   210 215 220
   Ser Phe Pro Glu Gly Phe Lys Trp Glu Arg Val Met Asn Phe Glu Asp
   225 230 235 240
   Gly Gly Val Val Thr Val Thr Gln Asp Ser Ser Leu Gln Asp Gly Glu
   245 250 255
   Phe Ile Tyr Lys Val Lys Leu Arg Gly Thr Asn Phe Pro Ser Asp Gly
   260 265 270
   Pro Val Met Gln Lys Lys Thr Met Gly Trp Glu Ala Ser Thr Glu Arg
   275 280 285
   Met Tyr Pro Glu Asp Gly Ala Leu Lys Gly Glu Ile Lys Met Arg Leu
   290 295 300
   Lys Leu Lys Asp Gly Gly His Tyr Asp Ala Glu Val Lys Thr Thr Tyr
   305 310 315 320
   Met Ala Lys Lys Pro Val Gln Leu Pro Gly Ala Tyr Lys Thr Asp Ile
   325 330 335
   Lys Leu Asp Ile Thr Ser His Asn Glu Asp Tyr Thr Ile Val Glu Gln
   340 345 350
   Tyr Glu Arg Ala Glu Gly Arg His Ser Thr Gly Ala Gly Leu Tyr Lys
   355 360 365
<210> 19
   <211> 1107
   <212> DNA
   <213> artificial sequence
<220>
   <223> anti-GFP chromobody
<400> 19
   atggccgatg tgcagctggt ggagtctggg ggagccttgg tgcagccggg ggggtctctg 60
   agactctcct gtgcagcctc tggattcccc gtcaatcgct atagtatggg gtggtaccgc 120
   caggctccag ggaaggagcg cgagtgggtc gcgggtatga gtagtgctgg tgatcgttca    180
   agttatgaag actccgtgaa gggccgattc accatctcca gagacgacgc caggaatacg    240
   gtgtatctgc aaatgaacag cctgaaacct gaggacacgg ccgtgtatta ctgtaatgtc    300
   aatgtgggct ttgagtactg gggccagggg acccaggtca ccgtctcctc aaggaattcg    360
   atatcaagct tatcgatacc gtcgacggta ccgcgggccc gggatccacc ggtcgacatg    420
   gcctcctccg aggacgtcat caaggagttc atgcgcttca aggtgcgcat ggagggctcc    480
   gtgaacggcc acgagttcga gatcgagggc gagggcgagg gccgccccta cgagggcacc    540
   cagaccgcca agctgaaggt gaccaagggc ggccccctgc ccttcgcctg ggacatcctg    600
   tcccctcagt tccagtacgg ctccaaggcc tacgtgaagc accccgccga catccccgac    660
   tacttgaagc tgtccttccc cgagggcttc aagtgggagc gcgtgatgaa cttcgaggac    720
   ggcggcgtgg tgaccgtgac ccaggactcc tccctgcagg acggcgagtt catctacaag    780
   gtgaagctgc gcggcaccaa cttcccctcc gacggccccg taatgcagaa gaagaccatg    840
   ggctgggagg cctccaccga gcggatgtac cccgaggacg gcgccctgaa gggcgagatc    900
   aagatgaggc tgaagctgaa ggacggcggc cactacgacg ccgaggtcaa gaccacctac    960
   atggccaaga agcccgtgca gctgcccggc gcctacaaga ccgacatcaa gctggacatc   1020
   acctcccaca acgaggacta caccatcgtg gaacagtacg agcgcgccga gggccgccac   1080
   tccaccggcg ccggcctgta caagtaa                                       1107
<210> 20
   <211> 374
   <212> PRT
   <213> artificial sequence
<220>
   <223> anti-cytokeratin-8 chromobody
<400> 20
   Met Ala Gln Val Gln Leu Val Glu Ser Gly Gly Gly Leu Val Gln Pro
   1               5                   10                  15
   Gly Glu Ser Leu Arg Leu Ser Cys Ala Ala Ser Gly Phe Asn Phe Ala
               20                  25                  30
   Ser Tyr Ser Met Thr Trp Val Arg Gln Ala Pro Gly Lys Glu Leu Asp
           35                  40                  45
   Trp Val Ala Thr Ile Ser Phe Ser Gly Gly Ser Thr Tyr Tyr Asp Asp
       50                  55                  60
   Ser Val Lys Gly Arg Ala Thr Ile Ser Arg Asp Asn Ala Lys Ser Thr
   65                  70                  75                  80
   Val Tyr Leu Gln Leu Asn Ser Leu Lys Thr Glu Asp Thr Gly Met Tyr
                   85                  90                  95
   Tyr Cys Thr Lys Gly Arg Val Ser Pro Pro Ile Pro Asp Gly Leu Ser
               100                 105                 110
   Arg Gly Gln Gly Thr Gln Val Thr Val Ser Ser Arg Asn Ser Ile Ser
           115                 120                 125
   Ser Leu Ser Ile Pro Ser Thr Val Pro Arg Ala Arg Asp Pro Pro Val
       130                 135                 140
   Asp Met Ala Ser Ser Glu Asp Val Ile Lys Glu Phe Met Arg Phe Lys
   145                 150                 155                 160
   Val Arg Met Glu Gly Ser Val Asn Gly His Glu Phe Glu Ile Glu Gly
                   165                 170                 175
   Glu Gly Glu Gly Arg Pro Tyr Glu Gly Thr Gln Thr Ala Lys Leu Lys
               180                 185                 190
   Val Thr Lys Gly Gly Pro Leu Pro Phe Ala Trp Asp Ile Leu Ser Pro
           195                 200                 205
   Gln Phe Gln Tyr Gly Ser Lys Ala Tyr Val Lys His Pro Ala Asp Ile
       210                 215                 220
   Pro Asp Tyr Leu Lys Leu Ser Phe Pro Glu Gly Phe Lys Trp Glu Arg
   225                 230                 235                 240
   Val Met Asn Phe Glu Asp Gly Gly Val Val Thr Val Thr Gln Asp Ser
                   245                 250                 255
   Ser Leu Gln Asp Gly Glu Phe Ile Tyr Lys Val Lys Leu Arg Gly Thr
               260                 265                 270
   Asn Phe Pro Ser Asp Gly Pro Val Met Gln Lys Lys Thr Met Gly Trp
           275                 280                 285
   Glu Ala Ser Thr Glu Arg Met Tyr Pro Glu Asp Gly Ala Leu Lys Gly
       290                 295                 300
   Glu Ile Lys Met Arg Leu Lys Leu Lys Asp Gly Gly His Tyr Asp Ala
   305                 310                 315                 320
   Glu Val Lys Thr Thr Tyr Met Ala Lys Lys Pro Val Gln Leu Pro Gly
                   325                 330                 335
   Ala Tyr Lys Thr Asp Ile Lys Leu Asp Ile Thr Ser His Asn Glu Asp
               340                 345                 350
   Tyr Thr Ile Val Glu Gln Tyr Glu Arg Ala Glu Gly Arg His Ser Thr
           355                 360                 365
   Gly Ala Gly Leu Tyr Lys
       370
<210> 21
   <211> 1125
   <212> DNA
   <213> artificial sequence
<220>
   <223> anti-cytokeratin-8 chromobody
<400> 21
   atggctcagg tgcagctggt ggagtctggg ggaggcttgg tgcagcctgg ggagtctctc      60
   agactctcct gtgcagcctc tggattcaac ttcgcaagtt attccatgac ctgggtccgc     120
   caggctccag ggaaggaact cgactgggtc gcaacaataa gtttcagtgg tgggtccact     180
   tactatgatg actccgtgaa gggccgggcc acaatctcca gagacaacgc caagagcaca     240
   gtgtatctcc agttaaacag cctgaaaact gaggacacgg gaatgtatta ctgtacaaaa     300
   ggcagagtat ccccgcccat tcccgatggc ctttctcgag gccaggggac ccaggtcacc     360
   gtctcctcaa ggaattcgat atcaagctta tcgataccgt cgacggtacc gcgggcccgg     420
   gatccaccgg tcgacatggc ctcctccgag gacgtcatca aggagttcat gcgcttcaag     480
   gtgcgcatgg agggctccgt gaacggccac gagttcgaga tcgagggcga gggcgagggc     540
   cgcccctacg agggcaccca gaccgccaag ctgaaggtga ccaagggcgg ccccctgccc     600
   ttcgcctggg acatcctgtc ccctcagttc cagtacggct ccaaggccta cgtgaagcac     660
   cccgccgaca tccccgacta cttgaagctg tccttccccg agggcttcaa gtgggagcgc     720
   gtgatgaact tcgaggacgg cggcgtggtg accgtgaccc aggactcctc cctgcaggac     780
   ggcgagttca tctacaaggt gaagctgcgc ggcaccaact tcccctccga cggccccgta     840
   atgcagaaga agaccatggg ctgggaggcc tccaccgagc ggatgtaccc cgaggacggc     900
   gccctgaagg gcgagatcaa gatgaggctg aagctgaagg acggcggcca ctacgacgcc     960
   gaggtcaaga ccacctacat ggccaagaag cccgtgcagc tgcccggcgc ctacaagacc    1020
   gacatcaagc tggacatcac ctcccacaac gaggactaca ccatcgtgga acagtacgag    1080
   cgcgccgagg gccgccactc caccggcgcc ggcctgtaca agtaa                    1125
<210> 22
   <211> 379
   <212> PRT
   <213> artificial sequence
<220>
   <223> anti-lamin-8 chromobody
<400> 22
   Met Ala Gln Val Gln Leu Gln Glu Ser Gly Gly Gly Leu Val Gln Ala
   1               5                   10                  15
   Gly Gly Ser Leu Thr Leu Ser Cys Thr Tyr Ser Gly Leu Thr Phe Asp
               20                  25                  30
   Asp Tyr Asn Met Gly Trp Phe Arg Gln Gly Pro Gly Lys Glu Arg Glu
           35                  40                  45
   Arg Val Ser Ala Ile Ser Phe Arg Gly Ile Thr Tyr Tyr Val Asp Ser
       50                  55                  60
   Val Lys Gly Arg Phe Thr Ile Ser Arg Asp Asn Ala Lys Lys Thr Leu
   65                  70                  75                  80
   Tyr Leu Gln Met Asn Gly Leu Thr Pro Asp Asp Thr Ala Thr Tyr Tyr
                   85                  90                  95
   Cys Ala Gly Ser Arg Phe Leu Ser Pro Phe Val Arg Asp Gly Asp Thr
               100                 105                 110
   Lys Leu Ile Asn Asp Trp Gly Gln Gly Thr Gln Val Thr Val Ser Ser
           115                 120                 125
   Arg Asn Ser Ile Ser Ser Leu Ser Ile Pro Ser Thr Val Pro Arg Ala
       130                 135                 140
   Arg Asp Pro Pro Val Asp Met Ala Ser Ser Glu Asp Val Ile Lys Glu
   145                 150                 155                 160
   Phe Met Arg Phe Lys Val Arg Met Glu Gly Ser Val Asn Gly His Glu
                   165                 170                 175
   Phe Glu Ile Glu Gly Glu Gly Glu Gly Arg Pro Tyr Glu Gly Thr Gln
               180                 185                 190
   Thr Ala Lys Leu Lys Val Thr Lys Gly Gly Pro Leu Pro Phe Ala Trp
           195                 200                 205
   Asp Ile Leu Ser Pro Gln Phe Gln Tyr Gly Ser Lys Ala Tyr Val Lys
       210                 215                 220
   His Pro Ala Asp Ile Pro Asp Tyr Leu Lys Leu Ser Phe Pro Glu Gly
   225                 230                 235                 240
   Phe Lys Trp Glu Arg Val Met Asn Phe Glu Asp Gly Gly Val Val Thr
                   245                 250                 255
   Val Thr Gln Asp Ser Ser Leu Gln Asp Gly Glu Phe Ile Tyr Lys Val
               260                 265                 270
   Lys Leu Arg Gly Thr Asn Phe Pro Ser Asp Gly Pro Val Met Gln Lys
           275                 280                 285
   Lys Thr Met Gly Trp Glu Ala Ser Thr Glu Arg Met Tyr Pro Glu Asp
       290                 295                 300
   Gly Ala Leu Lys Gly Glu Ile Lys Met Arg Leu Lys Leu Lys Asp Gly
   305                 310                 315                 320
   Gly His Tyr Asp Ala Glu Val Lys Thr Thr Tyr Met Ala Lys Lys Pro
                   325                 330                 335
   Val Gln Leu Pro Gly Ala Tyr Lys Thr Asp Ile Lys Leu Asp Ile Thr
               340                 345                 350
   Ser His Asn Glu Asp Tyr Thr Ile Val Glu Gln Tyr Glu Arg Ala Glu
           355                 360                 365
   Gly Arg His Ser Thr Gly Ala Gly Leu Tyr Lys
       370                 375
<210> 23
   <211> 1140
   <212> DNA
   <213> artificial sequence
<220>
   <223> anti-lamin-8 chromobody
<400> 23
   atggcccagg tgcagctgca ggagtctgga ggaggcttgg tgcaggcagg ggggtctctg     60
   acactctcct gtacatactc tggcttgact tttgatgatt ataacatggg ctggttccgc    120
   cagggtccag ggaaggagcg cgagagagtt tcggcgatta gctttcgtgg catcacctat    180
   tatgtagact ccgttaaggg ccgattcacc atctccaggg ataacgccaa gaagacgctg    240
   tatctgcaaa tgaacggcct gacacctgac gatacggcca cgtattactg tgcgggtagt    300
   cgcttcttaa gtccctttgt acgcgatggt gacacgaaat tgattaacga ttggggccag    360
   gggacccagg tcaccgtctc ctcaaggaat tcgatatcaa gcttatcgat accgtcgacg    420
   gtaccgcggg cccgggatcc accggtcgac atggcctcct ccgaggacgt catcaaggag    480
   ttcatgcgct tcaaggtgcg catggagggc tccgtgaacg gccacgagtt cgagatcgag 540
   ggcgagggcg agggccgccc ctacgagggc acccagaccg ccaagctgaa ggtgaccaag 600
   ggcggccccc tgcccttcgc ctgggacatc ctgtcccctc agttccagta cggctccaag 660
   gcctacgtga agcaccccgc cgacatcccc gactacttga agctgtcctt ccccgagggc 720
   ttcaagtggg agcgcgtgat gaacttcgag gacggcggcg tggtgaccgt gacccaggac 780
   tcctccctgc aggacggcga gttcatctac aaggtgaagc tgcgcggcac caacttcccc 840
   tccgacggcc ccgtaatgca gaagaagacc atgggctggg aggcctccac cgagcggatg 900
   taccccgagg acggcgccct gaagggcgag atcaagatga ggctgaagct gaaggacggc 960
   ggccactacg acgccgaggt caagaccacc tacatggcca agaagcccgt gcagctgccc 1020
   ggcgcctaca agaccgacat caagctggac atcacctccc acaacgagga ctacaccatc 1080
   gtggaacagt acgagcgcgc cgagggccgc cactccaccg gcgccggcct gtacaagtaa 1140
<210> 24
   <211> 702
   <212> DNA
   <213> artificial sequence
<220>
   <223> mRFPmars
<400> 24
   atggcatcat cagaagatgt tattaaagaa tttatgagat tcaaagttaa aatggaaggt 60
   tcagttaacg gtcatgaatt tgaaattgaa ggtgaaggtg aaggtagacc atatgaaggt 120
   acacaaacag caaaattaaa agttacaaaa ggtggtccat taccatttgc atgggatatt 180
   ctttcaccac aatttcaata tggtagtaaa gcatatgtta aacatccagc agatattcca 240
   gattatctta aactttcatt tccagaaggt tttaaatggg aaagaattat gaattttgaa 300
   gatggtggtg ttgtaacagt tactcaagat tcaacattac aagatggtga atttatttat 360
   aaagttaaat taagaggtac caattttcca tcagatggtc cagtaatgca aaaaaaaact 420
   atgggttggg aagcatcaac agaaagaatg tatccagaag atggtgcact aaaaggtgaa 480
   attaaaatga gattaaaact taaggatggt ggtcattatg atgcagaagt taaaacaact 540
   tataaagcta aaaaacaagt tcaattacca ggtgcttata aaacagatat taaattagat 600
   attatttcac ataatgaaga ttatactatt gttgaacaat atgaaagagc agaaggtaga 660
   cattcaacag gtgcaggatc ccatcatcac caccatcatt aa 702
<210> 25
   <211> 711
   <212> DNA
   <213> artificial sequence
<220>
   <223> mCherry
<400> 25
   atggtgagca agggcgagga ggataacatg gccatcatca aggagttcat gcgcttcaag      60
   gtgcacatgg agggctccgt gaacggccac gagttcgaga tcgagggcga gggcgagggc     120
   cgcccctacg agggcaccca gaccgccaag ctgaaggtga ccaagggtgg ccccctgccc     180
   ttcgcctggg acatcctgtc ccctcagttc atgtacggct ccaaggccta cgtgaagcac     240
   cccgccgaca tccccgacta cttgaagctg tccttccccg agggcttcaa gtgggagcgc     300
   gtgatgaact tcgaggacgg cggcgtggtg accgtgaccc aggactcctc cctgcaggac     360
   ggcgagttca tctacaaggt gaagctgcgc ggcaccaact tcccctccga cggccccgta     420
   atgcagaaga agaccatggg ctgggaggcc tcctccgagc ggatgtaccc cgaggacggc     480
   gccctgaagg gcgagatcaa gcagaggctg aagctgaagg acggcggcca ctacgacgct     540
   gaggtcaaga ccacctacaa ggccaagaag cccgtgcagc tgcccggcgc ctacaacgtc     600
   aacatcaagt tggacatcac ctcccacaac gaggactaca ccatcgtgga acagtacgaa     660
   cgcgccgagg gccgccactc caccggcggc atggacgagc tgtacaagta a              711
<210> 26
   <211> 681
   <212> DNA
   <213> artificial sequence
<220>
   <223> MRaspberry
<400> 26
   atggtgagca agggcgagga ggtcatcaag gagttcatgc gcttcaaggt gcgcatggag      60
   ggctccgtga acggccacga gttcgagatc gagggcgagg gcgagggccg cccctacgag     120
   ggcacccaga ccgccaagct gaaggtgacc aagggtggcc ccctgccctt cgcctgggac     180
   atcctgtccc ctcagtgcat gtacggctcc aagggctacg tgaagcaccc cgccgacatc     240
   cccgactact tgaagctgtc cttccccgag ggcttcaagt gggagcgcgt gatgaacttc     300
   gaggacggcg gcgtggtgac cgtgacccag gactcctccc tgcaggacgg cgagttcatc     360
   tacaaggtga agctgcgcgg caccaacttc ccctccgacg gccccgtaat gcagaagaag     420
   accatgggct gggaggcctc ctccgagcgg atgtaccccg aggacggcgc cctgaagggc     480
   gagatgaaga tgaggctgaa gctgaaggac ggcggccact acgacgccga ggtcaagacc     540
   acctacatgg ccaagaagcc cgtgcagctg cccggcgcct acaagaccga catcaagctg     600
   gacatcacct cccacaacga ggactacacc atcgtggaac agtacgagcg cgccgagggc     660
   cgccactcca ccggcgccta a                                               681
<210> 27
   <211> 681
   <212> DNA
   <213> artificial sequence
<220>
   <223> mPlum
<400> 27
   atggtgagca agggcgagga ggtcatcaag gagttcatgc gcttcaagga gcacatggag     60
   ggctccgtga acggccacga gttcgagatc gagggcgagg gcgagggccg cccctacgag    120
   ggcacccaga ccgccaggct gaaggtgacc aagggtggcc ccctgccctt cgcctgggac    180
   atcctgtccc ctcagatcat gtacggctcc aaggcctacg tgaagcaccc cgccgacatc    240
   cccgactact tgaagctgtc cttccccgag ggcttcaagt gggagcgcgt gatgaacttc    300
   gaggacggcg gcgtggtgac cgtgacccag gactcctccc tgcaggacgg cgagttcatc    360
   tacaaggtga aggtgcgcgg caccaacttc ccctccgacg gccccgtaat gcagaagaag    420
   accatgggct gggaggcctc ctccgagcgg atgtaccccg aggacggcgc cctgaagggc    480
   gagatgaaga tgaggctgag gctgaaggac ggcggccact acgacgccga ggtcaagacc    540
   acctacatgg ccaagaagcc cgtgcagctg cccggcgcct acaagaccga catcaagctg    600
   gacatcacct cccacaacga ggactacacc atcgtggaac agtacgagcg cgccgagggc    660
   cgccactcca ccggcgccta a                                              681
<210> 28
   <211> 117
   <212> PRT
   <213> artificial sequence
<220>
   <223> generic variable domain
<220>
   <221> misc feature
   <222> (29)..(37)
   <223> Xaa can be any naturally occurring amino acid
<220>
   <221> misc feature
   <222> (52)..(68)
   <223> Xaa can be any naturally occurring amino acid
<220>
   <221> misc feature
   <222> (101)..(106)
   <223> Xaa can be any naturally occurring amino acid
<400> 28
   Met Ala Asp Val Gln Leu Val Glu Ser Gly Gly Ala Leu Val Gln Pro
   1               5                   10                  15
   Gly Gly Ser Leu Arg Leu Ser Cys Ala Ala Ser Gly Xaa Xaa Xaa Xaa
               20                  25                  30
   Xaa Xaa Xaa Xaa Xaa Trp Tyr Arg Gln Ala Pro Gly Lys Glu Arg Glu
           35                  40                  45
   Trp Val Ala Xaa Xaa Xaa Xaa Xaa Xaa Xaa Xaa Xaa Xaa Xaa Xaa Xaa
       50                  55                  60
   Xaa Xaa Xaa Xaa Arg Phe Thr Ile Ser Arg Asp Asp Ala Arg Asn Thr
   65                  70                  75                  80
   Val Tyr Leu Gln Met Asn Ser Leu Lys Pro Glu Asp Thr Ala Val Tyr
                   85                  90                  95
   Tyr Ser Asn Val Xaa Xaa Xaa Xaa Xaa Xaa Trp Gly Gln Gly Thr Gln
               100                 105                 110
   Val Thr Val Ser Ser
           115
<210> 29
   <211> 351
   <212> DNA
   <213> artificial sequence
<220>
   <223> generic variable domain, a coding sequence for SEQ ID NO: 28
<220>
   <221> misc_feature
   <222> (85)..(111)
   <223> n is a, c, g, or t
<220>
   <221> misc_feature
   <222> (154)..(204)
   <223> n is a, c, g, or t
<220>
   <221> misc_feature
   <222> (301)..(318)
   <223> n is a, c, g, or t
<400> 29
   atggccgatg tgcagctggt ggagtctggg ggagccttgg tgcagccggg ggggtctctg     60
   agactctcct gtgcagcctc tggannnnnn nnnnnnnnnn nnnnnnnnnn ntggtaccgc    120
   caggctccag ggaaggagcg cgagtgggtc gcgnnnnnnn nnnnnnnnnn nnnnnnnnnn    180
   nnnnnnnnnn nnnnnnnnnn nnnncgattc accatctcca gagacgacgc caggaatacg    240
   gtgtatctgc aaatgaacag cctgaaacct gaggacacgg ccgtgtatta ctcgaatgtc    300
   nnnnnnnnnn nnnnnnnntg gggccagggg acccaggtca ccgtctcctc a              351
<210> 30
   <211> 117
   <212> PRT
   <213> artificial sequence
<220>
   <223> VHH fragment of fluorescent chromobody against GFP (mutant C92S)
<400> 30
   Met Ala Asp Val Gln Leu Val Glu Ser Gly Gly Ala Leu Val Gln Pro
   1               5                   10                  15
   Gly Gly Ser Leu Arg Leu Ser Cys Ala Ala Ser Gly Phe Pro Val Asn
               20                  25                  30
   Arg Tyr Ser Met Arg Trp Tyr Arg Gln Ala Pro Gly Lys Glu Arg Glu
           35                  40                  45
   Trp Val Ala Gly Met Ser Ser Ala Gly Asp Arg Ser Ser Tyr Glu Asp
       50                  55                  60
   Ser Val Lys Gly Arg Phe Thr Ile Ser Arg Asp Asp Ala Arg Asn Thr
   65                  70                  75                  80
   Val Tyr Leu Gln Met Asn Ser Leu Lys Pro Glu Asp Thr Ala Val Tyr
                   85                  90                  95
   Tyr Ser Asn Val Asn Val Gly Phe Glu Tyr Trp Gly Gln Gly Thr Gln
               100                 105                 110
   Val Thr Val Ser Ser
           115
<210> 31
   <211> 351
   <212> DNA
   <213> artificial sequence
<220>
   <223> sequence encoding VHH fragment of fluorescent chromobody of SEQ ID NO: 30
<400> 31
   atggccgatg tgcagctggt ggagtctggg ggagccttgg tgcagccggg ggggtctctg    60
   agactctcct gtgcagcctc tggattcccc gtcaatcgct atagtatgag gtggtaccgc   120
   caggctccag ggaaggagcg cgagtgggtc gcgggtatga gtagtgctgg tgatcgttca   180
   agttatgaag actccgtgaa gggccgattc accatctcca gagacgacgc caggaatacg   240
   gtgtatctgc aaatgaacag cctgaaacct gaggacacgg ccgtgtatta ctcgaatgtc   300
   aatgtgggct ttgagtactg gggccagggg acccaggtca ccgtctcctc a            351
<210> 32
   <211> 368
   <212> PRT
   <213> artificial sequence
<220>
   <223> anti-GFP chromobody (mutant C92S)
<400> 32
   Met Ala Asp Val Gln Leu Val Glu Ser Gly Gly Ala Leu Val Gln Pro
   1               5                   10                  15
   Gly Gly Ser Leu Arg Leu Ser Cys Ala Ala Ser Gly Phe Pro Val Asn
               20                  25                  30
   Arg Tyr Ser Met Arg Trp Tyr Arg Gln Ala Pro Gly Lys Glu Arg Glu
           35                  40                  45
   Trp Val Ala Gly Met Ser Ser Ala Gly Asp Arg Ser Ser Tyr Glu Asp
       50                  55                  60
   Ser Val Lys Gly Arg Phe Thr Ile Ser Arg Asp Asp Ala Arg Asn Thr
   65                  70                  75                  80
   Val Tyr Leu Gln Met Asn Ser Leu Lys Pro Glu Asp Thr Ala Val Tyr
                   85                  90                  95
   Tyr Ser Asn Val Asn Val Gly Phe Glu Tyr Trp Gly Gln Gly Thr Gln
               100                 105                 110
   Val Thr Val Ser Ser Arg Asn Ser Ile Ser Ser Leu Ser Ile Pro Ser
           115                 120                 125
   Thr Val Pro Arg Ala Arg Asp Pro Pro Val Asp Met Ala Ser Ser Glu
       130                 135                 140
   Asp Val Ile Lys Glu Phe Met Arg Phe Lys Val Arg Met Glu Gly Ser
   145                 150                 155                 160
   Val Asn Gly His Glu Phe Glu Ile Glu Gly Glu Gly Glu Gly Arg Pro
                   165                 170                 175
   Tyr Glu Gly Thr Gln Thr Ala Lys Leu Lys Val Thr Lys Gly Gly Pro
               180                 185                 190
   Leu Pro Phe Ala Trp Asp Ile Leu Ser Pro Gln Phe Gln Tyr Gly Ser
           195                 200                 205
   Lys Ala Tyr Val Lys His Pro Ala Asp Ile Pro Asp Tyr Leu Lys Leu
       210                 215                 220
   Ser Phe Pro Glu Gly Phe Lys Trp Glu Arg Val Met Asn Phe Glu Asp
   225                 230                 235                 240
   Gly Gly Val Val Thr Val Thr Gln Asp Ser Ser Leu Gln Asp Gly Glu
                   245                 250                 255
   Phe Ile Tyr Lys Val Lys Leu Arg Gly Thr Asn Phe Pro Ser Asp Gly
               260                 265                 270
   Pro Val Met Gln Lys Lys Thr Met Gly Trp Glu Ala Ser Thr Glu Arg
           275                 280                 285
   Met Tyr Pro Glu Asp Gly Ala Leu Lys Gly Glu Ile Lys Met Arg Leu
       290                 295                 300
   Lys Leu Lys Asp Gly Gly His Tyr Asp Ala Glu Val Lys Thr Thr Tyr
   305                 310                 315                 320
   Met Ala Lys Lys Pro Val Gln Leu Pro Gly Ala Tyr Lys Thr Asp Ile
                   325                 330                 335
   Lys Leu Asp Ile Thr Ser His Asn Glu Asp Tyr Thr Ile Val Glu Gln
               340                 345                 350
   Tyr Glu Arg Ala Glu Gly Arg His Ser Thr Gly Ala Gly Leu Tyr Lys
           355                 360                 365
<210> 33
   <211> 1107
   <212> DNA
   <213> artificial sequence
<220>
   <223> sequence encoding anti-GFP chromobody (mutant C92S)
<400> 33
   atggccgatg tgcagctggt ggagtctggg ggagccttgg tgcagccggg ggggtctctg     60
   agactctcct gtgcagcctc tggattcccc gtcaatcgct atagtatgag gtggtaccgc    120
   caggctccag ggaaggagcg cgagtgggtc gcgggtatga gtagtgctgg tgatcgttca    180
   agttatgaag actccgtgaa gggccgattc accatctcca gagacgacgc caggaatacg    240
   gtgtatctgc aaatgaacag cctgaaacct gaggacacgg ccgtgtatta ctcgaatgtc    300
   aatgtgggct ttgagtactg gggccagggg acccaggtca ccgtctcctc aaggaattcg    360
   atatcaagct tatcgatacc gtcgacggta ccgcgggccc gggatccacc ggtcgacatg    420
   gcctcctccg aggacgtcat caaggagttc atgcgcttca aggtgcgcat ggagggctcc    480
   gtgaacggcc acgagttcga gatcgagggc gagggcgagg gccgccccta cgagggcacc    540
   cagaccgcca agctgaaggt gaccaagggc ggccccctgc ccttcgcctg ggacatcctg    600
   tcccctcagt tccagtacgg ctccaaggcc tacgtgaagc accccgccga catccccgac    660
   tacttgaagc tgtccttccc cgagggcttc aagtgggagc gcgtgatgaa cttcgaggac    720
   ggcggcgtgg tgaccgtgac ccaggactcc tccctgcagg acggcgagtt catctacaag    780
   gtgaagctgc gcggcaccaa cttcccctcc gacggccccg taatgcagaa gaagaccatg     840
   ggctgggagg cctccaccga gcggatgtac cccgaggacg gcgccctgaa gggcgagatc    900
   aagatgaggc tgaagctgaa ggacggcggc cactacgacg ccgaggtcaa gaccacctac    960
   atggccaaga agcccgtgca gctgcccggc gcctacaaga ccgacatcaa gctggacatc   1020
   acctcccaca acgaggacta caccatcgtg gaacagtacg agcgcgccga gggccgccac   1080
   tccaccggcg ccggcctgta caagtaa                                       1107

## Description

The present invention relates to the embodiment as characterized in the claims specifically, the present invention relates to a fusion protein comprising a first (poly)peptide sequence comprising the variable region of a heavy chain antibody of *Camelidae* (including any camel or dromedary) and a second (poly)peptide sequence, which is a detectable protein, preferably derivable from a detectable, e.g. fluorescent or chromophoric or phosphorescent protein, wherein said (a) first (poly)peptide sequence is encoded by the nucleic acid sequence of SEQ ID NO: 11 or 31; and (b) second (poly)peptide sequence, if derived from a fluorescent or chromophoric protein, may be (i) the green fluorescent protein derivable from *Aequorea victoria* encoded by the nucleic acid sequence of SEQ ID NO: 7, or a fluorescent mutant or fragment thereof; (ii) the red fluorescent protein derivable from *Discosoma* (DsRed) encoded by the nucleic acid sequence of SEQ ID NO: 9, or a fluorescent mutant or fragment thereof; or (iii) a functional homologue of (i) or (ii) with at least 80% sequence identity; wherein said first (poly)peptide sequence is located N-terminally of said second (poly)peptide sequence, said sequences being optionally separated by a linker of at least one amino acid residues. The present specification also describes a method to purify an antigenic structure of interest, comprising a) contacting a sample containing said antigenic structure with I. a fusion protein directed to said antigenic structure, wherein said fusion protein comprises a first (poly)peptide sequence comprising the variable region of a heavy chain antibody of *Camelidae* and a second (poly)peptide sequence, which is a detectable protein, preferably derivable from a fluorescent or chromophoric protein, wherein said (1.) first (poly)peptide sequence is composed of framework 1, CDR1, framework 2, CDR2, framework 3 and CDR3, encoded by the nucleic acid sequence of SEQ ID NO: 2 or encoded by a nucleic acid sequence with at least 70% sequence identity or a fragment thereof; and (2.) second (poly)peptide sequence is a detectable protein, preferably (i) the green fluorescent protein derivable from *Aequorea victoria* encoded by the nucleic acid sequence of SEQ ID NO: 7, or a fluorescent mutant or fragment thereof; (ii) the red fluorescent protein derivable from *Discosoma* (DsRed) encoded by the nucleic acid sequence of SEQ ID NO: 9, or a fluorescent mutant or fragment thereof; or (iii) a functional homologue of (i) or (ii) with at least 80% sequence identity; wherein said first (poly)peptide sequence is located N-terminally or C-terminally of said second (poly)peptide sequence, said sequences being optionally separated by a linker of at least one amino acid residue, or II. a (poly)peptide comprising the variable region of a heavy chain antibody of Camelidae, composed of framework 1, CDR1, framework 2, CDR2, framework 3 and CDR3, encoded by the nucleic acid sequence of SEQ ID NO: 2 or encoded by a nucleic acid sequence with at least 70% sequence identity or a fragment thereof; wherein the fusion protein is attached to a solid support; b) optionally washing the solid support of step a) to remove unspecifically bound constituents; c) eluting the antigenic structure.

All or any combination of steps (including single steps only) carried out in the method of the present invention and cited throughout this specification can be carried out in any combination of in vivo, ex vivo or in vitro.

Antibodies are valuable tools to identify and visualize cellular structures. Unfortunately, the application of naturally occurring antibodies for the detection of intracellular antigens requires permeabilization (and often fixation) of cells. Moreover, the antibody-based detection of antigens within *intact* cells is essentially prevented by the fact that they are, by nature, designed to function in an oxidizing (extracellular) environment: the reducing environment in the cytoplasm leads to an in impaired disulfide bond formation, resulting in an inefficient assembly of epitope recognizing parts of the variable light and heavy chain^{1, 2}. In only a few cases intracellular antibodies (ICAbs) have been used to affect protein function *in vivo* but still little is known about their properties in living cells³⁻⁷.

In an attempt to avoid the problems associated with the application of antibodies in the cytoplasm of intact cells, protein expression has in the past been studied by fusing proteins of interest to fluorescent proteins, usually GFP ("GFP-tagging"). GFP-tagging has become an extremely popular method to study intracellular trafficking of proteins and, in combination with fluorescence photobleaching techniques, has provided unique information on protein dynamics in living cells. However, only the dynamics of chimeric proteins can be measured, whereas the authentic proteins, their posttranslational modification as well as non-proteinaceous components of the cell cannot be assessed by the available methods. To overcome these limitations, it would be desirable to generate detectable protein binders which avoid the problems and limitations of naturally occurring antibodies and to establish their application in the living cell preferably avoiding interference with cellular processes.

Thus, the technical problem underlying the present invention was to provide novel methods and compounds allowing the intracellular detection of antigens in intact cells. The solution to this technical problem is achieved by providing the embodiments characterized in the claims.

Described is a method of detecting the presence, amount or subcellular location of an antigenic structure of interest in a cell, comprising the steps of: (a) (i) expressing a fusion protein directed to the antigenic structure of interest in said cell or (ii) introducing a fusion protein directed to the antigenic structure of interest and coupled to a (poly)peptide capable of transducing into said cell; wherein said fusion protein comprises a first (poly)peptide sequence comprising the variable region of a heavy chain antibody of *Camelidae* and a second (poly)peptide sequence derivable from a fluorescent or chromophoric protein, wherein said (1.) first (poly)peptide sequence is composed of framework 1, CDR1, framework 2, CDR2, framework 3 and CDR3, of a Camelidae antibody and preferably , encoded by the nucleic acid sequence of SEQ ID NO: 2 or encoded by a nucleic acid sequence with at least 70% identity or a fragment thereof and a second polypeptide, which is a detectable (poly)peptide (such as a tag) and preferably derivable from a fluorescent or chromophoric protein, wherein said (1.) first (poly)peptide sequence is composed of framework 1, CDR1, framework 2, CDR2, framework 3 and CDR3, encoded by the nucleic acid sequence of a *Camelidae* antibody, preferably of SEQ ID NO: 7, or a fluorescent mutant or fragment thereof; b. the red fluorescent protein derivable from *Discosoma* (DsRed) encoded by the nucleic acid sequence of SEQ ID NO: 9, or a fluorescent mutant or fragment thereof; or c. a functional homologue of a. or b. with at least 80% sequence identity; (b) revealing the presence, amount as subcellular location of said antigenic structure of interest, if any, in said cell by means of said detectable protein; wherein said first (poly)peptide sequence is located N-terminally of said second (poly)peptide sequence, said sequences being optionally separated by a linker of at least one amino acid residue.

The term "subcellular location" or "distribution" refers to the presence of a compound, e.g. a protein or an antigen, within the cell, on the cell or related/connected to the cell.

The term "antigenic structure" or "antigen" refers to any kind of compound capable of inducing an immune response. An antigen may be a small molecular compound or a macromolecule such as a protein or nucleic acid molecule or fragments thereof as well as combinations of large molecules and small molecules coupled e.g. by chemical linkage. In particular, said fragments may comprise or consist of e.g. posttranslational modifications such as phosphorylation, methylation or glycosylation. In the context of the present invention the terms are used to describe cellular targets of the fusion proteins used to carry out the method of the invention. The antibody part, which is derived from Camelidae antibodies can be directed to any structure, against which antibodies can be raised.

In accordance with the present invention said fusion protein can be prepared by a method of generating a detectable protein capable of binding an antigen of interest, comprising the steps of: (a) obtaining from an antibody producing cell of *Camelidae* or a pool of such cells, a first nucleic acid molecule or a pool of such nucleic acid molecules, encoding the variable region of an immunoglobulin or recombinantly or (semi)synthetically producing such first nucleic acid molecule or pool of first nucleic acid molecules; (b) optionally selecting from said pool a particular nucleic acid molecule encoding the variable region of a specific immunoglobulin; (c) fusing the coding region of the first nucleic acid molecule, encoding the variable region of an immunoglobulin in frame to the coding region of a second nucleic acid molecule, encoding a detectable marker (poly)peptide, wherein the coding region of the first nucleic acid molecule is located 5' of the coding region of the second nucleic acid molecule and wherein the coding regions are optionally separated by a coding region encoding a linker of at least one amino acid residue; and (d) expressing the fused nucleic acid molecule encoding the fusion protein in a cell or cell free extract.

Throughout the present invention the term "directed to" is used to describe the property of a fusion protein or (poly)peptide as mentioned in this specification to specifically bind to an antigenic structure. This specific binding excludes or essentially excludes binding of structurally closely related antigens commonly known in the art as cross-reactivity. Specific binding can be measured, for example, in ELISA tests or other immunological tests where the antigenic structure of interest is bound to the well of an ELISA plate and structurally related antigens are bound to different wells of this ELISA plate. If the fusion protein binds to only the antigenic structure (antigen) of interest, there will be no signal beyond background level or essentially beyond background level (i.e. at the most 25% above background level) when binding for the related antigens is assessed.

The term "(poly)peptide capable of transducing into said cell" refers to the ability of certain proteins to transduce, i.e. translocate, from the outside of a cell to the inside. The mechanism is still largely unknown. The (poly)peptides capable of transducing are mostly basic. The fusion protein will be translocated into the cell together with the transducing molecule.

The term "(poly)peptide" refers alternatively to peptides or to polypeptides. Peptides conventionally are covalently linked amino acids of up to 30 residues, whereas polypeptides (also referred to herein as "proteins") comprise 31 and more amino acid residues.

The terms "detectable protein" and "detectable (poly)peptide" and "detectable marker (poly)peptide" refer to (poly)peptides which are detectable by visual means or give rise to a product detectable by visual means (such as is the case with enzymes). For example, upon excitation or by cleavage of a chromogenic substrate, such proteins/(poly)peptides result in the emission of detectable energy or colour. In this sense, the protein is to be understood as a detectably labelled protein, corresponding to the second (poly)peptide (being the label). In a preferred embodiment of the present invention said detectable proteins or detectable marker (poly)peptides are fluorescent, phosphorescent or chromophoric (poly)peptides. In fact, any (poly)peptide which results, after exposure to an excitation energy, in the emission of detectable energy, is understood in accordance with the present invention as a detectable marker. In a particular embodiment of the present invention, the energy emitted from a first protein may be transferred to a second (different) detectable protein which is also excitable and which can emit the energy obtained from the first detectable protein. One example of such energy resonance is the FRET system, which may be adapted according to the teaching of the present invention. The term "energy emitted ..." preferably refers to fluorescence, phosphorescence or the emission of light with a particular wavelength which is detectable.

The term "derivable" throughout the present invention does also encompass the term "derived".

The term "antibody producing cell of *Camelidae*" refers to any cell of *Camelidae* capable of producing antibodies. Such cells include cells of the lymphatic system such as spleen cells. In particular, antibody producing cells may be obtainable or derived from B-cells. Preferably, the first nucleic acid molecule is obtained from peripheral blood lymphocytes (PBLs). However, since the complete genetic information is present in any cell of the organism, at least on a theoretical level, the first nucleic acid molecule may also be derived from non-lymphatic cells. Even though the teaching of the present invention is preferably performed by using cells which have undergone somatic recombination to generate intact V-region exons, it is noteworthy, that an intact V-region exon may also be generated by recombinant DNA techniques. The term Camelidae refers to the family of camelidae including the genus Camelus comprising the species Camelus bactrianus (bactrian camel) and Camelus dromedarius (dromedary), the genus Lama comprising the species Lama glama (llama), Lama guanicoe (guanaco) and Lama pacos (alpaca) and the genus Vicugna comprising the species Vicugna vicugna (vicuna). Preferred Camelidae include Camelus bactrianus, Camelus dromedarius and Lama pacos

The term "nucleic acid molecule" as used throughout the specification of the present invention refers to DNA or RNA, including genomic DNA, cDNA, mRNA, hnRNA.

The term "obtaining from ... a first nucleic acid molecule" refers to the use of the nucleic acid molecule encoding the variable region of the immunoglobulin. One example of obtaining a nucleic acid molecule is the physical isolation of said nucleic acid molecule from a cell. Said nucleic acid molecule may e.g. be obtained using standard cloning and screening procedures, such as those for cloning cDNAs using mRNA as starting material.

Another example of obtaining a nucleic acid molecule involves nucleic acid amplification. In fact, in many cases a first step of physically isolating the nucleic acid molecule may precede a second step of nucleic acid amplification. In case the nucleic acid molecule is an RNA molecule such as hnRNA or mRNA, the nucleotide sequence of said molecule may be reverse transcribed into DNA.

The term "amplification" or "amplify" means increase in copy number. The person skilled in the art know various methods to amplify nucleic acid molecules, these methods may also be used in the present invention's methods. Amplification methods include, but are not limited to, "polymerase chain reaction" (PCR), "ligase chain reaction" (LCR, EPA320308), "cyclic probe reaction" (CPR), "strand displacement amplification" (SDA, Walker et al. 1992, Nucleic Acid Res. 7: 1691-1696), "transcription based amplification systems" (TAS, Kwoh et al. 1989, Proc. Nat. Acad. Sci. USA 86: 1173; Gingeras et al., PCT Application WO 88/10315). Preferably, amplification of DNA is accomplished by using polymerase chain reaction (PCR) [Methods in Molecular Biology, Vol. 226 (Bartlett J. M. S. & Stirling D., eds.): PCR protocols, 2nd edition; PCR Technology: Principles and Applications for DNA Amplification (Erlich H. A., ed.), New York 1992; PCR Protocols: A guide to methods and applications (Innis M. A. et al., eds.), Academic Press, San Diego 1990]. Nucleic acid amplification methods may be particularly useful in cases when the sample contains only minute amounts of nucleic acid. If said nucleic acid is RNA, an RT-PCR might be performed. Subsequently, another amplification step involving PCR may be performed. Alternatively, if said nucleic acid contained in the sample is DNA, PCR may be performed.

The PCR, generally, consists of many repetitions of a cycle which consists of: (a) a denaturing step, which melts both strands of a DNA molecule; (b) an annealing step, which is aimed at allowing the primers to anneal specifically to the melted strands of the DNA molecule; and (c) an extension step, which elongates the annealed primers by using the information provided by the template strand. Generally, PCR can be performed for example in a 50 µl reaction mixture containing 5 µl of 10 x PCR buffer with 1.5 mM MgCl₂, 200 µM of each deoxynucleoside triphosphate, 0.5 µl of each primer (10 µM), about 10 to 100ng of template DNA and 1 to 2.5 units of Taq Polymerase. The primers for the amplification may be labeled or be unlabeled. DNA amplification can be performed, e.g., with a model 2400 thermal cycler (Applied Biosystems, Foster City, CA): 2 min at 94°C, followed by 35 cycles consisting of annealing (30 s at 50°C), extension (1 min at 72°C), denaturing (10 s at 94°C) and a final annealing step at 55°C for 1 min as well as a final extension step at 72°C for 5 min. However, the person skilled in the art knows how to optimize these conditions for the amplification of specific nucleic acid molecules or to scale down or increase the volume of the reaction mix.

A further method of nucleic acid amplification is the "reverse transcriptase polymerase chain reaction" (RT-PCR). This method is used when the nucleic acid to be amplified consists of RNA. The term "reverse transcriptase" refers to an enzyme that catalyzes the polymerization of deoxyribonucleoside triphosphates to form primer extension products that are complementary to a ribonucleic acid template. The enzyme initiates synthesis at the 3'-end of the primer and proceeds toward the 5'-end of the template until synthesis terminates. Examples of suitable polymerizing agents that convert the RNA target sequence into a complementary, copy-DNA (cDNA) sequence are avian myeloblastosis virus reverse transcriptase and Thermus thermophilus DNA polymerase, a thermostable DNA polymerase with reverse transcriptase activity marketed by Perkin Elmer. Typically, the genomic RNA/cDNA duplex template is heat denatured during the first denaturation step after the initial reverse transcription step leaving the DNA strand available as an amplification template. Suitable polymerases for use with a DNA template include, for example, E. coli DNA polymerase I or its Klenow fragment, T4 DNA polymerase, Tth polymerase, and Taq polymerase, a heat-stable DNA polymerase isolated from Thermus aquaticus and developed and manufactured by Hoffmann-La Roche and commercially available from Perkin Elmer. The latter enzyme is widely used in the amplification and sequencing of nucleic acids. The reaction conditions for using Taq polymerase are known in the art and are described, e.g., in: PCR Technology, Erlich, H. A. 1989, Stockton Press, New York; or in: Innis, M. A., D. H. Gelfand, J. J. Sninsky, and T. J. White. 1990, PCR Protocols: A guide to methods and applications. Academic Press, New York. High-temperature RT provides greater primer specificity and improved efficiency. U.S. patent application Serial No. 07/746, 121, filed Aug. 15, 1991, describes a "homogeneous RT-PCR" in which the same primers and polymerase suffice for both the reverse transcription and the PCR amplification steps, and the reaction conditions are optimized so that both reactions occur without a change of reagents. Thermus thermophilus DNA polymerase, a thermostable DNA polymerase that can function as a reverse transcriptase, can be used for all primer extension steps, regardless of template. Both processes can be done without having to open the tube to change or add reagents; only the temperature profile is adjusted between the first cycle (RNA template) and the rest of the amplification cycles (DNA template). The RT Reaction can be performed, for example, in a 20µl reaction mix containing: 4 µl of 5x ANV-RT buffer, 2 µl of Oligo dT (100 µg/ml), 2µl of 10 mM dNTPs, 1µl total RNA, 10 Units of AMV reverse transcriptase, and H₂O to 20µl final volume. The reaction may be, for example, performed by using the following conditions: The reaction is held at 70 C° for 15 minutes to allow for reverse transcription. The reaction temperature is then raised to 95 C° for 1 minute to denature the RNA-cDNA duplex. Next, the reaction temperature undergoes two cycles of 95°C for 15 seconds and 60 C° for 20 seconds followed by 38 cycles of 90 C° for 15 seconds and 60 C° for 20 seconds. Finally, the reaction temperature is held at 60 C° for 4 minutes for the final extension step, cooled to 15 C°, and held at that temperature until further processing of the amplified sample. Any of the above mentioned reaction conditions may be scaled up according to the needs of the particular case.

The term "a pool of such nucleic acid molecules" refers to two or more nucleic acid molecules which may be obtained from a plurality of cells, preferably simultaneously. These molecules may be identical in sequence or may be differ. In a preferred embodiment of the present invention, the nucleic acid molecules contained in such a pool of nucleic acid molecules only differ in the codons of the so called "complementary determining region" (CDR) which form a crucial region within the binding pocket of the immunoglobulin, which is responsible for determining its antigen specificity.

The term "immunoglobulin" as used herein refers to any molecule comprising an immunoglobulin fold or immunoglobulin domain. Immunoglobulins domains or folds are characteristic features of proteins of the immunoglobulin superfamily of proteins that include molecules such as antibodies, T-cell receptors and MHC molecules. There are two main types of immunoglobulin domain, C domains with a three-strand and a four strand sheet, and V domains with an extra strand in each sheet.

The term "the variable region of an immunoglobulin" or "the variable domain of an immunoglobulin" refers to the most amino-terminal domain of an immunoglobulin, which is formed by recombination of V, D and J gene segments during lymphocyte development. The variable region comprises three regions of particular sequence variability which are termed hypervariable regions and are denoted HV1, HV2 and HV3. The rest of the V domains shows less sequence variability and the regions between the hypervariable regions, which are relatively invariant, are termed framework regions (FR1, FR2, FR3 and FR4). The hypervariable regions are formed by loops in the structure of the V domain, which together form the antigen binding site of the immunoglobulin. As the three hypervariable loops constitute the binding site for antigen and determine specificity by forming a surface complementary to the antigen, they are more commonly termed the complementary determining regions, or CDRs, and are denoted CDR1, CDR2 and CDR3. In the case of the naturally occurring heavy chain antibodies (HCAbs) observed in *Camelidae⁸,* the antigen binding site is only formed from the complementary determining region of the heavy chain, whereas in conventional antibodies the antigen binding site is formed from the complementary determining region of both light and heavy chain.

As used herein, the variable region comprises preferably amino acid residues 1 to 117 as encoded by the nucleic acid sequence of SEQ ID NO: 1.

However, the term "variable region" also refers to mutants and fragments thereof containing one or more mutations that preferably retain the binding specificity of the unmutated variable region which may be a naturally occurring region or stretch of amino acids. The term "mutation" as used throughout the specification of this invention refers to deletion, addition or substitution of an amino acid residue. "One or more" means 1, 2, 3, 4, 5, 6, 7, 8, 9 or 10 but also up to 15 or even 20 amino acid residues. The mutation may reside in N- or C-terminal position or within the variable region. Preferably, the variable region comprises three intact CDRs. However, it is conceivable that in particular cases sufficient antigen binding is still possible with only two or even one CDR. In such cases it is preferred that one of the remaining CDRs is CDR3. A central aspect of the present invention is the provision of fusion molecules capable of recognizing an antigen. In order to improve antigen recognition, it may be desirable to substitute one or more amino acid residues. Preferably, this substitution will be performed in CDR1, CDR2 and/or CDR3. A random approach or a selective approach may be chosen to generate these substitution mutants. Sometimes it may be necessary to exchange further amino acid residues outside of the CDRs in order to generate antibodies with sufficient stability or sufficient affinity to a given antigen. In contrast to VH domains of conventional antibodies, the VHH domains of camel heavychain antibodies are expressed efficiently as soluble non-aggregating recombinant proteins due to their unique hydrophilic substitutions in framework 2: V37F/Y , G44E, L45R. This shows the importance of the amino acid residues outside the CDRs for the stability and solubility of the fragments. A directed mutagenesis of single ore more amino acid residues could lead to an improvement of the stability or solubility. Amino acid substitutions outside the CDRs can also be introduced to humanize camelid VHHs (this is summarized in a review from Muyldermans S., 2001, Reviews in Molecular Biology, 74:277 - 302). As used herein, fragments comprising the variable domain or region are also sometimes described as "antigen recognition fragments".

The term "selecting ... a particular nucleic acid molecule encoding the variable region of a specific immunoglobulin" refers to a selection step which may optionally be performed as part of the described method and in order to identify nucleic acid molecules with particular strong affinity to a given antigen. To this end, the various variable domains encoded by the first nucleic acid molecule are expressed. Following expression, the affinity of the variable domains to a specific antigen is tested. Testing may be done by any suitable technique known to the skilled person including ELISA, RIA, EIA, FACS, MS-MALDI etc. In one example, the variable region is expressed on the surface of a phage or virus. Contacting the phage or virus with an antigen of interest will allow to isolate nucleic acid molecules encoding variable regions with sufficiently high affinity to the antigen. Such systems may be modified by the skilled person in order to identify only variable regions with particularly high affinity to the antigen. This modification may involve, for example, a modification of the buffer conditions or the addition of competitors.

The term "fusing the coding region ... in frame" refers to the generation of fusion proteins. Fusion proteins are chimeric proteins consisting of sequences derived from at least two different molecules. According to the teaching of the present invention, the coding region encoding the variable region of an immunoglobulin is fused in frame to the coding region encoding one or more detectable (poly)peptides, such as fluorescent (poly)peptides. Fusion may be performed by any technique known to the skilled person, as long as it results in the in frame fusion of said coding regions. Conventionally, generation of a fusion protein from two separate proteins or domains is based on the "two-sided splicing by overlap extension" described in Horton R., et al., 1989, Gene 77:61-68. The fragments coding for the single domains or proteins are generated in two separate primary PCR reactions. The inner primers for the primary PCR reactions contain a significant, approximately 20 bp, complementary region that allows the fusion of the two domain fragments in the second PCR. Alternatively, the coding regions may be fused by making use of restriction sites which may either be naturally occurring or be introduced by recombinant DNA technology.

The term "the coding region of the first nucleic acid molecule is located 5' of the coding region of the second nucleic acid molecule" refers to the arrangement of corresponding coding regions on DNA or mRNA level and indicates that the residues encoded by the first nucleic acid sequence are the N-terminal residues of the fusion protein, whereas the residues encoded by the second nucleic acid sequence are the C-terminal residues of the fusion protein.

The term "expressing the fused nucleic acid molecule encoding the fusion protein in a cell or cell free extract" relates to the transcription and translation of the fusion protein using appropriate expression control elements that function in the chosen cell or cell free extract. In this manner, the properties of individual fusion proteins may be tested in cellular expression systems or in cell free extracts. To this end, the nucleic acid molecule encoding the fusion protein may be cloned into a suitable expression vector, the composition of which, generally, depends on the expression system. The expression system may be prokaryotic or eukaryotic. A typical mammalian expression vector contains the promoter element, which mediates the initiation of transcription of mRNA, the protein coding sequence, and signals required for the termination of transcription and polyadenylation of the transcript. Additional elements might include enhancers, Kozak sequences and intervening sequences flanked by donor and acceptor sites for RNA splicing. Highly efficient transcription can be achieved with the early and late promoters from SV40, the long terminal repeats (LTRs) from retroviruses, e.g., RSV, HTLVI, HIVI, and the early promoter of the cytomegalovirus (CMV). However, cellular elements can also be used (e.g., the human actin promoter). Suitable expression vectors for use in practicing the present invention include, for example, vectors such as pSVL and pMSG (Pharmacia, Uppsala, Sweden), pRSVcat (ATCC 37152), pSV2dhfr (ATCC 37146) and pBC12Ml (ATCC 67109). Mammalian host cells that could be used include, human Hela, 293, H9 and Jurkat cells, mouse NIH3T3 and C127 cells, Cos 1, Cos 7 and CV1, quail QC1-3 cells, mouse L cells and Chinese hamster ovary (CHO) cells. Alternatively, the recombinant (poly)peptide can be expressed in stable cell lines that contain the gene construct integrated into a chromosome. The co-transfection with a selectable marker such as dhfr, gpt, neomycin, hygromycin allows the identification and isolation of the transfected cells. The transfected nucleic acid can also be amplified to express large amounts of the encoded (poly)peptide. The DHFR (dihydrofolate reductase) marker is useful to develop cell lines that carry several hundred or even several thousand copies of the gene of interest. Another useful selection marker is the enzyme glutamine synthase (GS) (Murphy et al.1991, Biochem J. 227:277-279; Bebbington et al. 1992, Bio/Technology 10:169-175). Using these markers, the mammalian cells are grown in selective medium and the cells with the highest resistance are selected. The expression vectors will preferably include at least one selectable marker. Such markers include dihydrofolate reductase, G418 or neomycin resistance for eukaryotic cell culture and tetracycline, kanamycin or ampicillin resistance genes for culturing in *E. coli* and other bacteria. Representative examples of appropriate hosts include, but are not limited to, bacterial cells, such as *E. coli,* Streptomyces and *Salmonella typhimurium* cells; fungal cells, such as yeast cells; insect cells such as Drosophila S2 and Spodoptera Sf9 cells; animal cells such as CHO, COS, 293 and Bowes melanoma cells; and plant cells. Appropriate culture mediums and conditions for the above-described host cells are known in the art.

The recombinantly expressed polypeptide may contain additional amino acid residues in order to increase the stability or to modify the targeting of the protein. For instance, a region of additional amino acids, particularly charged amino acids, may be added to the N-terminus of the polypeptide to improve stability and persistence in the host cell, during purification, or during subsequent handling and storage. Also, peptide moieties may be added to the polypeptide to facilitate purification. Such regions may be removed prior to final preparation of the polypeptide. The addition of peptide moieties to polypeptides to engender secretion or excretion, to improve stability and to facilitate purification, among others, are familiar and routine techniques in the art. A preferred fusion protein comprises a heterologous region from immunoglobulin that is useful to stabilize and purify proteins. For example, EP-A- 0 464 533 discloses fusion proteins comprising various portions of constant region of immunoglobulin molecules together with another human protein or part thereof. In many cases, the Fc part in a fusion protein is thoroughly advantageous for use in therapy and diagnosis and thus results, for example, in improved pharmacokinetic properties (EP-A 0 232 262). It is to be understood, however, that the F_{c} portion is advantageously no part of the fusion protein obtainable in accordance with the invention. On the other hand, for some uses it would be desirable to be able to delete the Fc part after the fusion protein has been expressed, detected and purified in the advantageous manner described. This is the case when the Fc portion proves to be a hindrance for example for the activity of the fusion protein. In drug discovery, for example, human proteins, such as hIL-5, have been fused with Fc portions for the purpose of high-throughput screening assays to identify antagonists of hIL-5. See, D. Bennett et al., J. Molecular Recognition 8:52-58 (1995) and K. Johanson et al., J. Biol. Chem. 270:9459-9471 (1995). The fusion protein of the present invention can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography and/or hydroxylapatite chromatography. Most preferably, fast protein liquid chromatography (FPLC) is employed for purification, however, high performance liquid chromatography ("HPLC") may also be used.

In accordance with the present invention it is preferred that the fusion protein is expressed from a viral expression system or an expression system involving a phage. A number of such expression systems have been described in the art (see e.g. Hoogenboom H.R. et al., 1998, Antibody phage display and its applications, Immunotechnology 4:1-20; Pluckthun A, 1994, Escherichia coli producing recombinant antibodies, Bioprocess Technol. 19:233-252, Verma R. et al, 1998, Antibody engineering: comparision of bacterial, yeast, insect and mammalian expression systems, J. immunol. Methods 216:165-181. Particularly preferred are expression systems which involve surface exposure of the amino acid residues forming the variable region, since this allows to perform selection based on the interaction of said variable region with an antigen of interest.

The terms "fluorescent" or "chromophoric" or "phosphorescent peptide or protein" refer to (poly)peptides with fluorescent or chromophoric or phosphorescent properties. A variety of fluorescent proteins and chromoproteins can be used as second (poly)peptide sequence. One group of such fluorescent proteins includes Green Fluorescent Protein isolated from *Aequorea victoria* (GFP), as well as a number of GFP variants, such as cyan fluorescent protein, blue fluorescent protein, yellow fluorescent protein, etc. (Zimmer, 2002, Chem. Rev. 102: 759-781 ; Zhang et al., 2002, Nature Reviews 3: 906-918). Typically, these variants share about 80%, or greater sequence identity with SEQ ID 6 or 7, respectively. A number of color shift mutants of GFP have been developed and may be used to generate the fluorescent antigen recognition fragments of the invention. These color-shift GFP mutants have emission colors blue to yellow-green, increased brightness, and photostability (Tsien, 1998, Annual Review of Biochemistry 67: 509-544). One such GFP mutant, termed the Enhanced Yellow Fluorescent Protein, displays an emission maximum at 529 nm. Additional GPF-based variants having modified excitation and emission spectra (Tsien et al., U.S. Patent Appn. 200201231 13A1), enhanced fluorescence intensity and thermal tolerance (Thastrup et al., U.S. Patent Appn. 20020107362A1; Bjorn et al., U.S. Patent Appn. 20020177189A1), and chromophore formation under reduced oxygen levels (Fisher, U.S. Patent No. 6,414,119) have also been described. Most recently, GFPs from the anthozoans *Renilla reniformis* and *Renilla kollikeri* were described (Ward et al., U.S. Patent Appn. 20030013849).

Another group of such fluorescent proteins includes the fluorescent proteins isolated from anthozoans, including without limitation the red fluorescent protein isolated from *Discosoma* species of coral , DsRed (Matz et al., 1999, Nat. Biotechnol. 17:969-973), e.g., SEQ ID NO: 8 or 9, respectively (see, e.g., accession number AF168419 version AF16849.2). DsRed and the other anthozoan fluorescent proteins share only about 26-30% amino acid sequence identity to the wild-type GFP from *Aequorea victoria,* yet all the crucial motifs are conserved, indicating the formation of the 11 -stranded beta-barrel structure characteristic of GFP. The crystal structure of DsRed has also been solved, and shows conservation of the 11-stranded beta-barrel structure of GFP MMDB ld: 5742.

A number of mutants of the longer wavelength red fluorescent protein DsRed have also been described, and similarly, may be employed in the generation of the fluorescent antigen recognition fragments of the invention. For example, recently described DsRed mutants with emission spectra shifted further to the red may be employed in the practice of the invention (Wiehler et al., 2001, FEBS Letters 487: 384-389; Terskikh et al., 2000, Science 290: 1585-1588; Baird et al., 2000, Proc. Natl. Acad. Sci. USA 97: 11984-11989).

An increasingly large number of other fluorescent proteins from a number of ocean life forms have recently been described, and the Protein Data Bank currently lists a number of GFP and GFP mutant crystal structures, as well as the crystal structures of various GFP analogs. Related fluorescent proteins with structures inferred to be similar to GFP from corals, sea pens, sea squirts, and sea anemones have been described, and may be used in the generation of the fluorescent antigen recognition fragments of the invention (for reviews, see Zimmer, 2002, Chem. Rev. 102: 759-781 ; Zhang et al., 2002, Nature Reviews 3: 906-918).

Fluorescent proteins from *Anemonia majano*, *Zoanthus sp.*, *Discosoma striata*, *Discosoma sp.* and *Clavularia sp.* have also been reported (Matz et al., supra). A fluorescent protein cloned from the stony coral species, *Trachyphyllia geoffroyi,* has been reported to emit green, yellow, and red light, and to convert from green light to red light emission upon exposure to UV light (Ando et al., 2002, Proc. Natl. Acad. Sci. USA 99: 12651 -12656). Recently described fluorescent proteins from sea anemones include green and orange fluorescent proteins cloned from *Anemonia sulcata* (Wiedenmann et al., 2000, Proc. Natl. Acad. Sci. USA 97: 14091-14096), a naturally enhanced green fluorescent protein cloned from the tentacles of *Heteractis magnifica* (Hongbin et al., 2003, Biochem. Biophys. Res. Commun. 301: 879-885), and a generally non fluorescent purple chromoprotein displaying weak red fluorescence cloned from *Anemonia sulcata*, and a mutant thereof displaying far-red shift emission spectra (595nm) (Lukyanov et al., 2000, J. Biol. Chem. 275: 25879-25882).

Additionally, another class of GFP-related proteins having chromophoric and fluorescent properties has been described. One such group of coral-derived proteins, the pocilloporins, exhibit a broad range of spectral and fluorescent characteristics (Dove and Hoegh-Guldberg, 1999, PCT application WO 00146233; Dove et al., 2001, Coral Reefs 19: 197-204). Recently, the purification and crystallization of the pocilloporin Rtms5 from the reef-building coral *Montipora efflorescens* has been described (Beddoe et al., 2003, Acta Cryst. D59: 597-599). Rtms5 is deep blue in colour, yet is weakly fluorescent. However, it has been reported that Rtms5, as well as other chromoproteins with sequence homology to Rtms5, can be interconverted to a far-red fluorescent protein via single amino acid substitutions (Beddoe et al., 2003, supra; Bulina et al., 2002, BMC Biochem. 3: 7; Lukyanov et al., 2000, supra).

Various other coral-derived chromoproteins closely related to the pocilloporins are also known (see, for example, Lukyanov et al. 2000, J. Biol. Chem. 275: 25879- 82; Gurskaya et al., 2001, FEBS Letters 507: 16-20). Any of the fluorescent or chromophoric proteins or fluorescent or chromophoric fragments thereof may be used in accordance with the teaching of the present invention. Further examples of fluorescent proteins are GFP form Renilla reniformis, mKO from Fungia concinna, Azami Green from Galaxeidae or cOFP from Cerianthus. Fragments of the fluorescent or chromophoric protein are preferably functional fragments.

Fluorescent, chromophoric or phosphorescent protein belong to the group referred to as "detectable proteins" and "detectable (poly)peptides" and "detectable marker (poly)peptides". In general, these terms refer to (poly)peptides which, upon excitation, result in the emission of detectable energy. Examples of detectable proteins are fluorescent proteins, chromophoric proteins or "phosphorescent", "fluorescent" and "phosphorescent" are used exchangeably. This means e.g. when an embodiment refers to "fluorescent", this embodiment comprises "phosphorescent ...", "fluorescent ...." and "phosphorescent ... ".

The term "the coding region of the first nucleic acid molecule is located 5' of the coding region of the second nucleic acid molecule" refers to the arrangement of corresponding coding regions on DNA or mRNA level and indicates that the residues encoded by the first nucleic acid sequence are the N-terminal residues of the fusion protein, whereas the residues encoded by the second nucleic acid sequence are the C-terminal residues of the fusion protein.

The term "functional fragment" or "functional homologue", as used throughout the specification, relates to fragments of the fusion proteins of the present invention, wherein these fragments retain the full or at least a partial antigen binding activity and/or retain a detectable, e.g. fluorescent or chromophoric, activity of the fusion protein of the present invention. The term "at least a partial antigen binding activity" means an activity which is reduced to no more than preferably 10%, more preferably 1% in comparison to full length fusion protein. A convenient measure for determining the antigen binding activity is the Kd for the pair of antigen/antibody.

The term "linker of at least one amino acid residue" refers to amino acid residues which may be arranged between the residues encoded by the first and second nucleotide sequence. Such a linker may in some cases be useful, for example, to improve separate folding of the individual domains or to modulate the stability of the fusion protein. Moreover, such linker residues may contain signals for transport, protease recognition sequences or signals for secondary modification. The amino acid residues forming the linker may be structured or unstructured. Preferably, the linker may be as short as 1 amino acid residue or up to 2, 3, 4, 5, 10, 20 or 50 residues. In particular cases, the linker may even involve up to 100 or 150 residues.

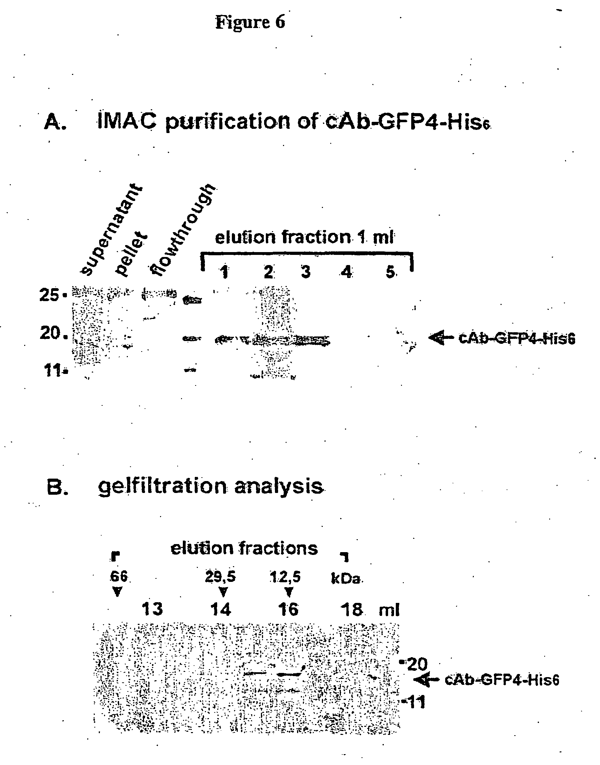

Described is the concept of detecting intracellular antigens by using detectable proteinaceous compounds which are capable of specifically binding to antigens. To this end, heavy chain antibodies (HCAbs) devoid of light chains of the *Camelidae⁸,* have been used (see Fig. 6). HCAbs recognize and bind their antigen via hypervariable regions (CDRs, complementarity determining regions) located in the heavy-chain variable domain (referred to as VHH), which present the smallest intact antigen-binding fragment (-15 kDa)⁹⁻¹¹. In comparison to other small antibody fragments derived from other mammals like Fab, Fv or scFv, the VHHs have a number of advantages. First, only one domain has to be cloned or synthesized and expressed to generate an *in vivo* matured antigen-binding fragment. Second, specific VHHs can be easily selected by cloning the VHH in phage display vectors followed by several rounds of panning for antigen binders. Third, VHHs are highly soluble and stable and can be efficiently expressed in heterologous systems⁹. The affinities found for VHHs so far were in the nanomolar range and comparable with those of scFv^{†2-†4}. For detection, the epitope binding part (VHH) is fused to a fluorescent polypeptide.

The small size of the antigen binding domain allows detection of otherwise inaccessible antigens in a cell. Furthermore, it allows higher resolution of complex biological structures. By the direct visualization of the antigen on a cell surface using the chromobody, a stoichiometric detection for linear quantification can take place. This is the first method to detect antigens in living cells. The method includes any antigenic structure like e.g. proteins or nucleic acids but also posttranslational modifications and non proteinaceous modifications such as e.g. lipids, sugars and nucleic acids. This method provides information about concentration and localization of intracellular antigens and can therefore be applied in basic research (detection, quantification and inactivation/masking of cellular antigens), diagnosis, target validation (selective inhibition of cellular components and/or masking of specific binding sites), therapy of mammals, in particular human mammals or in assay development for high throughput drug screening. The readout of the method described herein, e.g. different energy levels, enable for the applications described above.

The diagnostic application of the method as described herein is based on the comparison of the detected presence, amount or subcellular location of an antigen of interest in a cell of an individual suffering from a pathological condition to those in a cell of a healthy individual. For example, differences in the amount, presence or subcellular location of a protein having the or corresponding to the antigen of interest, may correlate with disease progress over time. Similarly, the efficacy of a medicament may be examined by following changes in the amount, presence or location of the antigenic structure correlated with a pathological status.

For research purposes potential alterations of the outcome can be detected upon treatment of cells with certain agents. The same principle underlies the application of the present invention for target validation or drug screening.

In general, the total fluorescence per cell depends on the expression level of a detectable fusion protein of the invention rather than on the concentration of the antigen. In the absence of the antigen, the fusion protein/chromobodies will be distributed all over the cell. In the presence of the antigen, the chromobody distribution will shift towards the respective subcellular compartment of the antigen, e.g. the cytoskeleton, nuclear lamina, replication sites or chromatin. Such a shift is not only indicative of the subcellular location of the antigenic structure, but also of its presence at all. Depending on the intensity of the signal, the amount of the antigenic structure within the cell may be determined. The method of image analysis depends on the antigen and can be an analysis of mean intensity variation (standard deviation of the fluorescence intensity) or any other method identifying the structure or compartment of interest by threshold based selection or by specific fluorescent markers and then determining the ratio between this structure/compartment versus the rest of the cell.

Applying the teaching of the present invention, in one example a 15 kDa epitope binding antibody fragment raised in *Lama pacos* against the green fluorescent protein (GFP) is disclosed. As shown herein, the epitope binding part (VHH) can be fused e.g. to the monomeric version of the red fluorescent protein (mRFP1). This anti-GFP "chromobody" can be stably expressed in mammalian cells where it recognizes its epitope in different cellular compartments and structures. With time lapse microscopy we demonstrate that the chromobody efficiently traces dynamic changes of target proteins throughout the cell cycle in living cells. As used herein, the term "chromobody" is understood as an antigen recognizing domain fused to a fluorophoric or chromogenic protein.

The results disclosed herein are surprising, in particular since a rather similar approach involving fusion proteins based on the same functional domains (variable domain and GFP) failed to provide any useful tools (see WO 03/091415A2 and Zeytun et al, Nature Biotechnology 2003, Vol. 21, 12: 1473-1479) and had to be retracted by the authors (Zeytun et al, Nature Biotechnology 2004, Vol. 22, 5: 601). In this work, Zeytun et al. described the insertion of diverse antibody binding loops into the four exposed loops at one end of the green fluorescent protein (GFP). In more detail, the antibody binding loop derived from the complementarity region 3 of the heavy chain was inserted in one of the four loops of GFP, which connect the beta strands of the ß-sheet structure. The resulting constructs were called fluorobodies. Specific fluorobodies recognizing a number of antigens were selected by phage display. The antigen recognition competence of the different fluorobodies was tested by various methods including band shifting experiments and immunofluorescence. This approach would have been an interesting combination of an antigen recognizing domain and the fluorescent properties of the green fluorescent properties. Interestingly, however, the authors themselves retracted their manuscript because the results initially presented were not reproducible. In the retraction (Zeytun et al., 2004, Nature Biotechnology Vol.22, 5:601), the authors stated that sequencing of the nucleic acids coding for the described constructs revealed so called out "*off frame* mutants". In other words, none of the fluorobodies initially described by Zeytun et al have the assumed coding nucleic acid or amino acid sequence. Further, Zeytun et al, declared that the observed biological phenomenon cannot be explained. In this case, the combination of the antigen recognizing domain and a fluorescent protein failed to function.

Herein a novel technique is disclosed allowing to target and trace antigens in living cells. The teaching of the present invention is illustrated by the examples, involving chromobodies generated by raising single domain antibodies against GFP, drosophila lamin Dm0 and cytokeratin-8 in *Lama pacos* respective *Camelus dromedarius* and fusing the VHH epitope binding part to mRFP1. As an example it is clearly shown that the anti-GFP chromobody can be stably expressed in cells as an active monomer and has access to all tested subcellular compartments and structures. Aggregates, as described for a large number of intracellular expressed intrabodies like scFvs²⁶, were not detected. This intracellular functionality of the llama derived VHH domain suggests that its intrinsic stability (ΔG= 30 kJ/mol)²⁷ even in the absence of the conserved C22-C92 disulfide bond is sufficiently high to fold properly. The absence of an extra pair of cysteines in the CDRs in the epitope binding domain of llama derived HCAbs could then be a natural advantage over VHH domains from dromedaries, where a frequently occurring interloop disulfide bond is essential for antigen recognition^{9, 28}. Another example describes a GFP chromobody, wherein one of the conserved cysteines in the VHH region has been mutated to serine. The Example clearly shows not only a higher stability of the resulting chromobody, but surprisingly also a better accessibility of the molecule within the cell.

Furthermore the feasibility of this technique is demonstrated with chromobodies raised against two endogenous protein: cytokeratin-8 and lamin. This novel approach employing chromobodies dramatically expands the possibilities of, for example, live cell microscopy since virtually any potentially antigenic component or structure can be targeted and traced within living cells. The fact that specific chromobodies can be selected out of recombinant libraries and their affinity further improved by mutagenesis cycles will in future reduce, possibly eliminate, the need for animal immunisation²⁹. Nevertheless, VHHs retrieved from 'immune libraries' benefit from the inherent antigen-specificity and affinity maturation occurring during immunisation. The fusion proteins/chromobodies generated in accordance with the present invention can be used, for example, for conventional antibody applications as the fluorescent part, the red fluorescent protein (mRFP1) in this study, can easily be replaced by any other fluorescent protein, chromogenic enzyme or can e.g. be coupled to fluorescent dyes and quantum dots. Examples of such chromogenic enzymes are horse radish peroxidase (HRP), alkaline phosphatase or β-galactosidase.

The fusion protein or the first polypeptide sequence comprising the variable domain of Camelidae antibodies as described in the present invention can also be coupled to non-proteinacious fluorochromes. Fluorochromes are available as N-Hydroxysuccinimidylester (NHS-ester) and can be coupled to proteins, e.g. the variable domains (VHHs) of the present invention, via primary amino groups of said protein. Commonly available fluorochromes show extinction maxima in different wavelength regions. Examples are Cy-dyes (e.g. Cy3, Cy5), Dy549, Oyster556, Alexa-dyes or Tamra. Successful coupling and application of the fusion protein of the invention is illustrated in the Examples.

As any other tool, also chromobodies will need careful controls. Like the fusion of proteins to GFP also the binding of chromobodies to their targets may affect their activity and regulation especially by preventing interaction with other cellular components. However, the ease of selecting and developing specific antibody fragments will regularly yield a set of binders to different domains of the target proteins. Thus binders can be selected that may be better suited for either tracing or for functional interference depending on their binding site.

In summary, it has been demonstrated that the fusion protein applied in the method described and in particular the chromobody presented here is suitable for the targeting and tracing of antigens in all tested subcellular compartments and structures. Even antigens from central parts of the replication machinery as well as antigens deeply embedded in the chromatin could be traced throughout S phase and mitosis demonstrating the suitability of chromobodies for live cell studies.

In a preferred embodiment of the described method, step (b) comprises (a) exposing the cell with light corresponding to the excitation wavelength of the fusion protein; (b) detecting energy emitted from the cell and/or detecting the subcellular distribution of the emitted energy, (c) comparing the energy detected in step (d) to: (i) the energy detected in a reference cell which contains a reference amount of said antigenic structure of interest; or expresses a reference fusion protein for which no binding partner is expressed in the cell; or (ii) a data control and (e) concluding from a different energy on the health state of an individual or assessing the presence of the antigenic structure of interest; wherein a higher energy detected in step (c) compared to that of step (d) is indicative of the presence of said antigen; and/or concluding form the amount and/or subcellular localization/distribution of the emitted energy to the amount or subcellular localization/distribution of the antigenic structure of interest.

The present specification describes a method wherein the fusion protein comprising a first (poly)peptide sequence comprising the variable region of a heavy chain antibody of *Camelidae* and a second (poly)peptide sequence derivable from a fluorescent or chromophoric protein, wherein said (a) first (poly)peptide sequence is composed of framework 1, CDR1, framework 2, CDR2, framework 3 and CDR3, encoded by the nucleic acid sequence of SEQ ID NO: 2 or encoded by a nucleic acid sequence with at least 70% sequence identity or a fragment thereof; and (b) second (poly)peptide sequence is (i) the green fluorescent protein derivable from *Aequorea victoria* encoded by the nucleic acid sequence of SEQ ID NO: 7, or a fluorescent mutant or fragment thereof; (ii) the red fluorescent protein derivable from *Discosoma* (DsRed) encoded by the nucleic acid sequence of SEQ ID NO: 9, or a fluorescent mutant or fragment thereof; or (iii) a functional homologue of (i) or (ii) with at least 80% sequence identity, more preferably at least 85%, even more preferably at least 90%, even more preferably at least 95% and most preferably with at least 98% sequence identity, wherein said first (poly)peptide sequence is located N-terminally of said second (poly)peptide sequence, said sequences being optionally separated by a linker of at least one amino acid residue. The term "fragment" refers to functional fragments capable of specifically binding to the antigen.

In a preferred embodiment said variable region comprises framework 1, CDR1, framework 2, CDR2, framework 3 and CDR3, and is encoded by the nucleic acid sequence of SEQ ID NO: 2 or is encoded by a nucleic acid sequence with at least 70%, more preferably 80%, more preferably at least 85%, even more preferably at least 90%, even more preferably at least 95% and most preferably at least 98% sequence identity or a fragment thereof. Preferably said fragment is a functional fragment, i.e. a fragment capable of specific antigen binding. "Specific binding" of may be described, for example, in terms of cross-reactivity. Preferably, specific binders are proteins (fusion proteins or fragments thereof) having a dissociation constant or K_{D} of less than 10⁻⁹M. In a preferred embodiment of the present invention, the sequence of (a) CDR1 consists of the residues shown in SEQ ID NO: 3; (b) CDR2 consists of the residues shown in SEQ ID NO: 4; and (c) CDR3 consists of the residues shown in SEQ ID NO: 5.

In a more preferred embodiment of the present invention, said first (poly)peptide sequence of said fusion protein or the (poly)peptide comprising the variable region of a heavy chain antibody of *Camelidae* has the sequence of any one of SEQ ID NOs: 10 or 30 or is encoded by the sequence of any one of SEQ ID NOs: 11 or 31.

In a more preferred embodiment of the present invention, said second (poly)peptide sequence comprises residues 1 to 239 of SEQ ID NO: 6 or 1 to 226 of SEQ ID NO: 8 or a fluorescent mutant or fragment thereof.

In another preferred embodiment of the present invention, said mutant of the red fluorescent protein is mRFP1 as encoded by SEQ ID NO: 17 or any of the fluorescent DsRed mutants encoded by any one of SEQ ID NOs 17 and 24 to 27 or by a fragment thereof. mRFP is the monomeric red fluorescent protein derived from DsRed and is often also termed mRFP1 (Campbell et al., (2002) PNAS, 99:7877-7882, coding sequence: accession number: AF506027.1 (SEQ ID NO: 17)). Several other monomeric derivatives of DsRed have in the meantime been generated, including mRFPmars (coding sequence: accession number: AY679163.1 (SEQ ID NO: 24)), published by Fischer et al., (2004) FEBS Letters, 577: 227 - 232, mCherry (coding sequence: accession number: AY678264 (SEQ ID NO: 25)), published by Shaner,N.C. et al., (2004) Nat. Biotech., 22:1567 - 1572), mRaspberry (coding sequence: accession number: AY86536 (SEQ ID NO: 26)), published by Wang, L. et al., (2004) PNAS, 22:1567 - 1572, mPlum (coding sequence: accession number: AY86537.1 (SEQ ID NO: 27)), published by Wang, L. et al., (2004) PNAS, 22:1567 - 1572. Any of these fluorescent mutants derived from DsRed may be used in accordance with the teaching of the present invention. In particular, the fusion proteins generated in accordance with the teaching of the present invention may comprise a fluorescent second (poly)peptide sequence which comprises any of the aforementioned DsRed mutants or fluorescent mutants or fragments thereof.

In another preferred embodiment of the present invention, the fusion protein of the present invention further comprises a targeting sequence selected from the group consisting of nuclear localization signal (NLS), endoplasmic reticulum import sequence, mitochondrial import sequence. An example of an NLS is the peptide sequence PKKKRKV (nuclear-localization signal (NLS) of the SV40 large T-antigen, D. Kalderon et al., 1984, Cell 39:499) which is capable of directing heterologous proteins into the nucleus. An example of the "Endoplasmatic Reticulum Import Sequence" is the peptide MMSFVSLLLVGILFWATEAEQLTRCVFQ (ER localisation signal of immunoglobulin light chain, Blobel G. & Dobberstein B., 1975 J.Cell.Biol. 67:835 - 851) which is capable of directing heterologous proteins into the lumen of the ER. An example of a "Mitochondrial Import Sequence" is the peptide MLSLRQSIRFFRPATRTLCSSRYLL (Neupert W. 1997 Annu. Rev. Biochem. 66:863-917) which is capable of directing heterologous proteins into the mitochondrium.

Described is also a fusion protein having the sequence of any one of SEQ ID NOs: 18, 32, 20 or 22 or being encoded by a nucleic acid molecule comprising the sequence of any one of SEQ ID NOs: 19, 33, 21 or 23.

In a preferred embodiment the cell is a cell obtained from an individual.

In another preferred embodiment said antigenic structure of interest is detected within a cell. In a more preferred embodiment said cell is a living cell. In another preferred embodiment, said cell is a cell within a living eukaryotic organism, preferably within a mammal.

In a preferred embodiment, said antigenic structure is selected from protein, protein modification, cofactor, small molecular compound, DNA and RNA.

In a preferred embodiment the (poly)peptide capable of transducing is selected from the group of basic (poly)peptides comprising TAT peptide, poly-arginine and poly-lysine.

In a more preferred embodiment, the protein is a cytoplasmic, nuclear or nucleolar protein.

The term "fragment" refers to deletion mutants of the variable region as defined above that retain the binding specificity (see above). Such deletion mutants may comprise one or more deletions, involving the deletion of e.g. 1, 2, 3, 4, 5, 6, 7, 8, 9 or 10 and up to 15 or even 20 amino acid residues. The deletions may reside in Nor C-terminal position or internally. Preferably, the variable region comprises three intact CDRs. However, it is conceivable that in particular cases sufficient antigen, binding is still possible with only two or even one CDR. In such cases it is preferred that one of the remaining CDRs is CDR3.

Sequence identity may be determined by using the Bestfit® program (Wisconsin Sequence Analysis Package, Version 8 for Unix, Genetics Computer Group, University Research Park, 575 Science Drive, Madison, WI 53711). Bestfit® uses the local homology algorithm of Smith and Waterman to find the best segment of homology between two sequences (Advances in Applied Mathematics 2:482-489 (1981)). When using Bestfit® or any other sequence alignment program to determine whether a particular sequence is, for instance, 95% identical to a reference sequence, the parameters are set, of course, such that the percentage of identity is calculated over the full length of the reference nucleotide sequence and that gaps in homology of up to 5% of the total number of nucleotides in the reference sequence are allowed. The identity between a first sequence and a second sequence, also referred to as a global sequence alignment, is determined using the FASTDB computer program based on the algorithm of Brutlag and colleagues (Comp. App. Biosci. 6:237-245 (1990)). In a sequence alignment the query and subject sequences are both DNA sequences. An RNA sequence can be compared by converting U's to T's. The result of said global sequence alignment is in percent identity. Preferred parameters used in a FASTDB alignment of DNA sequences to calculate percent identity are: Matrix=Unitary, k-tuple=4, Mismatch Penalty=1, Joining Penalty=30, Randomization Group Length=0, Cutoff Score=1, Gap Penalty=5, Gap Size Penalty 0.05, Window Size=500 or the length of the subject nucleotide sequence, whichever is shorter.

The protein fragment comprising the VHH sequence applied in the present invention can be produced with an antibody producing cell, which can be a peripheral blood lymphocyte (PBL). Peripheral blood lymphocytes may be obtained from blood of an immunised camel. The most common technique for separating leucocytes is to mix blood with a compound which aggregates the erythrocytes, thereby increasing their sedimentation rate. The sedimentation of leukocytes is only slightly affected and can be collected from the upper part of the tube when erythrocytes have settled (Thorsby E. & Bratilie A., 1970, Histocompatibility Testing 1970, ed. P.I. Terasaki, p.655 Munksgaard, Copenhagen). A standard procedure is preferred according to a protocol provided by e.g. AXIS-SHIELD PoC AS (Oslo, Norway) using Lymphoprep^{™}.

The PBLs can be challenged with an antigen for which the variable region of the immunoglobulin is specific. Said challenging may be performed in vitro (i.e. e.g. in cell culture) or in vivo (in an animal). Various protocols exist in the art, for challenging PBLs with antigens (see e.g. Harlow, E. and Lane , D. 1988 Antibodies, A Laboratory Manual, Chapter 5, Cold Spring Harbor, New York).

Furthermore, the fusion protein applied in the present invention may be altered the method of the present invention further comprises the additional step of modifying the first nucleic acid molecule encoding the variable region within CDR1, CDR2 and/or CDR3. This can be done with a view to retain the specificity but improve the affinity, for example. Alternatively, the specificity may be changed. The term "modifying" means mutagenizing or changing the nucleotide sequence encoding the variable region. Mutant first sequences may contain one or more deletions, substitutions or additions as outlined above.

CDR1, CDR2 and/or CDR3 can be modified by random mutagenesis of the first nucleic acid molecule. The complexity of phage libraries has often been limited to 10⁸ by the low efficiency of DNA transformation in bacteria. Thus a library comprises only a very small number of all the possible sequences. This is one of the main reasons why ligands selected from random antibody-fragment libraries are often of low affinity. An effective way to overcome this limitation and identify ligands with improved properties is through a process of "epitope maturation". Antigen recognizing CDR sequences selected from the initial library are partially mutagenized to generate a population of variants. Screening this library presenting an antigen allows to identify ligands that are better than the original lead sequence. This may be done e.g. by site-directed mutagenesis exchanging single nucleotides in the CDR regions (reference Clackson T. and Lowman H.B., 2004, Phage Display: A Practical Approach, Oxford University Press Inc., New York). Further techniques are to synthesize the CDR regions in order to exchange single nucleotides leading to single amino acid substitution. Artificial synthesized CDRs would be inserted into the scaffold of the antibody fragment in the particular positions either by ligation using classical restriction or " two-sided splicing by overlap extension" described in Horton R. et al., 1989, Gene 77:61-68. Furthermore, it is preferred to use random mutagenesis. This could be done by olignucleotide directed mutagenesis described by Zoller, M.J. 1992, Curr.Opinion in Biotechnology 3:348-354. A further development using Trinucleotide phosphoramidites in a solid-phase DNA synthesis is described in Virnekas T. et al., 1994, Nucl. Acids Res. 22, 5600-5607.

The first nucleic acid molecule encoding the variable region is selected by contacting the variable region with a binding partner (an antigen). Selection, and in particular detection of the variable domain may be performed on the basis of the fusion protein comprising the fluorescent or chromophoric (poly)peptide. Alternatively, selection of particular variable regions may also be performed in the absence of the fluorescent or chromophoric (poly)peptide. For example, prior to or after fusion, a pool of variable regions may be tested with regard to their specificity or affinity to a particular antigen. Suitable tests include FACS, ELISA, RIA, EIA; MS-MALDI etc.

In many cases the variable region will be expressed in an expression library as described herein below in detail. Methods of screening libraries are well known to those in the art. The libraries are typically screened using an antigen, or molecule of interest, for which it is desirable to select a binding partner. Typically, the antigen is attached to a solid surface or a specific tag, such as biotin. The antigen (or molecule of interest) is incubated with a library of the invention. Those polypeptides or library members that bind to the antigen are then separated from those that do not, using any of a number of different methods. These methods involve washing steps, followed by elution steps. Washing can be done, for example, with PBS, or detergent-containing buffers. Elution can be performed with a number of agents, depending on the type of library. For example, an acid, a base, bacteria, or a protease can be used when the library is a phage display library.

To facilitate the identification and isolation of the antigen-bound fluorescent antigen recognition fragment, the fluorescent antigen recognition fragment can also be engineered as a fusion protein to include selection markers (e.g., epitope tags). Antibodies reactive with the selection tags present in the fusion proteins or moieties that bind to the labels can then be used to isolate the antigen- fluorescent antigen recognition fragment complex via the epitope or label.

Other detection and purification facilitating domains include, e.g., metal chelating peptides such as polyhistidine tracts and histidine-tryptophan modules that allow purification on immobilized metals, protein A domains that allow purification on immobilized immunoglobulin, or the domain utilized in the FLAGS extension/affinity purification system (Immunex Corp, Seattle WA). Any epitope with a corresponding high affinity antibody can be used, e.g., a myc tag (see, e.g., Kieke, 1997, Protein Eng. 10:1303-1310) or an His-tag (Pharmacia). See also Maier, 1998, Anal. Biochem. 259:68-73; Muller, 1998, Anal. Biochem. 259:54-61.

The resulting fusion protein can be selected in an expression library. Expression may be performed as outlined above. The expression system may involve display of the variable domain. The expression library may be, for example, a yeast, bacterial or mammalian expression library.

In addition to phage display libraries, which is described in detail below, analogous epitope display libraries can also be used. For example, the methods of the invention can also use yeast surface displayed libraries (see, e.g., Boder, 1997, Nat. Biotechnol., 15553-15557), which can be constructed using such vectors as the pYDI yeast expression vector. Other potential display systems include mammalian display vectors and *E. coli* libraries.

*In vitro* display library formats known to those of skill in the art can also be used, *e.g.*, ribosome displays libraries and mRNA display libraries. In these *in vitro* selection technologies, proteins are made using cell-free translation and physically linked to their encoding mRNA after *in vitro* translation. In typical methodology for generating these libraries, DNA encoding the sequences to be selected are transcribed *in vitro* and translated in a cell-free system.

In ribosome display libraries (see, e.g., Mattheakis et al. , 1994, Proc. Natl. Acad. Sci USA 91:9022-9026; Hanes & Pluckthrun, 1997, Proc. Natl. Acad. Sci USA, 94:4937-4942) the link between the mRNA encoding the fluorescent antigen recognition fragment of the invention and the ligand is the ribosome itself. The DNA construct is designed so that no stop codon is included in the transcribed mRNA. Thus, the translating ribosome stalls at the end of the mRNA and the encoded protein is not released. The encoded protein can fold into its correct structure while attached to the ribosome. The complex of mRNA, ribosome and protein is then directly used for selection against an immobilized target. The mRNA from bound ribosomal complexes is recovered by dissociation of the complexes with EDTA and amplified by RT-PCR.

Method and libraries based on mRNA display technology, also referred to herein as puromycin display, are described, for example in US Patent Nos. 6,261,804; 6,281,223; 6207446; and 6,214553. In this technology, a DNA linker attached to puromycin is first fused to the 3'end of mRNA. The protein is then translated *in vitro* and the ribosome stalls at the RNA-DNA junction. The puromycin, which mimics aminoacyl tRNA, enters the ribosomal A site and accepts the nascent polypeptide. The translated protein is thus covalently linked to its encoding mRNA. The fused molecules can then be purified and screened for binding activity. The nucleic acid sequences encoding ligands with binding activity can then be obtained, for example, using RT-PCR.

The fluorescent antigen recognition fragments and sequences, *e.g.*, DNA linker for conjugation to puromycin, can be joined by methods well known to those of skill in the art and are described, for example, in US Patent Nos. 6,261,804; 6,281,223; 6207446; and 6,214553.

Other technologies involve the use of viral proteins *(e.g.*, protein A) that covalently attach themselves to the genes that encodes them. Fusion proteins are created that join the fluorescent antigen recognition fragment to the protein A sequence, thereby providing a mechanism to attach the binding ligands to the genes that encode them.

Plasmid display systems rely on the fusion of displayed proteins to DNA binding proteins, such as the lac repressor (see, e.g., Gates et al., 1996, J. Mol. Biol., 255:373-386; 1996, Methods Enzymol. 267:171-191). When the lac operator is present in the plasmid as well, the DNA binding protein binds to it and can be copurified with the plasmid. Libraries can be created linked to the DNA binding protein, and screened upon lysis of the bacteria. The desired plasmid/proteins are rescued by transfection, or amplification.

Selection can also be performed in a phage display library.

Construction of phage display libraries exploits the bacteriophage's ability to display peptides and proteins on their surfaces, *i.e*., on their capsids. Often, filamentous phage such as M13, fd, or f1 are used. Filamentous phage contain single-stranded DNA surrounded by multiple copies of genes encoding major and minor coat proteins, *e.g.*, plll. Coat proteins are displayed on the capsid's outer surface. DNA sequences inserted in-frame with capsid protein genes are co-transcribed to generate fusion proteins or protein fragments displayed on the phage surface. Phage libraries thus can display peptides representative of the diversity of the inserted sequences. Significantly, these peptides can be displayed in "naturally" folded conformations. The fluorescent antigen recognition fragments expressed on phage display libraries can then bind target molecules, i.e., they can specifically interact with binding partner molecules such as antigens, e.g., (Petersen, 1995, Mol. Gen. Genet., 249: 425-31), cell surface receptors (Kay, 1993, Gene 12859-65), and extracellular and intracellular proteins (Gram, 1993, J. Immunol. Methods, 161: 169-76).

The concept of using filamentous phages, such as M13 or fd, for displaying peptides on phage capsid surfaces was first introduced by Smith, 1985, Science 228:1315-1317. Peptides have been displayed on phage surfaces to identify many potential ligands (see, e.g., Cwirla, 1990, Proc. Natl. Acad. Sci. USA, 87:6378-6382). There are numerous systems and methods for generating phage display libraries described in the scientific and patent literature, See, *e.g.*, Sambrook and Russell, Molecule Cloning: A Laboratory Manual, 3rd edition, Cold Spring Harbor Laboratory Press, Chapter 18, 2001; Phage, Display of Peptides and Proteins: A Laboratory Manual, Academic Press, San Diego, 1996; Crameri, 1994, Eur. J. Biochem. 226:53- 58; de Kruif, 1995, Proc. Natl. Acad. Sci. USA, 92:3938-3942; McGregor, 1996, Mol. Biotechnol., 6:155-162; Jacobsson, 1996, Biotechniques, 20: 1070-1076; Jespers, 1996, Gene, 173:179-181; Jacobsson, 1997, Microbiol Res., 152:121-128; Fack, 1997, J. lmmunol. Methods, 206:43-52; Rossenu, 1997, J. Protein Chem., 16:499-503; Katz, 1997, Annu. Rev. Biophys. Biomol. Struct., 26:27-45; Rader, 1997, Curr. Opin. Biotechnol., 8:503-508; Griffiths, 1998, Curr. Opin. Biotechnol., 9:102-108.

Typically, exogenous nucleic acids encoding the protein sequences to be displayed are inserted into a coat protein gene, e.g. gene III or gene VIII of the phage. The resultant fusion proteins are displayed on the surface of the capsid. Protein VIII is present in approximately 2700 copies per phage, compared to 3 to 5 copies for protein III (Jacobsson (1996), supra). Multivalent expression vectors, such as phagemids, can be used for manipulation of the nucleic acid sequences encoding the fluorescent antigen recognition fragment library and production of phage particles in bacteria (see, *e.g.*, Felici, 1991, J. Mol. Biol., 222:301-310).

Phagemid vectors are often employed for constructing the phage library. These vectors include the origin of DNA replication from the genome of a single-stranded filamentous bacteriophage, e.g., M13 or f1 and require the supply of the other phage proteins to create a phage. This is usually supplied by a helper phage which is less efficient at being packaged into phage particles. A phagemid can be used in the same way as an orthodox plasmid vector, but can also be used to produce filamentous bacteriophage particle that contain single-stranded copies of cloned segments of DNA.

The displayed protein or protein fragment does not need to be a fusion protein between the (poly)peptide comprising the variable region and the protein of the display system. For example, the (poly)peptide comprising the variable region (i.e. the fluorescent antigen recognition fragment) may attach to a coat protein by virtue of a non-covalent interaction, *e.g.*, a coiled coil binding interaction, such as jun/fos binding, or a covalent interaction mediated by cysteines (see, e.g., Crameri et al., 1994, Eur. J. Biochem., 22653-58) with or without additional non-covalent interactions. Morphosys have described a display system in which one cysteine is put at the C terminus of the scFv or Fab, and another is put at the N terminus of g3p. The two assemble in the periplasm and display occurs without a fusion gene or protein.

The coat protein does not need to be endogenous. For example, DNA binding proteins can be incorporated into the phage/phagemid genome (see, *e.g.*, McGregor & Robins, 2001, Anal. Biochem., 294:108-117,). When the sequence recognized by such proteins is also present in the genome, the DNA binding protein becomes incorporated into the phage/phagemid. This can serve as a display vector protein. In some cases it has been shown that incorporation of DNA binding proteins into the phage coat can occur independently of the presence of the recognized DNA signal.

Other phage can also be used. For example, T7 vectors, T4 vector, T2 vectors, or lambda vectors can be employed in which the displayed product on the mature phage particle is released by cell lysis.

Another methodology is selectively infective phage (SIP) technology, which provides for the *in vivo* selection of interacting protein-ligand pairs. A "selectively infective phage" consists of two independent components. For example, a recombinant filamentous phage particle is made non-infective by replacing its N terminal domains of gene 3 protein (g3p) with a protein of interest, *e.g.*, an antigen. The nucleic acid encoding the antigen can be inserted such that it will be expressed. The second component is an "adapter" molecule in which the fluorescent antigen recognition fragment is linked to those N-terminal domains of g3p that are missing from the phage particle. Infectivity is restored when the displayed protein (e.g., a fluorescent antigen recognition fragment) binds to the antigen. This interaction attaches the missing N-terminal domains of g3p to the phage display particle. Phage propagation becomes strictly dependent on the protein-ligand interaction. See, e.g., Spada, 1997, J. Biol. Chem. 378:445-456; Pedrazzi, 1997, FEBS Lett. 41 5:289-293; Hennecke, 1998, Protein Eng. 11:405-410.

The present invention also relates to a fusion protein comprising a first (poly)peptide sequence comprising the variable region of a heavy chain antibody of *Camelidae* (including any camel or dromedary) and a second (poly)peptide sequence, which is a detectable protein, preferably derivable from a detectable, e.g. fluorescent or chromophoric or phosphorescent protein, wherein said (a) first (poly)peptide sequence is encoded by the nucleic acid sequence of SEQ ID NO: 1 or 31 or encoded by a nucleic acid sequence with at least 95% sequence identity to SED ID: 11 and (b) second (poly)peptide sequence, if derived from a fluorescent or chromophoric protein, may be (i) the green fluorescent protein derivable from *Aequorea victoria* encoded by the nucleic acid sequence of SEQ ID NO: 7, or a fluorescent mutant or fragment thereof; (ii) the red fluorescent protein derivable from *Discosoma* (DsRed) encoded by the nucleic acid sequence of SEQ ID NO: 9, or a fluorescent mutant or fragment thereof; or (iii) a functional homologue of (i) or (ii) with at least 80% sequence identity, more preferably at least 85%, even more preferably at least 90%, even more preferably at least 95% and most preferably with at least 98% sequence identity; wherein said first (poly)peptide sequence is located N-terminally of said second (poly)peptide sequence, said sequences being optionally separated by a linker of at least one amino acid residue. The term "fragment" refers to functional fragments capable of specifically binding to the antigen.

Fusion proteins are chimeric proteins consisting of sequences derived from at least, two different molecules. According to the teaching of the present invention, the coding region encoding the variable region of an immunoglobulin is fused in frame to the coding region encoding one or more detectable (poly)peptides, such as fluorescent (poly)peptides. Fusion may be performed by any technique known to the skilled person, as long as it results in the in frame fusion of said coding regions. Conventionally, generation of a fusion protein from two separate proteins or domains is based on the "two-sided splicing by overlap extension" described in Horton R., et al., 1989, Gene 77:61-68. The fragments coding for the single domains or proteins are generated in two separate primary PCR reactions. The inner primers for the primary PCR reactions contain a significant, approximately 20 bp, complementary region that allows the fusion of the two domain fragments in the second PCR. Alternatively, the coding regions may be fused by making use of restriction sites which may either be naturally occurring or be introduced by recombinant DNA technology.

In a preferred embodiment of the present invention the sequence of the second (poly)peptide of the fusion protein comprises residues 1 to 239 of SEQ ID NO: 6 or 1 to 226 of SEQ ID NO: 8 or a fluorescent mutant or fragment thereof.

In a more preferred embodiment of the present invention the mutant of the red fluorescent protein is mRFP1 as shown in SEQ ID NO: 17 or a protein or (poly)peptide encoded by the sequence of any one of SEQ ID NOs 17 and 24 to 27.

In an even more preferred embodiment of the present invention, the fusion protein has the sequence of any one of SEQ ID NOs: 18, 32, 20 or 22 or is encoded by a nucleic acid identical with or comprising the sequence of any one of SEQ ID NOs: 19, 33, 21 or 23.

The anti-GFP chromobody can be stably expressed in cells as an active monomer and has access to all tested subcellular compartments and structures. Aggregates, as described for a large number of intracellular expressed intrabodies like scFvs²⁶, were not detected. This intracellular functionality of the llama derived VHH domain suggests that its intrinsic stability (ΔG= 30 kJ/mol)²⁷ even in the absence of the conserved C22-C92 disulfide bond is sufficiently high to fold properly. The absence of an extra pair of cysteines in the CDRs in the epitope binding domain of llama derived HCAbs could then be a natural advantage over VHH domains from dromedaries, where a frequently occurring interloop disulfide bond is essential for antigen recognition^{9, 28}. The GFP chromobody, wherein one of the conserved cysteines in the VHH region has been mutated to serine shows not only a higher stability of the molecule, but surprisingly also a better accessibility of the molecule within the cell.

In another preferred embodiment of the present invention, the fusion protein of the present invention further comprises a targeting sequence selected from the group consisting of nuclear localization signal (NLS), endoplasmic reticulum import sequence, mitochondrial import sequence. An example of an NLS is the peptide sequence PKKKRKV (nuclear-localization signal (NLS) of the SV40 large T-antigen, D. Kalderon et al., 1984, Cell 39:499) which is capable of directing heterologous proteins into the nucleus. An example of the "Endoplasmatic Reticulum Import Sequence" is the peptide MMSFVSLLLVGILFWATEAEQLTRCVFQ (ER localisation signal of immunoglobulin light chain, Blobel G. & Dobberstein B., 1975 J.Cell.Biol. 67:835 - 851) which is capable of directing heterologous proteins into the lumen of the ER. An example of a "Mitochondrial Import Sequence" is the peptide MLSLRQSIRFFRPATRTLCSSRYLL (Neupert W. 1997 Annu. Rev. Biochem. 66:863-917) which is capable of directing heterologous proteins into the mitochondrium.

The present invention also relates to a fragment of the fusion protein of the present invention capable of specifically binding to its epitope, said fragment consisting or comprising of: framework 1, CDR1, framework 2, CDR2, framework 3 and CDR3, encoded by the nucleic acid molecule of any one of SEQ ID NOs: 11 or encoded by a nucleic acid molecule with at least 95% and most preferably at least 98% sequence identity or a fragment thereof. It is preferred that the changes with regard to the reference sequence SEQ ID NO: 1 be located outside of the CDRs. In one embodiment of the present invention, however, particular mutations are introduced into at least one position of the CDR in order to modulate the affinity of the fragment to GFP. "Modulate the affinity" means increase or decrease the dissociation constant K_{D} of the complex of GFP and fragment.

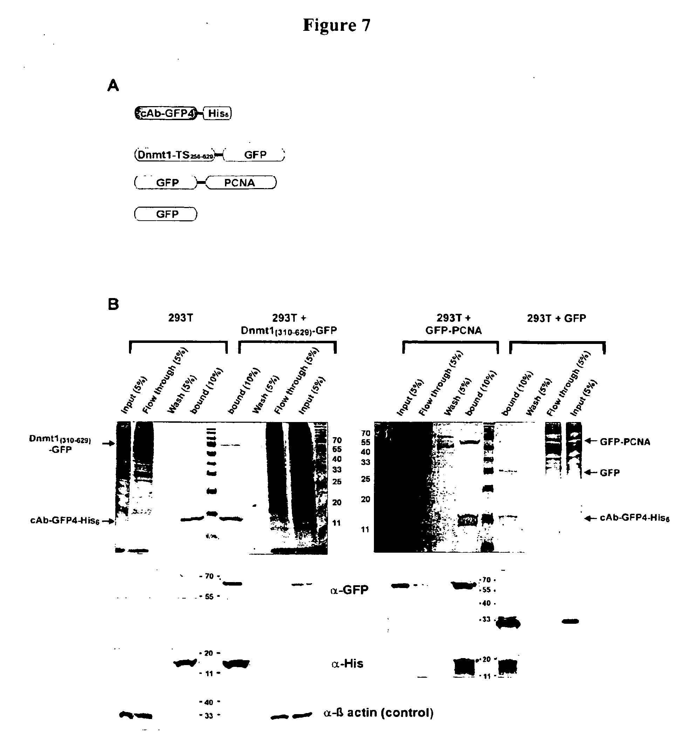

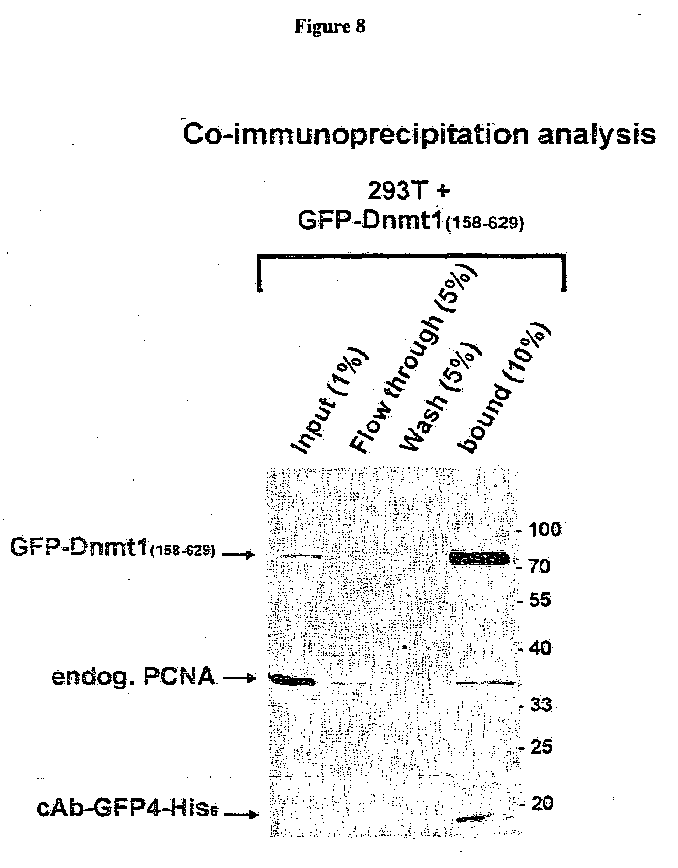

As illustrated in example 6 of the present invention, such a fragment is particularly effective in immunoprecipitation of GFP. The results described herein are rather surprising since this fragment, while not containing the Fc portion of conventional antibodies, is still capable of associating with protein A. The experiments shown herein demonstrate that this fragment is highly effective in conventional immunoprecipitation assays. Furthermore this fragment not only immunoprecipitates its antigen (as demonstrated in figure 7 B) but also interacting proteins (as shown in figure 8). The small molecular weight and the monomeric status of this antibody fragment enables a simple handling in biochemical approaches, starting with the high yield expression in a host cell like E.coli and a one step purification (shown in figure 6 A). In contrast to conventional antibodies this antibody fragment, due to its nature, does not interfere in applications like SDS-PAGE and/or a western blot. Conventional antibodies showing up a denatured light and heavy chain, which often interfere with the detection of the antigen and/or coprecipitated proteins either by immunodetection or mass spec analysis.

In another preferred embodiment of the present invention, the protein of the present invention further comprises (a) a tag selected from the group consisting of His tag, Strep tag, recognition site for biotinylation and optionally (b) the recognition site for a protease. Described is also a Hyc tag and GST tag. Generally speaking, any epitope with a corresponding high affinity antibody may be used as tag. Particularly preferred are myc tag (see, e.g., Kieke, 1997, Protein Eng. 10:1303-1310), His-tag (Pharmacia), or GST-tag (Pharmacia) or Strep-tag (see e.g. Skerra & Schmidt (1999) Biomolecular Engineering 16:79-86). One of the most commonly used approaches is to tag the recombinant protein at one of the termini with the enzyme glutathione S-transferase (GST) or a fragment thereof, which has high affinity for its natural ligand glutathione (which is the tripeptide γ-glutamylcysteinlyglycine, usually abbreviated GSH). The protein is purified using a resin with covalently attached GSH. After elution of contaminating proteins with buffer, the tagged protein is eluted with a GSH solution. Another popular method is to tag the protein at one of the termini with 6-10 His residues which confers on the recombinant protein the ability to bind to a Ni²⁺ resin. After elution of contaminating proteins with buffer, the tagged protein is competed off the Ni²⁺ column with a buffer solution containing imidazole (recall that the sidechain of His contains an imidazole ring).

The present invention also relates to a nucleic acid molecule encoding the fusion protein of the present invention or a fragment thereof. Said nucleic acid molecule may be an RNA or DNA molecule.

The present invention also relates to an expression vector comprising the nucleic acid molecule of the present invention. The expression vector may be a eukaryotic or prokaryotic expression vector, preferably a mammalian expression vector.

A typical mammalian expression vector contains the promoter element, which mediates the initiation of transcription of mRNA, the protein coding sequence, and signals required for the termination of transcription and polyadenylation of the transcript. Moreover, elements such as origin of replication, drug resistance gene, regulators (as part of an inducible promoter) may also be included. The *lac* promoter is a typical inducible promoter, useful for prokaryotic cells, which can be induced using the lactose analogue *isopropylthiol-b-D-galactoside*. ("IPTG"). For recombinant expression, the antibody fragment may be ligated between e.g. the PelB leader signal, which directs the recombinant protein in the periplasm and the gene III in a phagemid called pHEN4 (described in Ghahroudi et al, 1997, FEBS Letters 414:521-526). Additional elements might include enhancers, Kozak sequences and intervening sequences flanked by donor and acceptor sites for RNA splicing. Highly efficient transcription can be achieved with the early and late promoters from SV40, the long terminal repeats (LTRs) from retroviruses, e.g., RSV, HTLVI, HIVI, and the early promoter of the cytomegalovirus (CMV). However, cellular elements can also be used (e.g., the human actin promoter). Suitable expression vectors for use in practicing the present invention include, for example, vectors such as pSVL and pMSG (Pharmacia, Uppsala, Sweden), pRSVcat (ATCC 37152), pSV2dhfr (ATCC 37146) and pBC12Ml (ATCC 67109). Mammalian host cells that could be used include, human Hela, 293, H9 and Jurkat cells, mouse NIH3T3 and C127 cells, Cos 1, Cos 7 and CV1, quail QC1-3 cells, mouse L cells and Chinese hamster ovary (CHO) cells. Alternatively, the recombinant (poly)peptide can be expressed in stable cell lines that contain the gene construct integrated into a chromosome. The co-transfection with a selectable marker such as dhfr, gpt, neomycin, hygromycin allows the identification and isolation of the transfected cells. The transfected nucleic acid can also be amplified to express large amounts of the encoded (poly)peptide. The DHFR (dihydrofolate reductase) marker is useful to develop cell lines that carry several hundred or even several thousand copies of the gene of interest. Another useful selection marker is the enzyme glutamine synthase (GS) (Murphy et al. 1991, Biochem J. 227:277-279; Bebbington et al. 1992, Bio/Technology 10:169-175). Using these markers, the mammalian cells are grown in selective medium and the cells with the highest resistance are selected. As indicated above, the expression vectors will preferably include at least one selectable marker. Such markers include dihydrofolate reductase, G418 or neomycin resistance for eukaryotic cell culture and tetracycline, kanamycin or ampicillin resistance genes for culturing in *E. coli* and other bacteria. Representative examples of appropriate hosts include, but are not limited to, bacterial cells, such as *E. coli*, Streptomyces and *Salmonella typhimurium* cells; fungal cells, such as yeast cells; insect cells such as Drosophila S2 and Spodoptera Sf9 cells; animal cells such as CHO, COS, 293 and Bowes melanoma cells; and plant cells. Appropriate culture mediums and conditions for the above-described host cells are known in the art.

The recombinantly expressed polypeptide may contain additional amino acid residues in order to increase the stability or to modify the targeting of the protein. For instance, a region of additional amino acids, particularly charged amino acids, may be added to the N-terminus of the polypeptide to improve stability and persistence in the host cell, during purification, or during subsequent handling and storage. Also, peptide moieties may be added to the polypeptide to facilitate purification. Such regions may be removed prior to final preparation of the polypeptide. The addition of peptide moieties to polypeptides to engender secretion or excretion, to improve stability and to facilitate purification, among others, are familiar and routine techniques in the art. A preferred fusion protein comprises a heterologous region from immunoglobulin that is useful to stabilize and purify proteins. For example, EP-A- 0 464 533 (Canadian counterpart 2045869) discloses fusion proteins comprising various portions of constant region of immunoglobulin molecules together with another human protein or part thereof. In many cases, the Fc part in a fusion protein is thoroughly advantageous for use in therapy and diagnosis and thus results, for example, in improved pharmacokinetic properties (EP-A 0 232 262). On the other hand, for some uses it would be desirable to be able to delete the Fc part after the fusion protein has been expressed, detected and purified in the advantageous manner described. In drug discovery, for example, human proteins, such as hIL-5, have been fused with Fc portions for the purpose of high-throughput screening assays to identify antagonists of hIL-5. See, D. Bennett et al., J. Molecular Recognition 8:52-58 (1995) and K. Johanson et al., J. Biol. Chem. 270:9459-9471 (1995). The fusion proteins and proteins of the present invention can be recovered and purified from recombinant cell cultures by well-known methods including ammonium sulphate or ethanol precipitation, acid extraction, anion or cation exchange chromatography, phosphocellulose chromatography, hydrophobic interaction chromatography, affinity chromatography and/or hydroxyl apatite chromatography. Most preferably, fast protein liquid chromatography (FPLC) is used for purification, however, high performance liquid chromatography ("HPLC") may also be employed for purification.

The present invention also relates to a host cell comprising the nucleic acid molecule of the present invention and/or the expression vector of the present invention. The host cell may be a eukaryotic or prokaryotic host cell. Preferably host cells to be used for expression of the (poly)peptide, protein or fusion protein of the present invention are selected from the group consisting of bacterial cells including E.coli: XI1 blue, BL21, JM 109; lower eukaryotic cells including yeast Sacharomyces cerevisiae, Pichia Pastoris; or any cell or strain described in Sambrook and Russel, 2001, Molecular Cloning: A Laboratory Manual 3rd edition, Cold Spring Harbor Laboratory Press, Appendix 3.

Also described herein are fusion proteins according to the present invention or fragments thereof, which are obtainable from the host cell of the present invention or from the method of the present invention. The recombinant protein may be recovered from cells or cell culture supernatant by any suitable method known to the skilled person. Typical methods are described in Bollag, D.M. and Edelstein, S.J., 1991 (see Protein Methods Wiley-Liss, New York Methods in Enzymology, 1990, Guide to Protein Purification, Vol. 182, Academic Press, New York).

The specification also describes a method for generating a library of detectable, such as fluorescent, proteins capable of antigen binding, comprising the steps of the method as described above. A library generated by this method is also described. Such libraries allow a direct and fast screen for new detectable, such as fluorescent, proteins capable of antigen binding. The time consuming and expensive approach involving the traditional animal immunization can be bypassed. Also, less antigen is needed and adverse biological antigen effects during immunization can be avoided. These new proteins capable of antigen binding are immediately ready to be used, without any further experimental procedures like chemical crosslinking with e.g. fluorophores or chromogenic enzymes or molecular structures for detection or quantification. Fluorescent antibody fragments (chromobodies) identified in the initial screen can be further improved in their specificity and affinity by subsequent mutagenesis and further selection cycles.

Also described herein is a method for purifying the protein or fusion protein of the present invention, comprising (a) contacting a solution comprising the protein with a compound capable of specifically binding to the protein, wherein prior to or after binding, the compound is attached to a solid support; (b) washing the solid support of step (a) to remove unspecifically bound constituents; (c) eluting the fusion protein.

A "compound capable of specifically binding to the protein" may be an antibody or antibody fragment or an antigen for which the fusion protein of the present invention is specific. Solid supports that may be employed in accordance with the invention include filter material, chips, wafers, microtiter plates and beads, to name a few. Attachment means covalent or non-covalent attachment. The proteins and fusion proteins of the present invention may be solubilized in standard protein buffers such as PBS. 1xPBS with a pH of 7.5 is the preferred buffer, but other buffers may also be used (see Sambrook et al., Molecular Cloning: A Laboratory Manual 3rd edition, Cold Spring Harbor Labratory Press, Appendix A1.2, 2001). These other buffers may be selected by the skilled person using conventional methods known in the art. The step of washing may be performed at different salt strength in order to modify modulate the specificity of protein binding. For example in 1xPBS containing 150 mM - 1 M NaCl or KCl. The whole procedure is based on a standard protocol for e.g. Co-Immunoprecipitation provided by ProFound^{™} Co-Immunoprecipitation Kit Pierce, Rockford, USA. Any other protocol described for protein purification or identification of protein interactions and protein purification using antibodies may also be used. (References: Methods in Enzymology, 1990, Guide to Protein Purification, Vol. 182, Academic Press, New York; Harlow, E. and Lane , D. 1988 Antibodies, A Laboratory Manual, Chapter 11, Cold Spring Harbor, New York; Hermanson, G.T.,1992, Immobolized Affinity Ligands Techniques, Academic Press, Inc., San Diego,CA; Sambrook and Russel, 2001, Molecular Cloning: A Laboratory Manual 3rd edition, Cold Spring Harbor Laboratory Press, Appendix A1.2). In a preferred embodiment of the present invention, the fusion protein contains a tag sequence as defined above. For example any epitope with a corresponding high affinity antibody can be used as a tag, e.g., a myc tag (see, e.g., Kieke, 1997, Protein Eng. 10:1303-1310) or an His-tag (Pharmacia). See also Maier, 1998, Anal. Biochem. 259:68-73; Muller, 1998, Anal. Biochem. 259:54-61. In a more preferred embodiment of the present invention, said compound capable of specifically binding to the fusion protein is a compound specifically binding to the tag sequence. A number of tags are described in the specification of the present invention, all of these tags may be employed.

Also described herein is a method of identifying a detectable, such as fluorescent protein capable of specifically binding to an antigen, comprising the steps of: (a) providing a library created with the method described above (b) screening the library with an antigen; (c) selecting a library member that binds to the antigen. Screening of the library may be performed by any of the screening methods disclosed herein. Preferably the library is screened by Phage Display (reference and protocol: Clackson T. and Lowman H.B., 2004, Phage Display: A Practical Approach, Oxford University Press Inc., New York.)

The present specification also describes a method of detecting the presence, amount or subcellular location of an antigenic structure of interest on a cell, comprising the steps of: (a) contacting the cell with the fusion protein of as described above under conditions permitting the fusion protein to bind to said antigenic structure; (b) washing unbound fusion protein from the cell; (c) exposing the cell with light corresponding to the excitation wavelength of the fusion protein; and (d) detecting energy, such as fluorescence emitted from the cell, wherein the detected energy such as fluorescence indicates the presence of the structure on the cell surface. The small size of the antigen binding domain allows detection of otherwise inaccessible antigens on a cell surface. Furthermore, it allows higher resolution of complex biological structures. By the direct visualization of the antigen on a cell surface using the chromobody, a stoichiometric detection for linear quantification can take place.

The present specification also describes a method of detecting the presence or amount of an antigenic structure of interest, comprising the steps of: (a) contacting a sample suspected of containing the antigenic structure of interest with the fusion protein of the present invention under conditions permitting the fusion protein to bind to the protein of interest; (b) removing unbound fusion protein from the sample; (c) exposing the sample with light corresponding to the excitation wavelength of the fusion protein; and (d) detecting energy (such as fluorescence, phosphorescence, etc.) emitted from the sample, wherein the detected energy indicates the presence or amount of the antigenic structure. This biochemical approach allows a direct detection of an antigen using the fluorescent of the bound fusion protein. In contrast to conventional methods involving secondary antibody detection or enzymatic follow up reactions this provides a simple and fast method. Therefore this method is ideal for screening studies of cell extracts or liquid protein solutions for presence of the antigen of interest. The direct correlation of the fluorescence of the bound fusion protein and the antigen result in a simple and straightforward quantification of the antigen of interest in the probe. Preferably the detected energy is energy emitted from a fluorescent, phosphorescent or chromogenic protein.

The present specification describes that the fusion protein or the antigenic structure contained in the sample are coupled to a solid support.

The method comprises the additional step of performing chromatography prior to exposing the sample with light corresponding to the excitation wavelength of the fusion protein.

The present specification describes that said antigenic structure is selected from protein, protein modification, cofactor, small molecular compound, DNA and RNA.The specification furthermore relates to a method for purifying the fragment of the fusion protein as described above, comprising the steps of: (a) contacting a solution comprising said fragment with an antibody or a compound capable of specifically binding to the tag sequence, wherein prior to or after binding, the antibody or tag binding compound is attached to a solid support; (b) washing the solid support of step (a) to remove unspecifically bound constituents; (c) eluting the protein.

Furthermore described herein is a method for coupling the purified fragment to an immobilized support, comprising the steps of: (a) contacting a solution comprising the protein fragment with an antibody or compound capable of specifically binding to said protein; (b) attaching the antibody or compound bound prior to or after binding of said protein to a solid support; (c) washing the solid support of step (b) to remove unspecifically bound constituents.

The present specification also describes a method to purify an antigenic structure of interest, comprising a) contacting a sample containing said antigenic structure with I. a fusion protein directed to said antigenic structure, wherein said fusion protein comprises a first (poly)peptide sequence comprising the variable region of a heavy chain antibody of *Camelidae* and a second (poly)peptide sequence, which is a detectable protein, preferably derivable from a fluorescent or chromophoric protein, wherein said (1.) first (poly)peptide sequence is composed of framework 1, CDR1, framework 2, CDR2, framework 3 and CDR3, encoded by the nucleic acid sequence of SEQ ID NO: 2 or encoded by a nucleic acid sequence with at least 70% sequence identity or a fragment thereof; and (2.) second (poly)peptide sequence is a detectable protein, preferably (i) the green fluorescent protein derivable from *Aequorea victoria* encoded by the nucleic acid sequence of SEQ ID NO: 7, or a fluorescent mutant or fragment thereof; (ii) the red fluorescent protein derivable from *Discosoma* (DsRed) encoded by the nucleic acid sequence of SEQ ID NO: 9, or a fluorescent mutant or fragment thereof; or (iii) a functional homologue of (i) or (ii) with at least 80% sequence identity, preferably at least 85%, even more preferably at least 90%, even more preferably at least 95% and most preferably with at least 98% sequence identity; wherein said first (poly)peptide sequence is located N-terminally or C-terminally of said second (poly)peptide sequence, said sequences being optionally separated by a linker of at least one amino acid residue, or II. a (poly)peptide comprising the variable region of a heavy chain antibody of Camelidae, composed of framework 1, CDR1, framework 2, CDR2, framework 3 and CDR3, encoded by the nucleic acid sequence of SEQ ID NO: 2 or encoded by a nucleic acid sequence with at least 70% sequence identity or a fragment thereof; wherein the fusion protein is attached to a solid support; b) optionally washing the solid support of step a) to remove unspecifically bound constituents; c) eluting the antigenic structure.

The term "purify" as used throughout the present specification summarizes techniques used to isolate an antigenic structure. The term comprises, but is not limited to purification by immunoprecipitation, affinity or size exclusion chromatography, and the like and any combination thereof.

The present specification describes that said variable region comprises framework 1, CDR1, framework 2, CDR2, framework 3 and CDR3, and is encoded by the nucleic acid sequence of SEQ ID NO: 2 or is encoded by a nucleic acid sequence with at least 70%, more preferably 80%, more preferably at least 85%, even more preferably at least 90%, even more preferably at least 95% and most preferably at least 98% sequence identity or a fragment thereof. Preferably said fragment is a functional fragment, i.e. a fragment capable of specific antigen binding. "Specific binding" of may be described, for example, in terms of cross-reactivity. Preferably, specific binders are proteins (fusion proteins or fragments thereof) having a dissociation constant or K_{D} of less than 10⁻¹²M in particular 5X10⁻¹³M, 10⁻¹³M, 5X10⁻¹⁴M, 10⁻¹⁴M, 5X10⁻¹⁵M, and 10⁻¹⁵M.

Also described is that the sequence of (a) CDR1 consists of the residues shown in SEQ ID NO: 3; (b) CDR2 consists of the residues shown in SEQ ID NO: 4; and (c) CDR3 consists of the residues shown in SEQ ID NO: 5.

Further described is that said first (poly)peptide sequence of said fusion protein or the (poly)peptide comprising the variable region of a heavy chain antibody of *Camelidae* has the sequence of any one of SEQ ID NOs: 10, 30, 12 or 14 or is encoded by the sequence of any one of SEQ ID NOs: 11, 31, 13 or 15.

The present specification describes that said second (poly)peptide sequence comprises residues 1 to 239 of SEQ ID NO: 6 or 1 to 226 of SEQ ID NO: 8 or a fluorescent mutant or fragment thereof.

The present specification also describes that said mutant of the red fluorescent protein is mRFP1 as encoded by SEQ ID NO: 17 or any of the fluorescent DsRed mutants encoded by any one of SEQ ID NOs 17 and 24 to 27 or by a fragment thereof. Any fluorescent mutants derived from DsRed may be used in accordance with the teaching herein. In particular, the fusion proteins generated in accordance with the teaching herein may comprise a fluorescent second (poly)peptide sequence which comprises any of the aforementioned DsRed mutants or fluorescent mutants or fragments thereof.

The present specification also describes that the fusion protein of the present invention further comprises a targeting sequence selected from the group consisting of nuclear localization signal (NLS), endoplasmic reticulum import sequence, mitochondrial import sequence.

The present specification relates to a fusion protein having the sequence of any one of SEQ ID NOs: 18, 20 or 22 or being encoded by a nucleic acid molecule comprising the sequence of any one of SEQ ID NOs: 19, 21 or 23.

The present invention also relates to a method of specifically precipitating GFP or a GFP-tagged protein or other cellular antigens in a sample, comprising the step of contacting the sample with the anti-GFP chromobody of the present invention which is specific for GFP (SEQ ID NO: 18 and 32) of the present invention or a fragment thereof. This method may also be used to detect the latter protains or antigens. In a preferred embodiment of the present invention, said fragment comprises or consists of the amino acid residues as shown in SEQ ID NO: 10 or 30.

In a preferred embodiment of the present invention the anti-GFP chromobody or fragment thereof further comprises a tag selected form the group consisting of His-tag, Strep-tag, recognition site for biotinylation; and optionally the recognition site for a protease.

In another preferred embodiment of the present invention, GFP or the GFP-tagged protein or other cellular antigens and interacting proteins are pulled down (i.e. precipitated) by contacting a sample with the purified protein of the present invention or a fragment attached to a solid support. Co-precipitated proteins can then be analyzed by standard methods including mass spec analysis.

In a more preferred embodiment relating to any embodiment of the present invention, the fusion protein or fragment is attached to a solid support.

In a more preferred embodiment of the present invention said solid support is protein A. Protein A itself may be coupled to common resins such as sepharose.

In a more preferred embodiment of the present invention said fusion protein or fragment is bound to to said solid support. The term "bound to" comprises covalently coupled. In general, proteins can be coupled to matrices via primary amino groups.

In an even more preferred embodiment of the present invention said solid support is sepharose. Matrices or solid supports are for example NHS-activated Sepharose 4 (Amersham), Affi-Gel 10 or Affi-Gel 15 activated affinity media (BIO-RAD) or AminoLink Plus Coupling Gel (Pierce).Also described herein is a kit comprising the protein of the present invention and/or the nucleic acid molecule of the present invention and/or the expression vector of the present invention and/or the host cell of the present invention and/or the library of the present invention and optionally instructions for use.

The figures show:

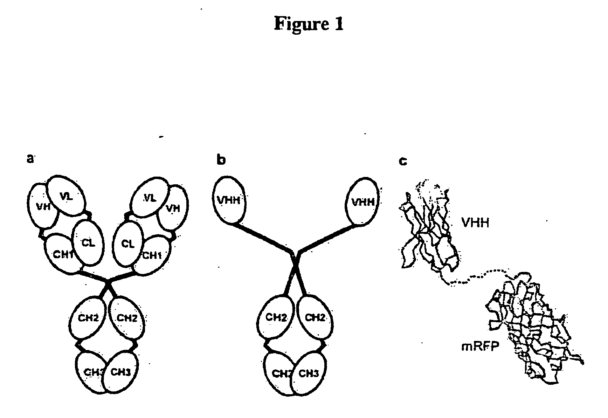

- **Figure 1:**: Schematic outline of a conventional IgG antibody in comparison with a llama derived heavy-chain IgG antibody (adapted from ref. 11). The antigen-binding domains of a conventional IgG **(a)** are composed of the variable light (VL) and heavy (VH) chains. In contrast, the heavy chain IgG antibody (**b**) of *Camelidae* is devoid of the light chain (VL & CL) and lacks also the CH1 domain. Antigen binding is carried out by three complementarity determining regions (CDRs) located in the variable domain of the heavy chain (VHH). For generating a fluorescent chromobody the small sized VHH is fused to a monomeric red fluorescent protein (mRFP1) and expressed in mammalian cells. **(c)** The putative structure of the chromobody was modelled based on the known crystal structures of a llama VHH¹¹ and the mRFP1³¹. Within this structure CDR1 is shown in yellow, CDR2 in cyan, CDR3 in green, mRFP1 in red and the flexible linker of 24 amino acids is shown as a black dotted line.

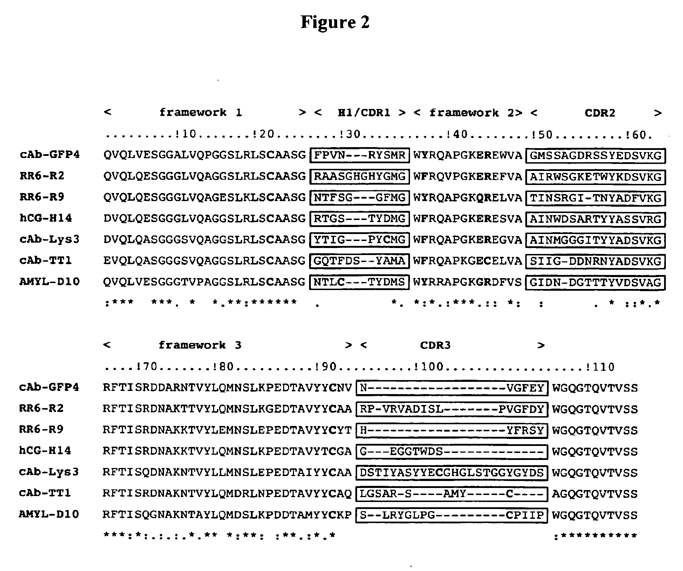

- **Figure 2:**: Alignment of seven VHH sequences derived from llama (lines 1-4) and camel (lines 5-7). From top to bottom: cAb-GFP4 (llama alpaca cAb raised against GFP, this work); RR6-R2 (llama cAb raised against red dye RR6³²); RR6-R9 (llama cAb raised against red dye RR6³³); hCG-H14 (llama cAb raised against hCG³⁴); cAb-Lys3 (camel cAb raised against lysozyme²⁸); cAb-TT1 (camel cAb raised against tetanus toxoid¹²), AMYL-D10 (camel cAb raised against porcine amylase³⁵). The CDR residues are coloured yellow, cyan and green for CDR 1 (H1), 2 and 3 respectively. Characteristic camelid VHH amino acid substitutions in framework 2 are highlighted in blue. Cysteines are shown in bold letters. Numbering is according to Kabat and Wu¹⁸. Identical (*), very similar (:) and similar (.) amino acid residues are indicated below.

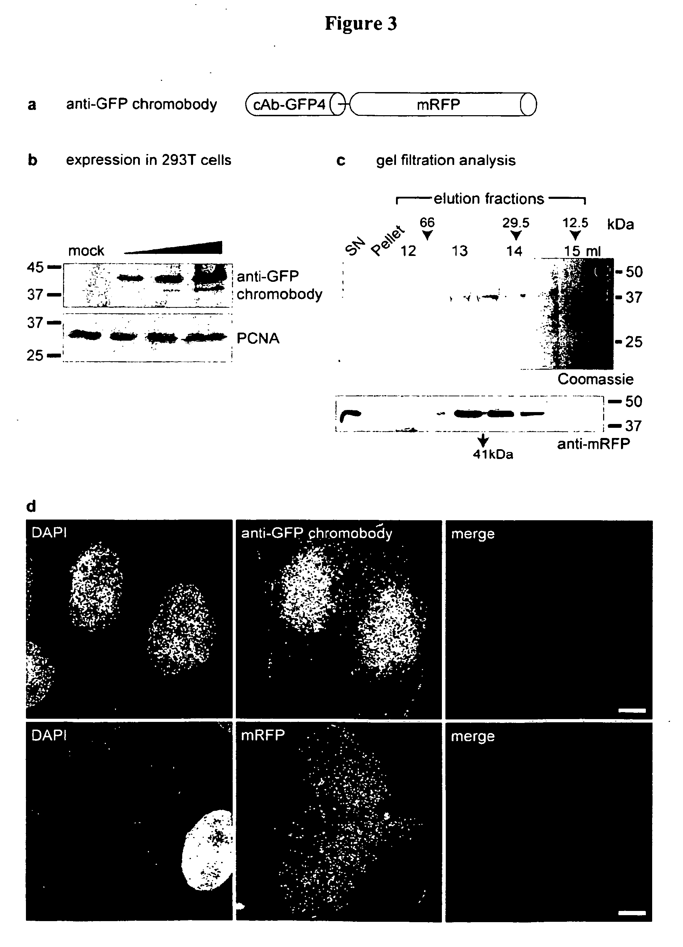

- **Figure 3:**: The anti-GFP chromobody is expressed as a stable monomeric protein in mammalian cells. **(a)** Schematic representation of the anti-GFP chromobody. **(b)** Total cell extracts of anti-GFP chromobody expressing 293T cells or mock treated cells were prepared 72 h after transfection. Increasing amounts of protein were analyzed by western blotting and detection with anti-mRFP1 antiserum. Lane 1; 30 µg of protein extract of mock transfected cells, lane 2-4, 10, 20 and 30 µg of protein extract of anti-GFP chromobody expressing cells. The predicted size of the chimeric protein is 41 kDa (upper panel). As a loading control, the blot was reincubated with an antibody against PCNA (lower panel). **(c)** Gel filtration analysis of extracts of mammalian cells expressing the chromobody. 1x10⁷ 293T cells expressing anti-GFP chromobody were lysed in the presence of 0.5% NP-40 and subjected to gel filtration on a Superose-12 column. Fractions were analyzed by SDS-PAGE and western blotting using an antibody against mRFP1. The anti-GFP chromobody elutes from the column in peak fractions corresponding to an apparent molecular mass of ~ 40 kDa. Arrows indicate the elution of calibration standards. **(d)** Expression of the anti-GFP chromobody in HeLa cells in the absence of any antigen shows a diffuse distribution of the protein throughout the cytoplasm and the nucleus (upper panel), which is comparable to the distribution of non-fused mRFP1 in the same cell type (lower panel).

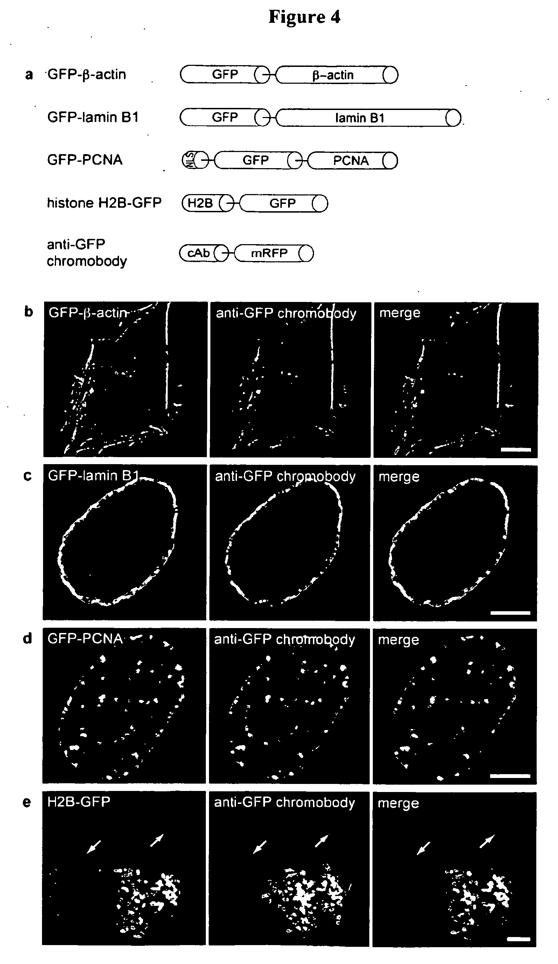

- **Figure 4:**: Targeting of the anti-GFP chromobody to binding sites of GFP fusion proteins in different cellular compartments and structures in living cells. **(a)** Schematic representation of the fusion proteins. **(b-e)** HeLa cells were transiently transfected with the indicated GFP fusion constructs and the anti-GFP chromobody expression plasmid (with the exception of **(e)** where HeLa cells stably expressing H2B-GFP were used). Representative images of live cells are shown. **(b)** Anti-GFP chromobodies colocalize with GFP-ß-actin on cytoskeletal actin filaments. **(c)** Anti-GFP chromobodies enter the nucleus and bind to GFP-lamin B1 incorporated into the nuclear lamina. **(d)** Anti-GFP chromobodies bind to a cell cycle regulated protein, GFP-PCNA, at replication foci. A time lapse series of a cell traversing S phase was taken. Shown is an image of the cell in mid S phase. **(e)** Anti-GFP chromobodies bind to the histone H2B-GFP incorporated into chromatin. The cell transfected with the anti-GFP chromobody is in prometaphase and single chromosomes are visible. Arrows indicate untransfected cells in meta- and interphase that do not express the chromobody. The absence of red fluorescence confirms that there is no unspecific bleed through from the GFP channel. Scale bars, 5 µm.

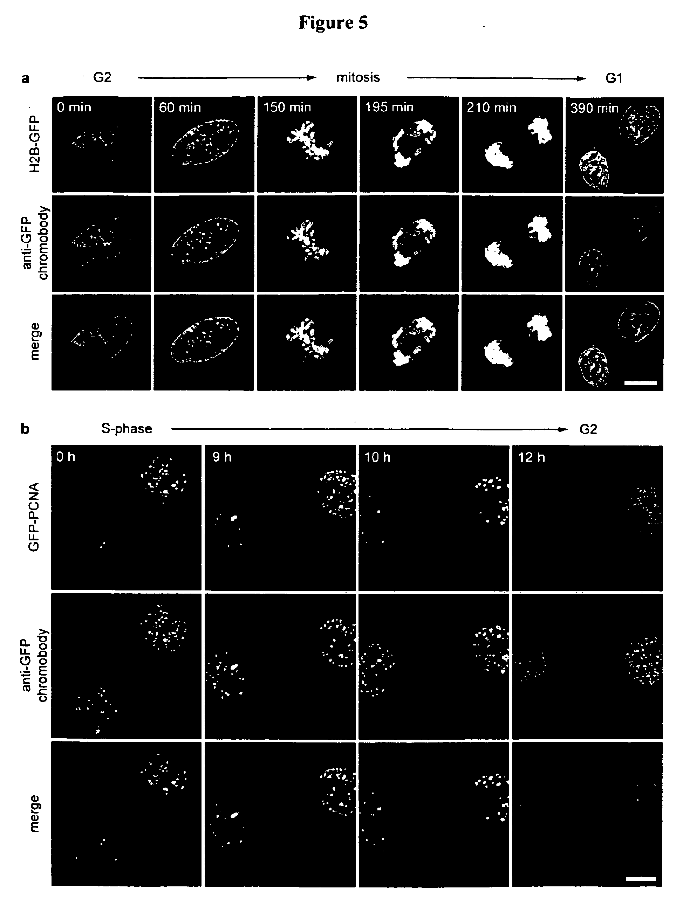

- **Figure 5:**: Antigen tracing with chromobodies. **(a)** Tracing of a chromatin protein throughout mitosis. Time lapse imaging of a HeLa cell stably expressing histone H2B-GFP transfected with anti-GFP chromobody. Selected frames from this time series are shown. At the time imaging was started (0 h), this cell was in late G2 phase. **(b)** Tracing of a component of the replication machinery throughout S phase until G2. Time lapse imaging of cotransfected HeLa cells expressing GFP-PCNA and anti-GFP chromobody. Selected frames from this series are shown. At the start of imaging (0 h) the cells were in early to mid S phase. Scale bars, 5 µm.
- **Figure 6:**: Purification of recombinant cAb-GFP4-His₆**(A)**. The recombinant expressed cAb-GFP4 antibody fragment can be purified in a one chromatography step. 5 ml of soluble *E.coli* protein extract was resuspended in binding buffer (1 x PBS, 500 mM NaCl, 20mM imidazol) and loaded onto a column containing 1 ml of NiNTA-resin. After extensive washing, bound protein was eluted by adding stepwise 1 ml of elution buffer (1 x PBS, 500 mM NaCl, 200mM imidazol). 2% of supernant, pellet after centrifugation, flow through and elution fraction were subjected to SDS-PAGE and stained with coomassie brilliant blue. The main fraction of cAb-GFP4 elutes in fraction 2 and 3, which were pooled and dialyzed against 1 x PBS and adjusted to a concentration of 1 µg/µl. **(B)** Gel filtration analysis of purified cAb-GFP antibody fragment. 10 µg of purified cAb-GFP4 was subjected to gel filtration on a Superose-12 column. Fractions were analyzed by SDS-PAGE and western blotting using an antibody against the C-terminal histidine (His₆) tag. The cAb-GFP4 antibody fragment elutes from the column in peak fractions corresponding to an apparent molecular mass of - 15 kDa. Arrows indicate the elution of calibration standards.
- **Figure 7:**: Immunoprecipitation of GFP tagged proteins using the purified cAb-GFP4 antibody fragment **(A)** Schematic representation of the fusion proteins. **(B)** 293T cells either mock treated or expressing GFP-fusion construct were lysed in the presence of 0.5% NP-40. The soluble protein extracts were subjected to immunoprecipitation by adding 20 µg respectively 10 µg of purified cAb-GFP4 coupled to protein A agarose beads. The single fractions containing input, flow through, wash and beads pellet were analysed by SDS-PAGE followed either by coomassie staining or immunoblot analysis. Overexpressed proteins were partially detectable in the input fraction. In the beads fraction the precipitated proteins were detectable as a clear band together with the cAb-GFP4 antibody fragment (indicated by arrows). To confirm that these bands are the expected GFP-fusion proteins respectively the antibody fragment, the gels were blotted and incubated with antibodies against GFP and the C-terminal histidine (His₆)-tag. The antibody signals reveals the nearly quantitative precipitation of the antigen, whereas no further unspecific protein bands occur in the gel showing the mock treated cells. As a control for the specificity of the immunprecipitation, the blots were reprobed with an antibody against ß actin. Corresponding signals appears only in the input and flow through lanes, demonstrating, that the cAb-GFP4 precipitates its antigen with a high specificity.
- **Figure 8:**: Co-immunoprecipitation analysis To confirm, that the cAb-GFP4 antibody fragment not only pull down it's epitope but also interacting proteins, co-immunprecipitation analysis were performed by expressing a GFP-tagged domain of Dnmt1 (aa 158 - 629), which is known to interact with PCNA. Immunprecipitation were carried out as described and the immunoblot was analyzed with antibodies against GFP and PCNA. While the precipitated GFP-Dnmt1 ₍₁₅₈₋₆₂₉₎ is clearly visible as a strong band at the corresponding molecular weight, also a significant fraction of the endogenous PCNA was coprecipitated.

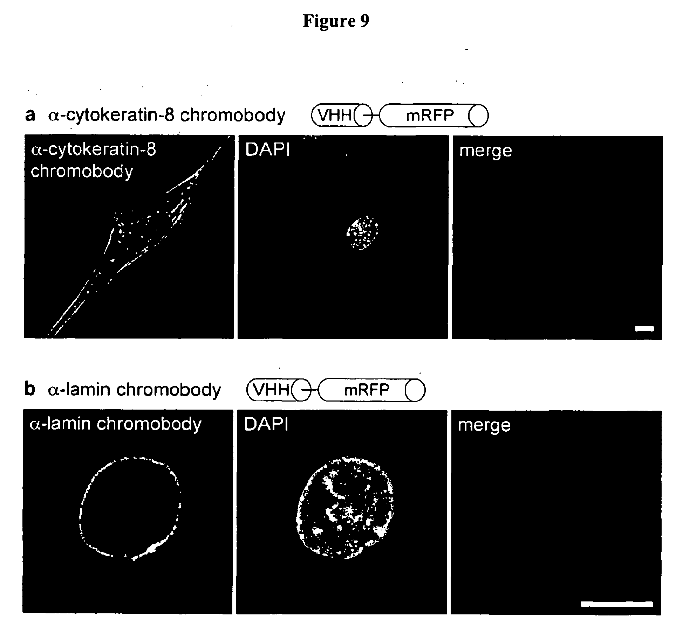

- **Figure 9:**: Recognition of subcellular structures by chromobodies raised against endogenous epitopes **(A)** Murine myoblast cells (C2C12) were transiently transfected with the anti-cytokeratin-8 chromobody expression plasmid. After five hours cells were subjected to live cell microscopy. To obtain a higher resolution cells were fixed, stained with DAPI to detect DNA and analyzed by confocal microscopy. A representative image of a live cell time lapse is shown. The red fluorescence on cytoplasmatic filaments indicates the recognition of cytokeratin fibres. **(B)** HeLa cells were transiently transfected with the anti-lamin chromobody expression plasmid. As described in 9(A) anti-lamin chromobody expressing cells were analyzed by confocal microscopy. The red fluorescence of the chromobody shows a clear surrounding structure of the nucleus indicating the staining of the nuclear lamina. Scale bars, 5 µm.

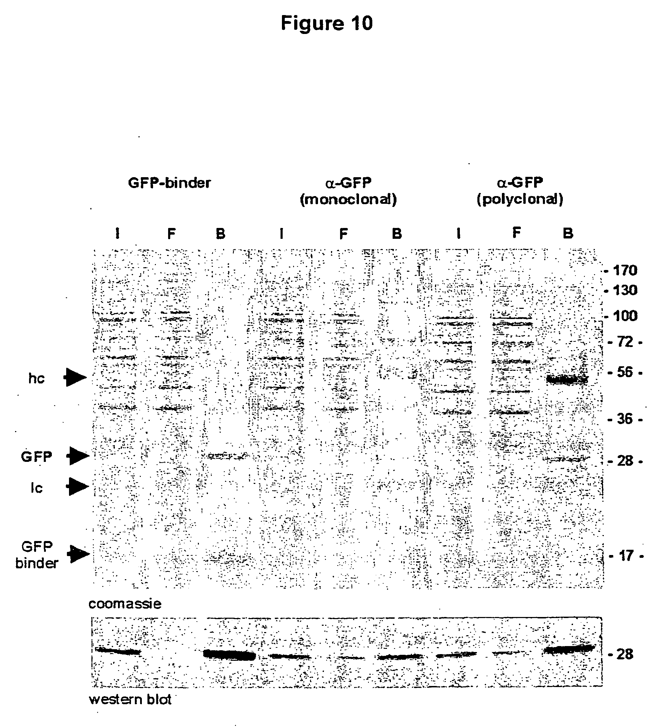

- **Figure 10:**: Comparison of the precipitation capacity of the GFP-binder with mono- and polyclonal GFP antibodies. Protein extracts of GFP producing 293T cells were incubated with 1 µg GFP-binder (cAbGFP4) or 2 µg of mono- or polyclonal GFP antibodies and immunocomplexes were precipitated with protein A/G sepharose. Aliquots of input (I), flow through (F), and bound fraction (B) were separated by SDS-PAGE followed either by coomassie staining (top) or immunoblot analysis using an anti-GFP antibody (bottom). Precipitated GFP, denatured heavy (hc) and light chains (Ic) of the IgGs and the GFP-binder (cAbGFP4) are marked by arrows.

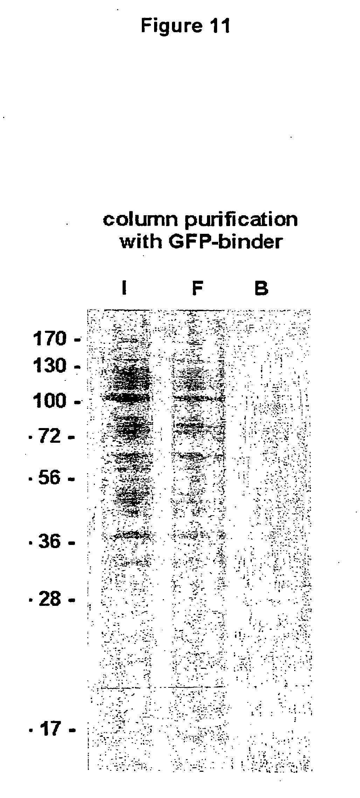

- **Figure 11:**: Column-based purification of GFP. Protein extracts of GFP producing 293T cells were loaded on a column containing GFP-binder (cAbGFP4) directly coupled to Sepharose 10. Bound proteins were eluted and aliquots of input (I), flow through (F), and bound fraction (B) were separated by SDS-PAGE and coomassie stained.

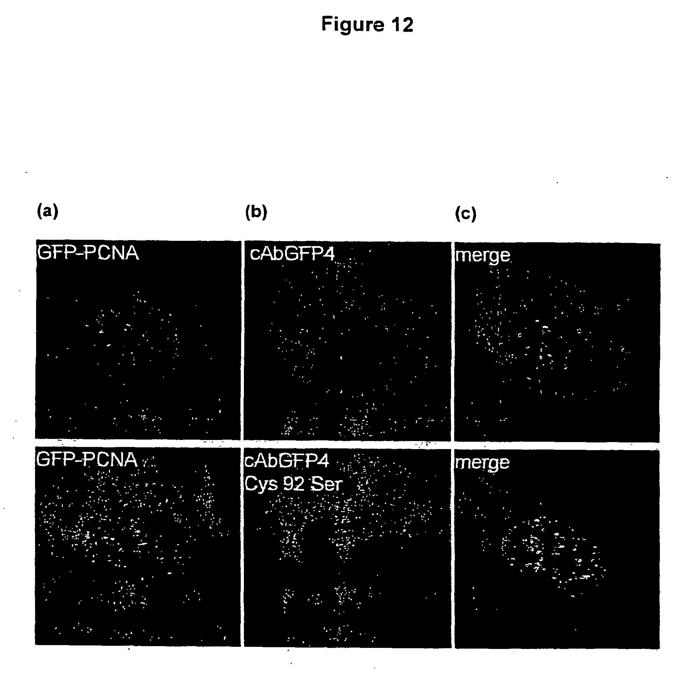

- **Figure 12:**: Comparison of expression of cAbGFP4 and cAbGFP4 (Cys92 Ser) in mammalian cells
Upper panel: Detection of intracellular distribution of cAbGFP4 (original) and cAbGFP4 (Cys 92 Ser) in human 293T cells in absence of antigen Lower panel: Detection of functionality in human 293T cells
(a) Signal of GFP-tagged PCNA
(b) Signal of cAbGFP4 (original) (upper panel) and cAbGFP4 (Cys 92 Ser) (lower panel)
(c) Merge of both signals indicates much better accessibility of cAbGFP4 (Cys 92 Ser) to the nucleus.

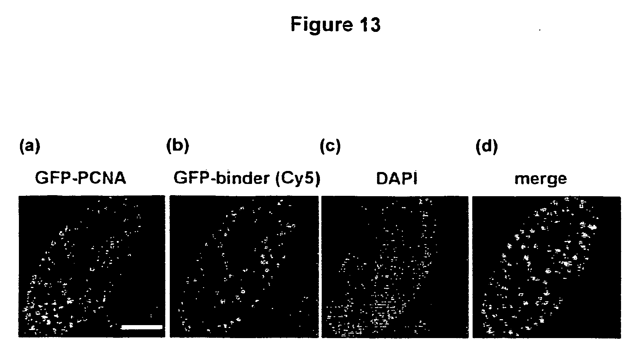

- **Figure 13:**: Activity and intracellular detection of recombinant GFP-binder (cAbGFP4) coupled to Cy5
Immunostaining of GFP-PCNA expressing HeLa cells with a GFP-specific VHH-domain (cAbGPF4, GFP-binder) coupled to Cy5-NHS (Amersham). Cells were fixed with 3.7 paraformaldehyde. Scale bar 5 µm.
(a) Signal of GFP-PCNA
(b) Signal of Cy5 coupled to the GFP-binder
(c) Staining with DAPI
(d) Merge of (a) and (b)

The Examples illustrate the invention:

### Example 1: Identification and characterization of a GFP specific VHH

To test the feasibility of creating fluorescent antibodies that can be expressed and traced in living cells, we chose GFP as the target molecule. GFP has already been fused to a variety of proteins with well-characterized subcellular localization and mobility and therefore provides a well-known and 'visible' antigen that can directly be compared with fluorescent antibodies. The variety of available GFP fusions allows us to test this approach in several subcellular compartments.

Lymphocytes were isolated from a Ilama alpaca (*Lama* pacos) immunised with purified recombinant GFP. The repertoire of the variable regions of the heavy-chain was amplified by PCR and cloned into a phage display vector. A VHH library of 10⁶ individual clones was obtained in *Escherichia coli* TG1 cells from which a VHH with specificity for GFP (cAb-GFP4) was retrieved after phage display and three rounds of panning. The sequence of the binder showed the amino acid substitutions in the framework-2 region (Fig. 1d) that are characteristic for a Ilama VHH: Y37, E44, R45 (numbering according to Kabat and Wu¹⁸). The nature of these amino acids in combination with the arginine residue on position 35 abrogates the interaction with a possible VL domain, and their hydrophilic character renders the domain soluble in aqueous medium. The CDR3 of the cAb-GFP4 with only six amino acids is unusually short for a VHH. The absence of additional cysteine residues besides the conserved C22 and C92 is a common feature among Ilamas VHHs distinguishing them from dromedary VHHs¹⁹.

To investigate the binding specificity of the cAb-GFP4, we constructed a C-terminal histidine (His₆)-tagged bacterial expression plasmid and purified the soluble recombinant antibody fragment from *Escherichia coli* WK6 cells. As expected, the cAb-GFP4 was highly expressed and yielded 0.7 - 1 mg of soluble VHH per 200 ml of IPTG-induced bacterial culture. The cAb-GFP4 eluted as a monomer with a molecular weight of 15 kDa in gel filtration analysis, which is consistent with the size predicted from its sequence (data not shown). Surface plasmon resonance measurements indicated a fast kinetic rate association of 7.68 x 10⁵ M⁻¹s⁻¹ and a slow dissociation of 1.74 x 10⁻⁴ s⁻¹ for the cAb-GFP4 and the GFP antigen interaction. The low dissociation constant (Kd= 0.23 nM) calculated from these values is consistent with an affinity-matured antibody recognizing its cognate antigen.

### Example 2: Generation and expression of a fluorescent version of the cAb-GFP4 in mammalian cells

To investigate intracellular antigen targeting by the cAb-GFP4 antibody fragment in living cells, we created a 'visible' antibody, termed chromobody, by fusing the coding region of the monomeric red fluorescent protein (mRFP1) at its C-terminus (Fig. 3a).The anti-GFP chromobody (SEQ ID NO: 18) is encoded by the nucleic acid sequence of SEQ ID NO: 19. Importantly, both GFP and mRFP1 do not interact with each other, which was analyzed by colocalization studies in cells coexpressing GFP and mRFP1 either alone or as fusion constructs (data not shown). The distinguishable excitation and emission maxima of the green fluorescent antigen and the red fluorescent antibody (eGFP: 498 nm / 516 nm; mRFP1: 584 nm / 607 nm) enabled the direct comparison of cellular distribution, mobility and dynamics of antigen and chromobody.

First we tested the expression and distribution of the chromobody in mammalian cells. Human 293T cells were transfected with an expression vector encoding the red fluorescent anti-GFP chromobody and subsequently total cell extracts were subjected to immunoblot analysis using a polyclonal anti-mRFP1 antiserum. In these cells we could detect a protein of 40 kDa, which corresponds to the expected size of the chromobody and was not present in untransfected cells (Fig. 3b). The absence of degradation products even at 72 h after transfection suggests that the anti-GFP chromobody is very stable in mammalian cells. Gel filtration analysis further showed that the fluorescent antibody fragment is present as a monomer when expressed in human cells (Fig. 3c).

To determine the subcellular distribution of the anti-GFP chromobody, HeLa cells expressing the chromobody were fixed, stained with DAPI (to highlight the DNA) and analyzed by confocal microscopy. Within 24 hours the cells displayed the red fluorescence of the chromobody distributed throughout the cytoplasm and the nucleus (Fig. 3d, upper panel), which is comparable to the distribution of mRFP1 alone (Fig. 3d, lower panel). The dispersed distribution of the chromobody clearly demonstrates the access to subcellular compartments and the absence of visible aggregation.

### Example 3: The fluorescent anti-GFP chromobody recognizes and targets its epitope in different cellular compartments

We then investigated the affinity and specificity of the fluorescent anti-GFP chromobody *in vivo* by analyzing its ability to recognize and bind GFP fusion proteins in different subcellular compartments and structures in living cells. A schematic overview of the GFP fusions used is shown in Fig. 4a.

To test a typical epitope localized in the cytoplasm we used GFP-ß-actin. This fusion protein is incorporated into growing actin filaments and allows the visualization of actin-containing structures in fixed and living cells²⁰. HeLa cells were cotransfected with GFP-ß-actin and anti-GFP chromobody expression vectors and 24 hours later analyzed by live cell microscopy. Fig. 4b shows a representative confocal image of a double-transfected cell where the green and the red fluorescence is detectable at the cytoskeleton. From this we conclude that GFP-ß-actin is correctly incorporated into the actin filaments, while the colocalization with the red fluorescence results from the targeting of GFP-ß-actin by the anti-GFP chromobody. This finding demonstrates that the anti-GFP chromobody efficiently recognizes and targets its epitope located in the cytoplasm even when it is part of highly organized structures.

In order to analyze the access and activity of the GFP specific chromobody in other subcellular compartments, we performed distribution and colocalization studies using a GFP labelled component of the nuclear envelope, GFP-lamin B1, as an epitope. Lamins are a major component of the nuclear lamina, a fibrous structure lining the nucleoplasmic surface of the nuclear membrane. Recent studies showed the correct assembly of GFP-lamin B1 into the nuclear lamina of mammalian cells²¹. Double transfected HeLa cells showed a clear colocalization of GFP-lamin B1 with anti-GFP chromobody (Fig. 4c) demonstrating that the chromobody can enter the nucleus, remains in a functional conformation and efficiently recognizes and binds its specific antigen.

To investigate antigen binding within the nucleoplasm we chose the proliferating cell nuclear antigen fused to GFP (GFP-PCNA). PCNA is a central component of the DNA replication machinery²². GFP-PCNA, similarly to its endogenous counterpart localizes at DNA replication sites^{23,24}. As described before we coexpressed GFP-PCNA and the anti-GFP chromobody in HeLa cells. The confocal mid-section shown in Fig. 4d reveals a clear costaining of a mid S phase replication pattern in a HeLa cell by GFP-PCNA and the anti-GFP chromobody.

Finally, we investigated the binding of the anti-GFP chromobody to a GFP-tagged chromatin protein. For this purpose we chose a stable HeLa cell line expressing a green fluorescent version of histone H2B (H2B-GFP). This fusion protein is known to be assembled into nucleosomes as the wild type H2B itself²⁵. After transfection and expression of the anti-GFP chromobody we detected the colocalization of the red chromobody with its green epitope in a prophase cell (Fig. 4e). The untransfected cells in this picture serve as a negative control and show that under these experimental conditions no fluorescence bleed through occurs between the green and the red channel.

These results show that the binding efficiency of the anti-GFP chromobody seems to be independent of the structural context and the subcellular localization of the epitope. To quantitate the degree of colocalization an overlap coefficient was calculated using the a colocalization plug-in of the ImageJ software yielding on average 90% - 97% overlap of the fluorescent intensities of antigen and chromobody. In this regard, we cannot distinguish whether the binding to the antigen occurs already in the cytoplasm or just after the epitope enters its appropriate position. However our data show that the observed GFP labelled proteins are correctly sorted and integrated in the cellular structures in the presence of the chromobody. Together these data demonstrate that the anti-GFP chromobody efficiently recognizes and binds its epitope in the cytoplasm as well as in subnuclear compartments.

### Example 4: The fluorescent anti-GFP chromobody traces its epitope through different stages of the cell cycle

Next, we investigated whether the anti-GFP chromobody can be used to trace proteins whose localization changes over the cell cycle and performed time lapse analysis tracing two different nuclear proteins, the histone protein H2B and the replication protein PCNA. As described above H2B-GFP is assembled into nucleosomes without affecting the cell cycle and allows high resolution imaging of mitotic chromosomes and interphase chromatin, revealing various chromatin condensation states in living cells²⁵. We transfected HeLa H2B-GFP cells with the anti-GFP chromobody coding expression vector and followed transfected and non-transfected cells over the cell cycle. Time lapse analysis (Fig. 5a) showed the colocalization of the anti-GFP chromobody with H2B-GFP persisting throughout mitosis. These results show that expression of the anti-GFP chromobody does neither affect H2B-GFP incorporation into chromatin nor cell cycle progression.

As a second target we chose PCNA, which constitutes a special challenge to live cell microscopy as it is an essential and central component of the replication machinery. GFP-PCNA, like the endogenous PCNA itself is concentrated at replication foci in the nucleus during early to late S phase and shows a diffused pattern in G1 and G2^{23, 24}. We followed cells expressing GFP-PCNA and anti-GFP chromobody from early S until G2 phase by taking confocal 3D image stacks every 20 min. The results are summarized in Fig. 5b and shown in full in Supplementary Information, Video online. Both, GFP-PCNA and the chromobody showed identical changes in their subnuclear distribution throughout S and G2 phases. From the highly specific association of the anti-GFP chromobody to GFP-PCNA and the observed colocalization throughout the S phase we concluded that the anti-GFP chromobody traces GFP-PCNA in the nucleus throughout the cell cycle without impairing DNA replication and S phase progression.

These time lapse analyses show that integral chromatin components like H2B as well as essential components of the replication machinery can be traced by a specific chromobody without affecting cell cycle progression and viability.

### Example 5: Methods used in examples 1 to 4

### A. GFP expression, purification and Ilama immunisation

*Escherichia coli* BL21DE3 cells were transformed with pRSETB-GFPS65T³⁰ (kindly provided by Roger Y. Tsien, UCSD) and overexpressed (His₆)-tagged GFPS65T was purified using ion-metal affinity chromatography according to the manufacturer's instructions (Talon, Clontech, CA, USA). One Ilama alpaca (*Lama pacos*) was immunised with recombinant purified GFP in Gerbu adjuvant according to the following scheme: day 0, 250 µg GFP; days 7, 14, 21, 28 and 35, 100 µg GFP; day 42, a bleed of 150 ml was collected.

### B. VHH library construction and selection of the GFP binder

Heparinized blood (36 ml) was diluted with prewarmed RPMI and layered on Lymphoprep^{™} (AXIS-Shield, Oslo, Norway) to purify the PBL cells according to the manufacturer's instructions. A total of 2x10⁷ PBL's were isolated and stored at - 80°C in aliquots of 6x10⁶ cells. The mRNA was extracted from 6x10⁶ lymphocytes and cDNA was synthesised with Superscriptll RNaseH- reverse transcriptase (Invitrogen, CA, USA) using an oligo-dt primer. The first PCR on the cDNA template was performed using CALL001 (5'-GTC CTG GCT GCT CTT CTA CA AGG-3') and CALL002 (5'-GGT ACG TGC TGT TGA ACT GTT CC-3') primers annealing at the leader sequence and at the CH2 exon of the heavy chains of all Ilama IgGs, respectively. The PCR products lacking the CH1 sequences (i.e. fragments with sizes between 650 -750 bp) were purified from an agarose gel using QIAquick PCR gel extraction kit (Qiagen-GmbH, Hilden, Germany). A nested PCR was done with an equimolar mixture of primers SM017 and SM018 (5'-CCA GCC GGC CAT GGC TCA GGT GCA GCT GGT GGA GTC TGG-3'and 5'-CCA GCC GGC CAT GGC TGA TGT GCA GCT GGT GGA GTC TGG-3', respectively) and CALL002 primer, and the PCR product repurified from agarose gel as described before. The VHH genes were finally re-amplified with primers A4short (5'-CAT GCC ATG ACT CGC GGC CAC GCC GGC CAT GGC-3') and 38 (5'-GGA CTA GTG CGG CCG CTG GAG ACG GTG ACC TGG GT-3') and digested with restriction enzymes Ncol and Notl to obtain sticky DNA ends. The fragment was purified with QIAquick, ligated into pHEN4 vector¹² cut with the same enzymes and the ligation mixture used to transform *Escherichia coli* TG1 cells. After overnight growth on LB/ampicillin plates, the bacterial colonies were scraped from the plates in LB, washed in the same medium and stored in LB/15% glycerol at -80°C until further use. A representative aliquot of this library was used to inoculate LB/ampicillin until cells reached the exponential growth phase before infection with M13K07 helper phages to express the cloned VHH. The phage displayed VHH library was panned for the presence of GFP binders on solid phase coated GFP (0.1 µg GFP / 100 µl per well) for three consecutive rounds. After the third round of selection, individual colonies were picked and expression of their soluble periplasmic protein was induced with 1 mM IPTG. The recombinant VHH extracted from the periplasm was tested for antigen recognition in an ELISA.

### C. Expression and purification of the single-domain antibody fragment

The VHH gene of the clone that scored positive in ELISA (cAb-GFP4) was recloned into the pHEN6 expression vector and used to transform *Escherichia coli* WK6 cells. Large scale production and purification followed the protocol described in van Koningbruggen *et al.*¹⁵

### D. Affinity measurements.

Affinity measurements were done by addition of different concentrations of GFP, ranging from 500 nM to 7.5 nM, to purified his-tailed VHH attached on a nickel-nitrilo triacetic acid biochip (Biacore International AB, Uppsala, Sweden) according to the manufacturer's description. The kinetic binding parameters k_{on,} k_{off} and K_{D} were determined with the BlAevaluation software (version 3.0).

### E. Mammalian expression constructs

The plasmid construct encoding a translational fusion of cAb-GFP4 and mRFP1 was derived by PCR amplification of the cAb-GFP4 coding region with primers gfp4#F (5'-GGG GGC TCG AGC CGG CCA TGG CCG ATG TGC AG-3') and gfp4#RC (5'-GGG GGA ATT CCT TGA GGA GAC GGT GAC-3'). The PCR product was purified as described and digested with restriction enzymes Xhol and EcoRI and ligated into a modified pEYFP-N1 vector (Clontech, CA, USA) were the YFP sequence had been replaced by the mRFP1 coding region³¹ (kindly provided by Roger Y. Tsien, UCSD). The anti-GFP chromobody (SEQ ID NO: 18) is encoded by the nucleic acid sequence of SEQ ID NO: 19

The plasmid constructs encoding translational fusions of GFP were as follows: GFP-ß-actin (Clontech, CA, USA), GFP-lamin B¹²¹ (kindly provided by Jan Ellenberg, EMBL), GFP-PCNA²³_{.}

### F. Mammalian cell culture and transfection

293T cells, HeLa cells and HeLa cells stably expressing H2B-GFP²⁵ ( kindly provided by Kevin F. Sullivan, The Scripps Research Institute) were cultured in DMEM supplemented with 10% FCS. 293T cells were transfected with plasmid DNA using TransFectin™ reagent (Bio-Rad Laboratories, Hercules, CA, USA) according to the manufacturer's guidelines and incubated overnight, 48 h or 72 h respectively before performing the immunoblots. For microscopy HeLa cells were grown to 50-70 % confluence either on 18x18 mm glass coverslips, 40mm round glass coverslips or on Lab-Tek™ Chambered Coverglass (Nunc-GmbH, Wiesbaden, Germany) and were transfected with the indicated expression constructs using Polyplus transfection reagent jetPEI™ (BIOMOL GmbH, Hamburg, Germany) according to the manufacturer's instructions. After 4-6 hours the transfection medium was changed to fresh culture medium and cells were then incubated for another 24 hours before performing live cell microscopy or fixation with 3.7 % formaldehyde in PBS for 10 min at room temperature. Fixed cells were permeabilized with 0.2 % Triton X-100 in PBS for 3 min, counterstained with DAPI and mounted in Vectashield (Vector Laboratories, CA, USA).

### G. Western blot analysis

Increasing protein amounts of total cell extracts of 293T cells either mock transfected or expressing the cAb-GFP4-mRFP1 construct were separated on a 12% SDS-PAGE and then electrophoretically transferred to nitrocellulose membrane (Bio-Rad Laboratories, CA, USA). The membrane was blocked with 3% milk in PBS and incubated overnight at 4°C with an anti-mRFP1 rabbit polyclonal antibody. After washing with PBS containing 0.1% Tween-20, the blots were incubated with anti-rabbit IgG antibody conjugated with horseradish peroxidase. Immunoreactive bands were visualized with ECL plus Western Blot Detection Kit (Amersham Biosciences, NJ, USA). As a loading control, membranes were reprobed with anti-PCNA antibody.

### H. Gel filtration

Extracts from 293T cells expressing the anti-GFP chromobody were subjected to gel filtration analysis. Briefly, 1x10⁷ cells were homogenized in 500µl lysis buffer (20 mM Tris/HCl pH 7.5, 150 mM NaCl, 0.5 mM EDTA, 2 mM PMSF, 0.5% NP40). After a centrifugation step (10 min, 20,000xg, 4°C) the clear supernatant was loaded on a Superose-12 column (Amersham Pharmacia Biotech, NJ, USA) and chromatographed at a flowrate of 0.4 ml/min in column buffer (20 mM Tris/HCl pH 7.5, 150 mM NaCl, 0.5 mM EDTA). Fractions (500µl each) were analyzed by SDS-PAGE and proteins were either stained with Coomassie Brilliant Blue R-250 or probed further by western blotting followed by incubation with an antibody against mRFP1 as described above. As calibration standards bovine serum albumin (66 kDa), carbonic anhydrase (29.5 kDa) and cytochrome c (12.5 kDa) were used.

### I. Microscopy

Live or fixed cells expressing fluorescent proteins were analyzed using a Leica TCS SP2 AOBS confocal microscope equipped with a 63x/1.4 NA Plan-Apochromat oil immersion objective. Fluorophores were excited with a 405 nm Diode laser, a 488 nm Ar laser, a 561 nm Diode-Pumped Solid-State (DPSS) laser. Confocal image stacks of living or fixed cells were typically recorded with a frame size of 512x512 pixels, a pixel size of 70-160 nm, a z step size of 280 nm and the pinhole opened to 1 Airy unit. A maximum intensity projection of several mid z-sections was then performed using ImageJ (Version 1.34, http://rsb.info.nih.gov/ij/). For long term live cell observation 40 mm diameter glass coverslips were mounted in a FCS2 live-cell chamber (Bioptechs, Butler, PA, USA) and maintained at 37°C. Light optical sections were acquired with a Zeiss LSM410 confocal laser scanning microscope using the 488 nm Ar laser line and the 543 nm HeNe laser line. Three mid z-sections at 1 µm intervals and the pinhole opened to 2 Airy Units were taken at indicated time intervals. Cells were followed up to 12 hours. Focus drift over time was compensated with a macro, which uses the reflection at the coverslip to medium interface as reference. After image acquisition, a projection of the three z-sections was performed from each time point. For colocalization analysis the "colocalization-finder" plug-in (Version 1.1) for ImageJ written by C. Laummonerie was used.

### Example 6: Immunoprecipitation assays using a VHH domain

Practically every molecular and cell biology laboratory has generated or used GFP-fusion constructs. By now fusion constructs are available to most, if not all known proteins. GFP fusions are the method of choice to study protein localization in living cells. We now have developed tools that offer a fast and efficient possibility to study which cellular proteins or factors associate with these GFP-fusion proteins.

For recombinant expression, we constructed a C-terminal histidine (His₆)-tagged bacterial expression plasmid and purified the soluble recombinant antibody fragment cAb-GFP4 from *E.coli* BI21 cells. The small antigen fragment cAb-GFP4 is expressed as a soluble protein to a yield of 5 mg / litre of induced *E.coli* culture. Figure 6A shows the purification of the cAb-GFP4-His₆ out of 50 ml IPTG induced bacterial culture performing immobilised affinity chromatography (IMAC) using a NiNTA resin column. The purified cAb-GFP4 eluted as a monomer of around 15 kDa in gel filtration analysis (figure 6 B).

For testing the capacity of the purified cAb-GFP4 fragment to recognize and precipitate its antigen GFP in the combination with different fusion constructs (Figure 7 A), we performed immunoprecipitation assays. The cAb-GFP4 was coupled to immobilized protein A. It has been described that the human IgG variable domain of subgroup III (V_{H} III) binds to the bacterial superantigen protein A^{1a}, ^{2a}. Protein A recognizes a nonlinear epitope within the V_{H} domain and it was proposed for specific purification of correctly folded V_{H} protein only ^{3a}. As the *Camelidae* variable heavy chain (VHHs) evolved within the V_{H} subgroup III, also the VHH domains raised in *Camelidae* are proposed to bind to protein A. Consistent with this, the purification of recombinant expressed VHH fragments using protein A agarose was reported ^{4a}

For immunoprecipitation experiments purified cAb-GFP4 was added to the soluble cell extract of 293T cells either mock treated, expressing the TS domain of Dnmt1 fused to GFP (Dnmt1 TS₂₅₆₋₆₂₉-GFP), GFP-PCNA or GFP alone. For precipitation protein A agarose was added, and precipitated proteins were analysed by coomassie staining or immunoblot analysis.

Both, the coomassie-staining and the immunoblot analysis shows an efficient precipitation of GFP-tagged proteins using the protein A coupled cAb-GFP4 antibody fragment. GFP can be at the C-terminus (Dnmt1 TS₂₅₆₋₆₂₉-GFP) or the N-terminus (GFP-PCNA) of the fusion protein. The immunoblot analysis reveals furthermore that the GFP fusion constructs are precipitated in an almost quantitative manner. This precipitation is highly specific as cAb-GFP4 coupled to protein A does not precipitates ß-actin used as a control. We concluded from this, that the recombinant expressed cAb-GFP4 efficiently recognizes and precipitates its antigen and therefore it is a valuable tool for biochemical approaches using GFP-tagged fusion proteins.

Furthermore we demonstrated, that the cAb-GFP4 fragment also can be used for co-immunoprecipitation. Figure 8 shows the co-precipitation of endogenous PCNA with a described PCNA interacting domain of Dnmt1 fused to GFP (GFP-Dnmt1₁₅₈-629).

To summarize, here we demonstrated the efficient use of an alpaca derived antibody fragment raised against GFP. We want to emphasis, that this fragment as it has a low molecular weight (15 kDa) is an outstanding new tool for analysing GFP tagged proteins using a biochemical techniques. It provides a range of exceptional advantages, as it can be expressed in high amounts in *E.coli* and one step purified. As we could show, it precipitates its antigen in a quantitative manner out of soluble cells extracts. There is no preference for N- or C-terminal GFP tagged proteins, which is consistent with our *in vivo* observations (see above). In contrast to conventional antibodies used for immunoprecipitation (mono- or polyclonal) the cAb-GFP4 fragment only appears as a single band in the gel and there is no interference with the detecting of proteins above 15 kDa. The simple and efficient expression of functional binders in E.coli and mammals makes it further possible to combine it with other functional elements. In particular, a fusion of a biotinylation sequence can be used for purification and or detection using commercially available streptavidin products.

### Example 7: Methods used in example 6

### A. GFP expression, purification and Ilama immunisation

*Escherichia coli* BL21 DE3 cells were transformed with pRSETB-GFPS65T^{5a} (kindly provided by Roger Y. Tsien, UCSD) and overexpressed (His₆)-tagged GFPS65T was purified using ion-metal affinity chromatography according to the manufacturer's instructions (Talon, Clontech, CA, USA). One Ilama alpaca (*Lama pacos*) was immunised with recombinant purified GFP in Gerbu adjuvant according to the following scheme: day 0, 250 µg GFP; days 7, 14, 21, 28 and 35, 100 µg GFP; day 42, a bleed of 150 ml was collected.

### B. VHH library construction and selection of the GFP binder.

Heparinized blood (36 ml) was diluted with prewarmed RPMI and layered on Lymphoprep^{™} (AXIS-Shield, Oslo, Norway) to purify the PBL cells according to the manufacturer's instructions. A total of 2x10⁷ PBL's were isolated and stored at - 80°C in aliquots of 6x10⁶ cells. The mRNA was extracted from 6x10⁶ lymphocytes and cDNA was synthesised with SuperscriptII RNaseH⁻ reverse transcriptase (Invitrogen, CA, USA) using an oligo-dT primer. The first PCR on the cDNA template was performed using CALL001 (5'-GTC CTG GCT GCT CTT CTA CA AGG-3') and CALL002 (5'-GGT ACG TGC TGT TGA ACT GTT CC-3') primers annealing at the leader sequence and at the CH2 exon of the heavy chains of all Ilama IgGs, respectively. The PCR products lacking the CH1 sequences (i.e. fragments with sizes between 650 -750 bp) were purified from an agarose gel using QIAquick PCR gel extraction kit (Qiagen-GmbH, Hilden, Germany). A nested PCR was done with an equimolar mixture of primers SM017 and SM018 (5'-CCA GCC GGC CAT GGC TCA GGT GCA GCT GGT GGA GTC TGG-3'and 5'-CCA GCC GGC CAT GGC TGA TGT GCA GCT GGT GGA GTC TGG-3', respectively) and CALL002 primer, and the PCR product repurified from agarose gel as described before. The VHH genes were finally re-amplified with primers A4short (5'-CAT GCC ATG ACT CGC GGC CAC GCC GGC CAT GGC-3') and 38 (5'-GGA CTA GTG CGG CCG CTG GAG ACG GTG ACC TGG GT-3') and digested with restriction enzymes Ncol and Notl to obtain sticky DNA ends. The fragment was purified with QlAquick, ligated into pHEN4 vector^{4a} cut with the same enzymes and the ligation mixture used to transform *Escherichia coli* TG1 cells. After overnight growth on LB/ampicillin plates, the bacterial colonies were scraped from the plates in LB, washed in the same medium and stored in LB/15% glycerol at -80°C until further use. A representative aliquot of this library was used to inoculate LB/ampicillin until cells reached the exponential growth phase before infection with M13K07 helper phages to express the cloned VHH. The phage displayed VHH library was panned for the presence of GFP binders on solid phase coated GFP (0.1 µg GFP / 100 µl per well) for three consecutive rounds. After the third round of selection, individual colonies were picked and expression of their soluble periplasmic protein was induced with 1mM IPTG. The recombinant VHH extracted from the periplasm was tested for antigen recognition in an ELISA.

### C. Expression and purification of the single-domain antibody fragment

The VHH gene of the clone that scored positive in ELISA (cAb-GFP4) was recloned into the pHEN6 expression vector and used to transform *Escherichia coli* BI21 cells. Large scale production and purification followed the protocol described in van Koningbruggen *et al.*^{15a}

### D. Mammalian cell culture and transfection

293T cells were cultured in DMEM supplemented with 10% FCS. 293T cells were transfected with plasmid DNA using TransFectin™ reagent (Bio-Rad Laboratories, Hercules, CA, USA) according to the manufacturer's guidelines and incubated overnight, 48 h or 72 h respectively before performing immunprecipitations.

### E. Immunoprecipitation

2x10⁷ 293T cells either mock treated or transiently expressing Dnmt1 (310-629)-GFP, GFP-PCNA or GFP were homogenized in 200µl lysis buffer (20 mM Tris/HCl pH 7.5, 150 mM NaCl, 0.5 mM EDTA, 2 mM PMSF, 0.5% NP40). After a centrifugation step (10 min, 20,000xg, 4°C) the clear supernatant was adjusted with dilution buffer (20 mM Tris/HCl pH 7.5, 150 mM NaCl, 0.5 mM EDTA, 2 mM PMSF) to 500 µl. 50µl were added to SDS-containing sample buffer (referred as input). 10 µl respectively 20 µl of purified cAb-GFP4-His₆ (conc. 1 µg/µl) were added and incubated for 2 hours on an end over end rotor at 4°C in the cold room. 25 µl of protein A agarose beads (Amersham Pharmacia, NJ, USA) were washed 3 times in 500 µl dilution buffer prior adding. Incubation continued for 1 hour. After a centrifugation step (2 min, 5000xg, 4°C) the beads pellet was resuspended in dilution buffer containing 300 mM NaCl. Supernatant was declared as flowthrough. The washing step was performed twice including changing the cups. 50µl (10%) was removed referred as wash. After the last washing step, the beads pellet were incubated in 2x SDS-containing sample buffer, boiled for 10 min at 95°C.

### F. Western blot analysis.

After boiling the beads, the soluble supernatants as well as the input, flowthrough and wash fractions were separated on a 12% SDS-PAGE and then either stained with coomassie or electrophoretically transferred to nitrocellulose membrane (Bio-Rad Laboratories, CA, USA). The membrane was blocked with 3% milk in PBS and incubated overnight at 4°C with corresponding antibodies. After washing with PBS containing 0.1% Tween-20, the blots were incubated with the appropriate secondary antibody conjugated with horseradish peroxidase. Immunoreactive bands were visualized with ECL plus Western Blot Detection Kit (Amersham Biosciences, NJ, USA).

### G. Gel filtration

10 µg of the purified cAb-GFP-His₆ antibody fragment was subjected to gel filtration analysis. 100 µl were loaded on a Superose-12 column (Amersham Pharmacia Biotech, NJ, USA) and chromatographed at a flowrate of 0.4 ml/min in column buffer (20 mM Tris/HCl pH 7.5, 150 mM NaCl, 0.5 mM EDTA). Fractions (500 µl each) were analyzed by SDS-PAGE, blotted and probed with an antibody against a C-terminal His6-tag (Invitrogen). As calibration standards bovine serum albumin (66 kDa), carbonic anhydrase (29.5 kDa) and cytochrome c (12.5 kDa) were used.

### Example 9: In vivo expressed fluorescent chromobodies efficiently targets endogenous proteins

To investigate whether intracellular expressed chromobodies can also recognize endogenous epitopes we generated chromobodies against cytoplasmic and nuclear antigens.

Single chain antibodies against nuclear and cytoplasmic proteins were raised in camel. VHH domains of two strong binders against cytokeratin-8 and lamin Dm0 were amplified by PCR with primers VHH (BgIII)#F (5'-GGG GAG ATC TCC GGC CAT GGC TCA GGT GCA G-3') and gfp4#RC (5'-GGG GGA ATT CCT TGA GGA GAC GGT GAC-3'). The PCR product was purified as described and digested with restriction enzymes Bglll and EcoRI and ligated into a modified pEYFP-N1 vector (Clontech, CA, USA) were the YFP sequence had been replaced by the mRFP1 coding region [31] (kindly provided by Roger Y. Tsien, UCSD).

To analyze a cytoplasmic antigen we fused a VHH against cytokeratin-8 to mRFP1 to generate a anti-cytokeratin-8 chromobody. This anti-cytokeratin-8 chromobody (SEQ ID NO: 20) is encoded by the nucleic acid sequence of SEQ ID NO:21. Murine myoblasts were transfected with an expression vector coding for the anti-cytokeratin-8 chromobody. Within 12 to 24 hours filamentous structures in the cytoplasm of C2C12-cells expressing the anti-cytokeratin-8 chromobody were detectable by the red fluorescence. Accordingly, cells were fixed, DNA counterstained with DAPI and analyzed by confocal microscopy to obtain a higher resolution (Fig. 9A).

Despite a substantial fluorescent background single filamentous structures could be detected. The high level of dispersed chromobody could be caused by a shifted ratio between antigen and chromobody. As cytokeratin-8 is expressed in murine myoblast, the expression level is rather low, which results in an excess of unbound chromobodies. Nevertheless, in comparison to the dispersed distribution of a chromobody in the absence of an antigen (Fig. 3D, upper panel) and also of mRFP1 alone (Fig. 3D, lower panel) the red fluorescence of the chromobody shows a accentuated pattern in the cytoplasm, which reflects the filamentous structures of cytokeratin-8.

In order to analyse an endogenous nuclear epitope we generated an anti-lamin chromobody. Therefore we fused an antibody fragment directed against lamin to mRFP1. The anti-lamin chromobody (SEQ ID NO: 22) is encoded by the nucleic acid sequence of SEQ ID NO: 23. In this case we took the advantage of a recently identified single chain antibody binding fragment raised against drosophila lamin Dm0. Due to the high conservation we anticipated a possible recognition of the mammalian orthologue. For localization studies HeLa cells were transfected with the anti-lamin chromobody expression vector. HeLa cells expressing the anti-lamin chromobody were fixed within 12-24 hours after transfection, stained with DAPI and subjected to confocal microscopy (Fig. 9B). Shown is a representative cell where the typical lamin structure, surrounding the nucleus, is highlighted by the red fluorescence of the chromobody. Only a low background of dispersed distributed chromobody was observable. This demonstrates a high specific recognition of the epitope by the anti-lamin chromobody which is comparable to the targeting of lamin B1-GFP by the anti-GFP chromobody as shown in figure 4C.

These data demonstrates, that endogenous epitopes like the cytoplasmatic localized cytokeratin-8 as well as the nuclear lamin protein can be specifically and efficiently visualized by intracellular expressed chromobodies. The cellular proteins used in this study are examples only, any other e.g. cellular protein can be detected in the same manner by using specific chromobodies (or fluorobodies). Importantly, the examples demonstrate that no limitation with regard to the subcellular location, in particular compartmentalisation is to be expected.

### Example 10: Comparison of expression of cAbGFP4 and cAbGFP4 (Cys92 Ser) in mammalian cells

cAbGFP4 was mutated to increase solubility and to decrease the tendency for aggregation of the protein due to formation of unspecific disulphide bridges. Therefore the cysteine at position 98 of SEQ ID NO: 18 was altered to serine (SEQ ID NO: 32).

### (A) Detection of intracellular distribution

Both Proteins were expressed in 293T cells. As depicted in figure 12 (upper panel) cAbGFP4 (Cys 92 Ser) is more evenly distributed within the human cell compared to original cAbGFP4 if no antigen is present. Remarkably, the nucleus is accessible to a much higher percentage of cAbGFP4 (Cys 92 Ser) compared to original cAbGFP4. This indicates higher solubility as well as the absence of unspecific aggregation, since one of two cysteines present in the molecule was mutated to prevent formation of unwanted disulphide bridges.

### (B) Detection of functionality in human 293T cells

Figure 12 (lower panel) clearly shows that in the presence of GFP-PCNA as antigen in human cells cAbGFP4 (Cys92Ser) binds its antigen more efficiently than the original cAbGFP4 molecule. The amount of free molecule is much lower in the former case.

### Example 11: Comparison of the precipitation capacity of the GFP-binder with mono- and polyclonal GFP antibodies

Protein extracts of GFP producing 293T cells were incubated with 1 µg GFP-binder (cAbGFP4) or 2 µg of mono- or polyclonal GFP antibodies and immunocomplexes were precipitated with protein A/G sepharose. Aliquots of input (I), flow through (F), and bound fraction (B) were separated by SDS-PAGE. Figure 10 illustrates the results after coomassie staining (top) or immunoblot analysis using an anti-GFP antibody (bottom). Precipitated GFP, denatured heavy (hc) and light chains (Ic) of the IgGs and the GFP-binder (cAbGFP4) are marked by arrows. It is clearly shown that the precipitation/purification of the antigen GFP with the GFP-binder is much more specific than with either monoclonal or polyclonal antibodies directed to GFP. No band other than those corresponding to GFP and the GFP-binder are visible on the gel.

### Example 12: Column-based purification of GFP

Protein extracts of GFP producing 293T cells were loaded on a column containing GFP-binder (cAbGFP4) covalently coupled to Sepharose 10 (Amersham). Bound proteins were eluted and aliquots of input (I), flow through (F), and bound fraction (B) were separated by SDS-PAGE and coomassie stained. As depicted in Figure 11 the protein of interest, in this case GFP could be enriched and purified

### Example 13: Activity and intracellular detection of recombinant GFP-binder (cAbGFP4) coupled to Cy5

The recombinant GFP-binder (cAbGFP4) was purified by immobilized affinity chromatography (IMAC) and the protein concentration was adjusted to 1 mg/ml. 1 ml of the protein solution was coupled to the fluorescent NHS-ester Cy5 (Amersham) according to the manufacturer's instruction. The coupled protein was subjected to gel filtration on a Superdex-75 column (Amersham) exchanging the coupling buffer (0.1 M NaHCO3) to 1xPBS. For immunostainings the purified GFP-binder-Cy5 was used in concentration of 50 to 100 ng/ml. Figure 13 shows an immunostaining of GFP-PCNA expressing HeLa cells with a GFP-specific VHH-domain (cAbGPF4, GFP-binder) coupled to Cy5-NHS (Amersham). Cells were fixed with 3.7 paraformaldehyde. Scale bar 5 µm. The merge of the two signals arising from GFP-PCNA and the Cy5 emission of the GFP-binder-Cy5 fusion in (a) and (b) shows colocalization of both proteins (Figure 13(d)). This demonstrates the suitability of fluorescent dyes for the applications of the present invention.

### References

1. Biocca, S., Neuberger, M.S. & Cattaneo, A. Expression and targeting of intracellular antibodies in mammalian cells. Embo J 9, 101-108 (1990).
2. Cattaneo, A. & Biocca, S. The selection of intracellular antibodies. Trends Biotechnol. 17, 115-121 (1999).
3. Biocca, S., Pierandrei-Amaldi, P. & Cattaneo, A. Intracellular expression of anti-p21 ras single chain Fv fragments inhibits meiotic maturation of xenopus oocytes. Biochem: Biophys. Res. Commun. 197, 422-427 (1993).
4. Biocca, S., Neuberger, M.S. & Cattaneo, A. Expression and targeting of intracellular antibodies in mammalian cells. Embo J. 9, 101-108 (1990).
5. Marasco, W.A., Chen, S., Richardson, J.H., Ramstedt, U. & Jones, S.D. Intracellular antibodies against HIV-1 envelope protein for AIDS gene therapy. Hum. Gene. Ther. 9, 1627-1642 (1998).
6. Cardinale, A., Lener, M., Messina, S., Cattaneo, A. & Biocca, S. The mode of action of Y13-259 scFv fragment intracellularly expressed in mammalian cells. FEBS Lett. 439, 197-202 (1998).
7. Kontermann, R.E. Intrabodies as therapeutic agents. Methods 34, 163-170 (2004).
8. Hamers-Casterman, C. et al. Naturally occurring antibodies devoid of light chains. Nature 363, 446-448 (1993).
9. Muyldermans, S., Atarhouch, T., Saldanha, J., Barbosa, J.A. & Hamers, R. Sequence and structure of VH domain from naturally occurring camel heavy chain immunoglobulins lacking light chains. Protein Eng. 7, 1129-1135 (1994).
10. Sheriff, S. & Constantine, K.L. Redefining the minimal antigen-binding fragment. Nat. Struct. Biol. 3, 733-736 (1996).
11. Muyldermans, S. Single domain camel antibodies: current status. J. Biotechnol. 74, 277-302 (2001).
12. Arbabi Ghahroudi, M., Desmyter, A., Wyns, L., Hamers, R. & Muyldermans, S. Selection and identification of single domain antibody fragments from camel heavy-chain antibodies. FEBS Lett. 414, 521-526 (1997).
13. van der Linden, R.H. et al. Comparison of physical chemical properties of Ilama VHH antibody fragments and mouse monoclonal antibodies. Biochim. Biophys. Acta 1431, 37-46 (1999).
14. Lauwereys, M. et al. Potent enzyme inhibitors derived from dromedary heavy-chain antibodies. Embo J. 17, 3512-3520 (1998).
15. van Koningsbruggen, S. et al. Llama-derived phage display antibodies in the dissection of the human disease oculopharyngeal muscular dystrophy. J. Immunol. Methods 279, 149-161 (2003).
16. Cortez-Retamozo, V. et al. Efficient tumor targeting by single-domain antibody fragments of camels. Int. J. Cancer 98, 456-462 (2002).
17. Jobling, S.A. et al. Immunomodulation of enzyme function in plants by single-domain antibody fragments. Nat. Biotechnol. 21, 77-80 (2003).
18. Kabat, E.A. & Wu, T.T. Identical V region amino acid sequences and segments of sequences in antibodies of different specificities. Relative contributions of VH and VL genes, minigenes, and complementarity-determining regions to binding of antibody-combining sites. J. Immunol. 147, 1709-1719 (1991).
19. Conrath, K.E., Wernery, U., Muyldermans, S. & Nguyen, V.K. Emergence and evolution of functional heavy-chain antibodies in Camelidae. Dev. Comp. Immunol. 27, 87-103 (2003).
20. Westphal, M. et al. Microfilament dynamics during cell movement and chemotaxis monitored using a GFP-actin fusion protein. Curr. Biol. 7, 176-183 (1997).
21. Daigle, N. et al. Nuclear pore complexes form immobile networks and have a very low turnover in live mammalian cells. J. Cell. Biol. 154, 71-84 (2001).
22. Warbrick, E. The puzzle of PCNA's many partners. Bioessays 22, 997-1006 (2000).
23. Leonhardt, H. et al. Dynamics of DNA replication factories in living cells. J. Cell. Biol. 149, 271-280 (2000).
24. Sporbert, A., Gahl, A., Ankerhold, R., Leonhardt, H. & Cardoso, M.C. DNA polymerase clamp shows little turnover at established replication sites but sequential de novo assembly at adjacent origin clusters. Mol. Cell 10, 1355-1365 (2002).
25. Kanda, T., Sullivan, K.F. & Wahl, G.M. Histone-GFP fusion protein enables sensitive analysis of chromosome dynamics in living mammalian cells. Curr. Biol. 8, 377-385 (1998).
26. Cardinale, A., Filesi, I., Mattei, S. & Biocca, S. Intracellular targeting and functional analysis of single-chain Fv fragments in mammalian cells. Methods 34, 171-178 (2004).
27. Saerens, D. et al. Identification of a Universal VHH Framework to Graft Non-canonical Antigen-binding Loops of Camel Single=domain Antibodies. J. Mol. Biol. In Press (2005).
28. Desmyter, A. et al. Crystal structure of a camel single-domain VH antibody fragment in complex with lysozyme. Nat. Struct. Biol. 3, 803-811 (1996).
29. Riechmann, L. & Muyldermans, S. Single domain antibodies: comparison of camel VH and camelised human VH domains. J. Immunol. Methods 231, 25-38 (1999).
30. Heim, R., Cubitt, A.B. & Tsien, R.Y. Improved green fluorescence. Nature 373, 663-664 (1995).
31. Campbell, R.E. et al. A monomeric red fluorescent protein. Proc. Natl Acad. Sci. U S A 99, 7877-7882 (2002).
32. Spinelli, S. et al. Camelid heavy-chain variable domains provide efficient combining sites to haptens. Biochemistry 39, 1217-1222 (2000).
33. Spinelli, S. et al. Domain swapping of a Ilama VHH domain builds a crystal-wide beta-sheet structure. FEBS Lett. 564, 35-40 (2004).
34. Spinelli, S. et al. The crystal structure of a Ilama heavy chain variable domain. Nat. Struct. Biol. 3, 752-757 (1996).
35. Desmyter, A. et al. Three camelid VHH domains in complex with porcine pancreatic alpha-amylase. Inhibition and versatility of binding topology. J. Biol. Chem. 277, 23645-23650 (2002).

### Additional references

1a. Sasso, E.H., Silverman, G.J. & Mannik, M. Human IgA and IgG F(ab')2 that bind to staphylococcal protein A belong to the VHIII subgroup. J Immunol 147, 1877-1883 (1991).
2a. Riechmann, L. & Davies, J. Backbone assignment, secondary structure and protein A binding of an isolated, human antibody VH domain. J Biomol NMR 6, 141-152 (1995).
3a. Riechmann, L. & Muyldermans, S. Single domain antibodies: comparison of camel VH and camelised human VH domains. J. Immuno/. Methods 231, 25-38 (1999).
4a. Arbabi Ghahroudi, M., Desmyter, A., Wyns, L., Hamers, R. & Muyldermans, S. Selection and identification of single domain antibody fragments from camel heavy-chain antibodies. FEBS Lett. 414, 521-526 (1997).
5a. Heim, R., Cubitt, A.B. & Tsien, R.Y. Improved green fluorescence. Nature 373, 663-664 (1995).
6a. van Koningsbruggen, S. et al. Llama-derived phage display antibodies in the dissection of the human disease oculopharyngeal muscular dystrophy. J. Immunol. Methods 279, 149-161 (2003).

## Claims

1. A polypeptide comprising the variable region of a heavy chain antibody of *Camelidae* and capable of specifically binding to a GFP epitope, consisting or comprising of: framework 1, CDR1, framework 2, CDR2, framework 3 and CDR3,
(a) having the sequence of any one of SEQ ID NOs: 10 or 30 or encoded by the nucleic acid molecule of any one of SEQ ID NOs: 11 or 31; or
(b) encoded by a nucleic acid molecule with at least 95% sequence identity to SEQ ID NO: 11.

2. The polypeptide of claim 1, further comprising
(a) a tag selected from the group consisting of His-tag, Strep-tag, recognition site for biotinylation; and optionally
(b) the recognition site for a protease.

3. The polypeptide of claim 1 or 2, further comprising a (poly)peptide sequence which is a detectable protein, preferably derivable from a fluorescent or chromophoric protein.

4. A nucleic acid molecule encoding the polypeptide of any one of claims 1 to 3.

5. An expression vector comprising the nucleic acid molecule of claim 4.

6. A host cell comprising the nucleic acid molecule of claim 4 and/or the expression vector of claim 5.

7. A method of specifically precipitating GFP, a GFP-tagged protein or proteins interacting therewith in a sample, comprising the step of contacting the sample with the polypeptide of claim 1.

8. The method of claim 7, wherein the polypeptide are bound to a solid support.

9. The method of claim 8, wherein the solid support is sepharose.

## Patentansprüche

1. Polypeptid, das die variable Domäne eines Schwere-Kette-Antikörpers der *Camelidae* umfasst und spezifisch an ein GFP-Epitop binden kann, bestehend aus oder umfassend: Gerüstregion 1, CDR 1, Gerüstregion 2, CDR 2, Gerüstregion 3 und CDR 3,
(a) mit der Sequenz einer der SEQ-ID-Nummern 10 oder 30 oder kodiert durch das Nukleinsäuremolekül einer der SEQ-ID-Nummern 11 oder 31; oder
(b) kodiert durch ein Nukleinsäuremolekül mit einer Sequenzidentität von zumindest 95% zur SEQ-ID-Nr. 11.

2. Polypeptid nach Anspruch 1, ferner umfassend
(a) einen Tag ausgewählt aus der Gruppe bestehend aus His-Tag, Strep-Tag, Erkennungsstelle für Biotinylierung; und gegebenenfalls
(b) eine Erkennungsstelle für eine Protease.

3. Polypeptid nach Anspruch 1 oder 2, ferner umfassend eine (Poly)peptidsequenz, die ein nachweisbares Protein ist, vorzugsweise von einem fluoreszenten oder chromophoren Protein ableitbar.

4. Nukleinsäuremolekül, das das Polypeptid nach einem der Ansprüche 1 bis 3 kodiert.

5. Expressionsvektor umfassend das Nukleinsäuremolekül nach Anspruch 4.

6. Wirtszelle umfassend das Nukleinsäuremolekül nach Anspruch 4 und/oder den Expressionsvektor nach Anspruch 5.

7. Verfahren zur spezifischen Ausfällung von GFP, eines GFP-markierten Proteins oder damit interagierender Proteine in einer Probe, umfassend den Schritt des Inkontaktbringens der Probe mit dem Polypeptid nach Anspruch 1.

8. Verfahren nach Anspruch 7, wobei das Polypeptid an einen festen Träger gebunden ist.

9. Verfahren nach Anspruch 8, wobei der feste Träger Sepharose ist.

## Revendications

1. Polypeptide comprenant la région variable d'une chaîne lourde d'anticorps de camélidés et capable de se lier spécifiquement à un épitope de la GFP, constitué de ou comprenant : une région charpente 1, une région CDR1, une région charpente 2, une région CDR2, une région charpente 3 et une région CDR3,
(a) ayant la séquence de l'une quelconque des SEQ ID NO : 10 ou 30 ou codé par la molécule d'acide nucléique de l'une quelconque des SEQ ID NO : 11 ou 31 ; ou
(b) codé par une molécule d'acide nucléique présentant au moins 95 % d'identité de séquence avec SEQ ID NO : 11.

2. Polypeptide selon la revendication 1, comprenant en outre
(a) une étiquette choisie dans le groupe constitué par l'étiquette His, l'étiquette Strep, le site de reconnaissance pour la biotinylation ; et éventuellement
(b) le site de reconnaissance pour une protéase.

3. Polypeptide selon la revendication 1 ou 2, comprenant en outre une séquence (poly)peptidique qui est une protéine détectable, de préférence pouvant être dérivée d'une protéine fluorescente ou chromophorique.

4. Molécule d'acide nucléique codant pour le polypeptide selon l'une quelconque des revendications 1 à 3.

5. Vecteur d'expression comprenant la molécule d'acide nucléique selon la revendication 4.

6. Cellule hôte comprenant la molécule d'acide nucléique selon la revendication 4 et/ou le vecteur d'expression selon la revendication 5.

7. Procédé de précipitation spécifique de la GFP, d'une protéine marquée par la GFP ou de protéines interagissant avec celle-ci dans un échantillon, comprenant l'étape de mise en contact de l'échantillon avec le polypeptide selon la revendication 1.

8. Procédé selon la revendication 7, dans lequel le polypeptide est lié à un support solide.

9. Procédé selon la revendication 8, dans lequel le support solide est le sépharose.
